(19) Europäisches Patentamt
European Patent Office
Office européen des brevets

(11) **EP 3 986 438 B1**

(12) **EUROPEAN PATENT SPECIFICATION**

(45) Date of publication and mention of the grant of the patent:
**16.04.2025 Bulletin 2025/16**

(21) Application number: **20827691.5**

(22) Date of filing: **16.06.2020**

(51) International Patent Classification (IPC):
***C07K 7/06*** *(2006.01)* ***C07K 7/08*** *(2006.01)*
***A61P 35/00*** *(2006.01)* ***A61K 45/06*** *(2006.01)*
*A61K 38/00* *(2006.01)*

(52) Cooperative Patent Classification (CPC):
**A61K 45/06; A61P 35/00; C07K 7/06; C07K 7/08;**
A61K 38/00

(86) International application number:
**PCT/US2020/037869**

(87) International publication number:
**WO 2020/257153 (24.12.2020 Gazette 2020/52)**

# (54) P53 ACTIVATOR PEPTIDOMIMETIC MACROCYCLES

PEPTIDOMIMETISCHE P53-AKTIVATOR-MAKROCYCLEN

MACROCYCLES PEPTIDOMIMÉTIQUES ACTIVATEURS DE P53

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2019 US 201962864531 P**

(43) Date of publication of application:
**27.04.2022 Bulletin 2022/17**

(73) Proprietors:
- **Merck Sharp & Dohme LLC**
  **Rahway, New Jersey 07065 (US)**
- **Agency for Science, Technology and Research**
  **Singapore 138632 (SG)**
- **MSD International GmbH (Singapore Branch)**
  **Singapore 638408 (SG)**

(72) Inventors:
- **ARONICA, Pietro**
  **Singapore 138632 (SG)**
- **BROWN, Christoper, J.**
  **Singapore 138632 (SG)**
- **FERRER, Fernando, J.**
  **Singapore 138632 (SG)**
- **JOHANNES, Charles, W.**
  **Singapore 138632 (SG)**
- **KANNAN, Srinivasaraghavan**
  **Singapore 138632 (SG)**
- **LANE, David, P.**
  **Singapore 138632 (SG)**
- **PARTRIDGE, Anthony, W.**
  **Singapore 638408 (SG)**
- **TAN, Yaw Sing**
  **Singapore 138632 (SG)**
- **VERMA, Chandra, S.**
  **Singapore 138632 (SG)**
- **YUEN, Tsz Ying**
  **Singapore 138632 (SG)**

(74) Representative: **Merck Sharp & Dohme LLC**
**120 Moorgate**
**London EC2M 6UR (GB)**

(56) References cited:
**WO-A1-2008/095063 WO-A1-2013/123266**
**WO-A1-2016/154058 WO-A1-2017/205786**
**US-A1- 2012 328 692**

- **MORIMOTO JUMPEI ET AL: "Isolation of a peptide containing D-amino acid residues that inhibits the [alpha]-helix-mediated p53-MDM2 interaction from a one-bead one-compound library", BIOORGANIC & MEDICINAL CHEMISTRY LETTERS, vol. 28, no. 3, 2 January 2018 (2018-01-02), Amsterdam NL, pages 231 - 234, XP093056749, ISSN: 0960-894X, DOI: 10.1016/j.bmcl.2018.01.001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 986 438 B1

- **ZHAN CHANGYOU ET AL: "An Ultrahigh Affinity D-Peptide Antagonist of MDM2", JOURNAL OF MEDICINAL CHEMISTRY, vol. 55, no. 13, 13 June 2012 (2012-06-13), US, pages 6237 - 6241, XP055775739, ISSN: 0022-2623, Retrieved from the Internet <URL:https://pubs.acs.org/doi/pdf/10.1021/jm3005465> DOI: 10.1021/jm3005465**
- **ZHAN CHANGYOU, ZHAO LE, WEI XIAOLI, WU XUEJI, CHEN XISHAN, YUAN WEIRONG, LU WEI-YUE, PAZGIER MARZENA, LU WUYUAN: "An Ultrahigh Affinity D-Peptide Antagonist of MDM2", JOURNAL OF MEDICINAL CHEMISTRY, vol. 55, no. 13, 13 June 2012 (2012-06-13), pages 6237 - 6241, XP055775739**
- **NEOCHORITIS CONSTANTINOS G, KAZEMI MIRAKI MARYAM, ABDELRAHEEM EMAN M M, SURMIAK EWA, ZARGANES-TZITZIKAS TRYFON, ŁABUZEK BEATA, HOL: "Design of indole- and MCR-based macrocycles as p53-MDM2 antagonists", BEILSTEIN JOURNAL OF ORGANIC CHEMISTRY, vol. 15, 20 February 2019 (2019-02-20), pages 513 - 520, XP055775741**
- **KANNAN SRINIVASARAGHAVAN, ARONICA PIETRO G. A., NG SIMON, THEAN DAWN, FROSI YURI, CHEE SHARON, SHIMIN JIANG, YUEN TSZ YING, SADRUD: "Macrocyclization of an all-D linear peptide improves target affinity and imparts cellular activity: A novel stapled alpha-helical peptide modality", BIORXIV, 12 September 2019 (2019-09-12), pages 1 - 37, XP055775746**


Remarks:

The complete document including Reference Table(s) and the Sequence Listing(s) can be downloaded from the EPO website

## Description

BACKGROUND OF THE INVENTION

(1) Field of the Invention

The present invention provides peptidomimetic macrocycles that comprise all-D configuration α-amino acids and bind mouse double minute 2 (MDM2 aka E3 ubiquitin-protein ligase) and MDMX (aka MDM4). These all-D configuration α-amino acid peptidomimetic macrocycles are protease resistant, cell permeable without inducing membrane disruption, and intracellularly activate p53 by binding MDM2 and MDMX, thereby antagonizing MDM2 and MDMX binding to p53. These peptidomimetic macrocycles may be useful in anticancer therapies, particularly in combination with chemotherapy or radiation therapy.

(2) Description of Related Art

p53 is a key tumor suppressor protein that primarily functions as a DNA transcription factor. It is commonly abrogated in cancer and plays a crucial role in guarding the cell in response to various stress signals through the induction of cell cycle arrest, apoptosis, of senescence [46]. Mechanisms that frequently result in the inactivation of p53 and tumorigenesis include increased expression of the p53-negative regulators MDM2 and MDMX (aka MDM4). Both MDM2 and MDMX attenuate p53 function by interacting directly with p53 and preventing its interaction with the relevant activation factors required for transcription, e.g $dTAF_{II}$, $hTAF_{II}$. In addition, they are both E3 ligase components and target p53 for proteosomal mediated degradation. MDMX, unlike MDM2, has no intrinsic E3 ubiquitin ligase activity. Instead, MDMX forms heterodimeric complexes with MDM2 whereby it stimulates the ubiquitin activity of MDM2. As a result, p53 activity and protein levels are acutely suppressed by MDM2 and MDMX overexpression. Development of inhibitors to disrupt the interactions of p53 with either MDM2 or MDMX, or both, are therefore highly desirable as they will prevent p53 degradation and restore a p53 dependent transcriptional anti-tumor response [47,48].

The structural interface of the p53 MDM2/MDMX complex is characterized by an α-helix from the *N*-terminal transactivation domain of p53 which binds into a hydrophobic groove on the surface of the *N*-terminal domain of both MDM2 and MDMX. Three hydrophobic residues, Phe19, Trp23 and Leu26, of p53 are critical determinants of this interaction and project deeply into the MDM2/MDMX interaction groove [See **Fig. 1**]. The isolated p53 peptide is largely disordered, morphing into an α-helical conformation upon binding. There are several examples of small molecules, peptides, and biologics that mimic these interactions and compete for MDM2/MDMX binding, with the release of p53 [49]. However, a large majority of the small molecules developed exhibit little affinity and activity against MDMX, which possesses several distinct structural differences in the p53 peptide binding groove compared to MDM2. Although several MDM2 specific molecules have entered initial clinical trials, they have largely been met with dose limiting toxicities in patients [49]. Overexpression of MDMX in tumors has been demonstrated to attenuate the effectiveness of MDM2 specific compounds, presumably through the maintenance of heterodimeric complexes of MDM2 and MDMX that inhibit and target p53 for proteosomal degradation. MDM2-selective inhibitors may also induce higher levels of MDMX. This highlights the importance of targeting both proteins simultaneously to achieve efficient activation of p53 to achieve an optimal therapeutic response.

Protein-protein interactions (PPIs) are central to most biological processes and are often dysregulated in disease [1, 2] . Therefore, PPIs are attractive therapeutic targets for novel drug discovery. However, in contrast to the deep protein cavities that typically accommodate small molecules, PPI surfaces are generally large and flat, and this has contributed to the limited successful development of small molecule inhibitors for PPI targets [3]. The realization that 40% of all PPIs are mediated by relatively short peptide motifs gave rise to the possibility of developing peptide-based inhibitors that would compete orthosterically for the interface between ligand-target cognate partners [4]. When taken out of the protein ligand context and synthesized, such peptides may often be unstructured and intrinsically disordered, yet capable to achieve their biologically -relevant conformation upon protein target binding [4]. However, for intracellular targets, the peptide modality may be challenging due to proteolytic sensitivity, low conformational stability (yielding weak affinities and off target effects), and poor cell permeability (further limiting prosecution of intracellular targets and/or oral bioavailability) [5-11]. To address these issues, several strategies have been pursued, including macrocyclization and modifications of the peptide backbone to yield molecules with improved activities and pharmacokinetic properties as well as constraining the peptide into to its biologically -relevant conformation to bind its target) [5-13]. First, by biasing the peptides toward their bound conformations, entropic penalties upon binding are reduced, thus improving binding constants as well as presumably decreasing the opportunity for unwanted off-target effects. Secondly, macrocyclization may confer varying degrees of proteolytic resistance by modifying key backbone and/or side-chain structural moieties in the peptide. Thirdly, macrocyclization may enhance cell permeability, such as through increased stability of intramolecular hydrogen bonding to reduce the desolvation penalty otherwise incurred in the transport of peptides cross an apolar cell membrane. Amongst

the several cyclization techniques described, stapling *via* metathesis using a non-proteogenic amino acid such as alpha methyl alkenyl side chains has proven to be very effective [13-18], particularly when the desired secondary structure of the peptide macrocycle is helical. Stapling requires incorporation of the appropriate unnatural amino acid precursors to be placed at appropriate locations along the peptide sequence such that they do not interfere with the binding face of the helix. The linkers can be of different types, and can span different lengths, resulting in *i,i+3 i,i+4, i,i+7* staples. Although they have largely been used to stabilize helical conformations, recent studies have also applied ring-closing metathesis (RCM) strategies to non-helical peptides [19, 20].

The stapled peptide strategy has been successfully applied to inhibit several PPIs of therapeutic potential including, BCL-2 family-BH3 domains [21-24], β-catenin-TCF [25], Rab-GTPase-Effector [26], ERα-coactivator protein [27], Cullin3-BTB [28], VDR-coactivator protein [29], elf4E [30], ATSP-7041 [See WO2013123266, SAH-p53-8 [Bernal et al., Cancer Cell 18: 411-422 (2010)], and p53-MDM2/MDMX [31-34]. Noteworthy, in the case of p53-MDM2/MDMX, a dual selective stapled peptide (ALRN-6924; Aileron Therapeutics, Inc.) has been further successfully advanced to phase II clinical trials [35-37]. Although this example is unquestionably encouraging for the advancement of stapled peptides into the clinic, challenges yet remain. Amongst these, engineering molecules with sufficient proteolytic stability for sustained target binding and cellular activity is critical. Indeed, although stapling L-amino acid peptides can confer resistance to protease-mediated degradation, the effect is often not complete, and may affect residues located outside of the macrocycle [38-40].

On the other hand, all-D configuration α-amino acid peptides are hyperstable against proteolysis as most proteases are chiral, they distinguish between L- and D-enantiomeric versions of the substrate; as a result, all-D configuration α-amino acid peptides are able to resist the activity of proteases. All-D configuration α-amino acid peptides have been engineered with strong binding affinity against a variety of targets including p53-MDM2 [41-42], VEGF-VEGF-receptor [43], PD-1-PD-L1 [44], and human immunodeficiency virus type 1 (HIV-1) entry [45]. Unfortunately, although all-D configuration α-amino acid peptides are intrinsically hyperstable to proteolysis, the generally lack membrane permeability and cellular activity.

For example, $^{D}$PMI-δ, is an all-D configuration α-amino acid linear peptide (PMI: p53-MDM2/MDMX inhibitor) that was derived from a mirror image phage display screen reported by Liu et al. [41] and in U.S. Pub. Patent No. 20120328692. Specifically, they reported several 12-mer D-peptide antagonists of MDM2 (termed $^{D}$PMI-α, β, γ) that bind with affinities as low as 35 nM and are resistant to proteolytic degradation. $^{D}$PMI-δ is a corresponding analogue in which the tryptophan at position 3 was substituted with 6-fluoro-D-tryptophan (6-F-$^{D}$Trp3) and the phenylalanine at position 7 was substituted with p-trifluoromethyl-D-phenylalanine (*p*-$CF_3$-$^{D}$Phe7) to improve the MDM2 binding $K_d$ to 220 pM [51]. Crystal structures [51] of the complex between this peptide and the *N*-terminal domain of MDM2 showed that the peptide was bound in a conformation similar to that adopted by the wild-type peptide (the all-L amino acid peptide derived from p53). The helix, as expected, was left-handed and projected the side chains of $^{D}$Trp2, *p*-$CF_3$-$^{D}$Phe7 and $^{D}$Leu11 into the hydrophobic pocket of MDM2, in conformations similar to those adopted by the side chains of Phe19, Trp23 and Leu26 in the wild type peptide [**Fig. 1**]. However, this peptide lacked cell permeability, but did activate p53 in cells when delivered using nano-carriers [42]. US 2012/328692 to Lu et al. discloses PMI compounds, which are all-D configuration alpha-amino acid linear peptide derivatives (PMI: p53- MDM2/MDMX inhibitor). Lu et al. also disclose hydrocarbon-stapled D-peptides that bind with high affinity to MDM2 and/or MDMX, and that antagonize the ability of the ligases to regulate p53 activity in vivo. The stapled D-peptides are prepared using the D-peptides and variants of the D-peptides of PMI and are useful for the treatment of cancer.

## BRIEF SUMMARY OF THE INVENTION

The scope of the present invention is defined by the appended set of claims. The present invention provides peptidomimetic macrocycles comprising stably cross-linked peptides having all-D configuration α-amino acids. These peptides are derived from a peptidomimetic analog of a portion of human p53 having the amino acid sequence set forth in SEQ ID NO: 1 and having the formula

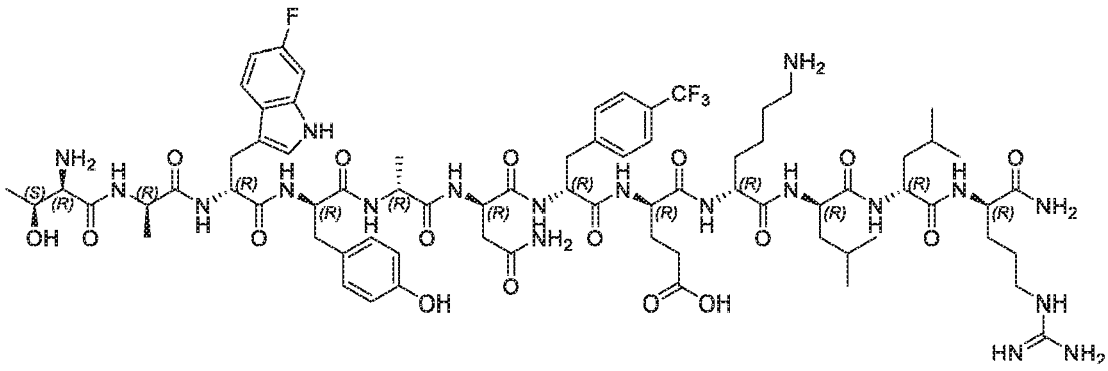

These cross-linked peptidomimetic macrocycles contain at least two modified amino acids that together form an intramolecular cross-link that stabilizes the alpha-helical secondary structure of a portion of the peptides that antagonizes the binding of p53 to MDM2 and/or MDMX. In embodiments comprising a crosslink between two modified amino acids, the crosslink is referred to as a staple and the peptide as a stapled peptide. A peptide may have one or more staples. In embodiments comprising two crosslinks between modified amino acids and the two crosslinks share a common modified amino acid, the crosslinks are referred to as stitches and the peptide as a stitched peptide.

The peptidomimetic macrocycles interfere with binding of p53 to MDM2 and/or of p53 to MDMX, thereby liberating functional p53 and inhibiting its destruction. The peptidomimetic macrocycles described herein can be used therapeutically, for example to treat cancers and other disorders characterized by an undesirably low level or a low activity of p53, and/or to treat cancers and other disorders characterized by an undesirably high level of activity of MDM2 or MDMX. The peptidomimetic macrocycles may also be useful for treatment of any disorder associated with disrupted regulation of the p53 transcriptional pathway, leading to conditions of excess cell survival and proliferation such as cancer and autoimmunity, in addition to conditions of inappropriate cell cycle arrest and apoptosis such as neurodegeneration and immunodeficiencies.

The peptidomimetic macrocycles of the present invention bind MDM2 and MDMX, are cell permeable without inducing detectable disruption to the cell membrane, resistant to digestion by extracellular and intracellular proteases, and activate p53 intracellularly.

Thus, the present invention provides a peptidomimetic macrocycle comprising a peptide of D configuration α-amino acids having the amino acid sequence set forth in SEQ ID NO: 16 and two staples or one stitch, wherein each staple comprises a hydrocarbon crosslinker linking the α-carbons of two α,α-disubstituted amino acids separated by at least two α-amino acids and each stitch comprises two hydrocarbon crosslinkers linking the α-carbons of two α,α-disubstituted amino acids to the α-carbon of a common α,α-disubstituted amino acid. In particular aspects, at least one α,α-disubstituted amino acid of the peptidomimetic macrocycle has a D configuration.

In a further embodiment of the peptidomimetic macrocycle, wherein each α,α-disubstituted amino acid comprises one or two α-carbon-linked reactive groups wherein the reactive group of a first α,α-disubstituted amino acid is capable of reacting with the reactive group of a second α,α-disubstituted amino acid to form a crosslinker. In particular aspects, the reactive group comprises a terminal olefin group.

In a further embodiment of the peptidomimetic macrocycle, the peptide comprises a stitch in which a first crosslinker links the α-carbon of an α,α-disubstituted amino acid at position 1 to the α-position of a common α,α-disubstituted amino acid at position 5 and a second crosslinker links the α-position of an α,α-disubstituted amino acid at position 5 to the α-position of the common α,α-disubstituted amino acid at position 5.

In a further embodiment of the peptidomimetic macrocycle, the α,α-disubstituted amino acid at position 1 is (R)-2-(4'-pentenyl)alanine, at position 12 is (R)-2-(7'-octenyl)alanine, and at position 5 is 2,2-(4'-pentenyl)glycine.

In a further embodiment of the peptidomimetic macrocycle, the peptidomimetic macrocycle comprises the amino acid sequence set forth in SEQ ID NO: 8, which in a further aspect is SEQ ID NO: 23 represented by the formula

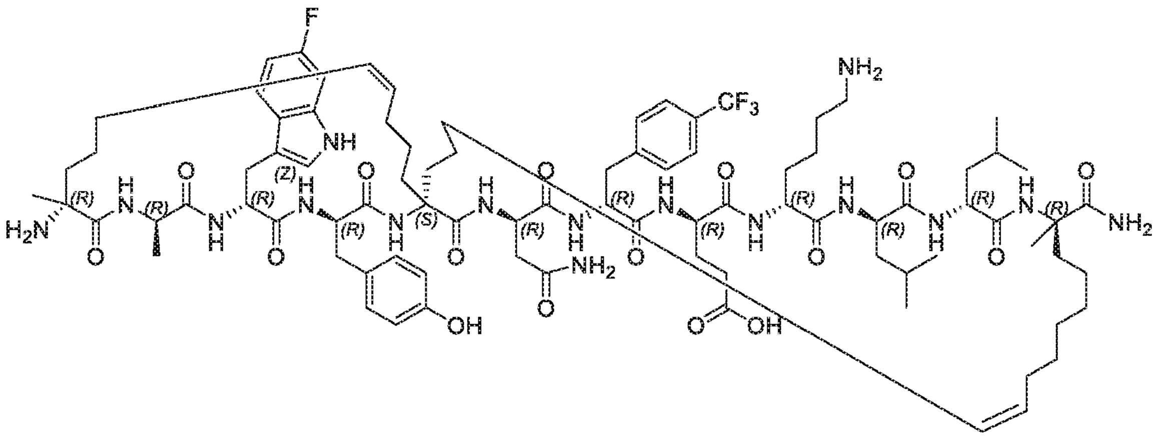

In a further embodiment of the peptidomimetic macrocycle, the peptide comprises two staples wherein a first staple comprises a first crosslinker that links the α-position of an α,α-disubstituted amino acid at position 1 to the α-position of an α,α-disubstituted amino acid at position 5 and a second staple comprises a second crosslinker that links the α-position of an α,α-disubstituted amino acid at position 9 to the α-position of an α,α-disubstituted amino acid at position 12.

In a further embodiment of the peptidomimetic macrocycle, the α,α-disubstituted amino acids at positions 1 and 5 are each (R)-2-(4'-pentenyl)alanine and the amino acids at positions 9 and 12 are (S)-2-(4'-pentenyl)alanine and (R)-2-(7'-octenyl)alanine, respectively.

In a further embodiment of the peptidomimetic macrocycle, the peptidomimetic macrocycle comprises the amino acid sequence set forth in SEQ ID NO: 9, which in a further aspect is SEQ ID NO: 24 represented by the formula

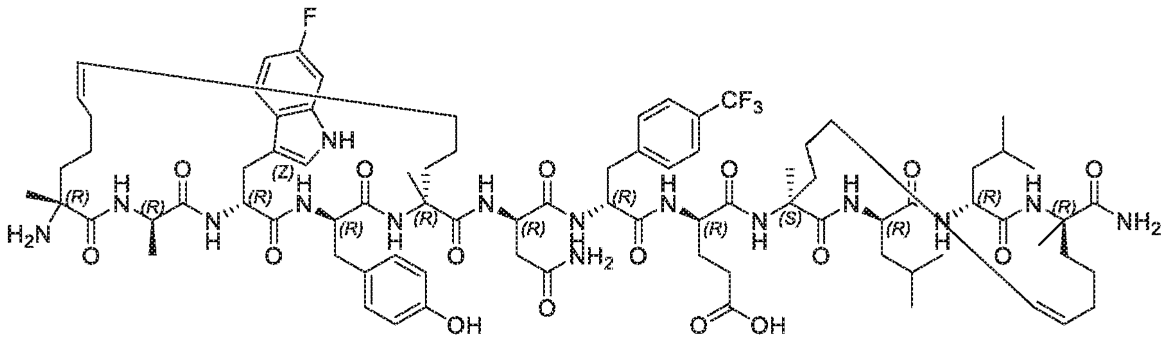

In further embodiments of the present invention, the peptidomimetic macrocycle binds both MDM2 and MDMX, is protease resistant and cell permeable with no detectable disruption of the cell membrane as determined by a lactate dehydrogenase (LDH) release assay, and activates p53 intracellularly.

The references to methods of treatment in the subsequent paragraphs of this description are to be interpreted as references to the compounds, pharmaceutical compositions and medicaments of the present invention for use in a method for treatment of the human (or animal) body by therapy. The present invention further provides a method of modulating the activity of p53 and/or MDM2 and/or MDMX in a subject comprising administering to the subject a peptidomimetic macrocycle of any one of the aforementioned peptidomimetic macrocycles. The present invention further provides a method of antagonizing the interaction between p53 and MDM2 and/or between p53 and MDMX proteins in a subject comprising administering to the subject a peptidomimetic macrocycle of any one of the aforementioned peptidomimetic macrocycles.

The present invention further provides a peptidomimetic macrocycle of any one of the aforementioned peptidomimetic macrocycles for the treatment of cancer. For example, a method for treating cancer in a subject having a cancer comprising administering to the subject any one of the aforementioned peptidomimetic macrocycles. The present invention further provides use of a peptidomimetic macrocycle of any one of the aforementioned peptidomimetic macrocycles for the preparation of a medicament for treating cancer.

In particular embodiments, the cancer is selected from the group consisting of melanoma, non-small cell lung cancer, head and neck cancer, urothelial cancer, breast cancer, gastrointestinal cancer, multiple myeloma, hepatocellular cancer, non-Hodgkin lymphoma, renal cancer, Hodgkin lymphoma, mesothelioma, ovarian cancer, small cell lung cancer, esophageal cancer, anal cancer, biliary tract cancer, colorectal cancer, cervical cancer, thyroid cancer, salivary cancer, pancreatic cancer, bronchus cancer, prostate cancer, pancreatic cancer, stomach cancer, ovarian cancer, urinary bladder

cancer, brain or central nervous system cancer, peripheral nervous system cancer, uterine or endometrial cancer, cancer of the oral cavity or pharynx, liver cancer, kidney cancer, testicular cancer, biliary tract cancer, small bowel or appendix cancer, adrenal gland cancer, osteosarcoma, chondrosarcoma, and cancer of hematological tissues.

The present invention further provides a combination therapy for treating cancer comprising administering to a subject a therapeutically effective amount of a peptidomimetic macrocycle of any one of the aforementioned peptidomimetic macrocycles and a therapeutically effective dose of a chemotherapy agent or radiation. In particular embodiments, the chemotherapy agent or radiation is administered to the subject followed by administration of the peptidomimetic macrocycle; the peptidomimetic macrocycle is administered to the subject followed by administration of the chemotherapy agent or radiation; or the chemotherapy agent or radiation is administered to the subject simultaneously with administration of the peptidomimetic macrocycle. Thus, the present invention further provides a combination therapy for the treatment of a cancer comprising a therapeutically effective amount of a peptidomimetic macrocycle of any one of the aforementioned peptidomimetic macrocycles and a therapeutically dose of a chemotherapy agent or radiation.

The present invention further provides a combination therapy for treating cancer comprising administering to a subject a therapeutically effective amount of a peptidomimetic macrocycle of any one of the aforementioned peptidomimetic macrocycles and a therapeutically effective amount of a checkpoint inhibitor. In particular aspects, the checkpoint inhibitor is an anti-PD1 antibody or an anti-PD-L1 antibody. In further aspects, the therapy further includes administering to the subject a therapeutically effective dose of a chemotherapy agent or radiation.

The present invention further provides a treatment for cancer comprising administering to a subject having the cancer a vector comprising a nucleic acid molecule encoding a wild-type p53 protein or p53 variant with transcriptional activation activity followed by one or more administrations of a therapeutically effective amount of a peptidomimetic macrocycle of any one of the aforementioned peptidomimetic macrocycles. In particular embodiments, the vector is a plasmid, a retrovirus, adenovirus, or adeno-associated virus. In further embodiments, the subject is administered a chemotherapy or radiation treatment prior to administering the vector to the subject or subsequent to administering the vector to the subject. In further still embodiments, the therapy includes administering to the subject a checkpoint inhibitor prior to administering the vector to the subject or subsequent to administering the vector to the subject. The checkpoint inhibitor may be administered prior to administering the chemotherapy or radiation treatment to the subject or subsequent to administering the chemotherapy or radiation treatment to the subject.

In particular embodiments of the aforementioned treatments or therapies, the chemotherapy agent is selected from the group consisting of actinomycin, all-trans retinoic acid, alitretinoin, azacitidine, azathioprine, bexarotene, bleomycin, bortezomib, carmofur, carboplatin, capecitabine, cisplatin, chlorambucil, cyclophosphamide, cytarabine, dacarbazine, daunorubicin, docetaxel, doxifluridine, doxorubicin, epirubicin, epothilone, etoposide, fluorouracil, gemcitabin, hydroxyurea, idarubicin, imatinib, ixabepilone, irinotecan, mechlorethamine, melphalan, mercaptopurine, methotrexate, mitoxantrone, nitrosoureas, oxaliplatin, paclitaxel, pemetrexed, romidepsin, tegafur, temozolomide(oral dacarbazine), teniposide, tioguanine, topotecan, utidelone, valrubicin, vemurafenib, vinblastine, vincristine, vindesine, vinorelbine, and vorinostat.

The present invention further comprises a composition comprising any one of the aforementioned peptidomimetic macrocycles and a pharmaceutically acceptable carrier or excipient, e.g., comprising any one of SEQ ID NO: 6, SEQ ID NO: 8, SEQ ID NO: 9, SEQ ID NO: 23, and SEQ ID NO: 24. and a pharmaceutically acceptable carrier or excipient. The present invention further comprises a composition comprising a peptidomimetic selected from the group consisting of consisting of SEQ ID NO: 2, SEQ ID NO: 3, SEQ ID NO: 4, SEQ ID NO: 5, SEQ ID NO: 6, SEQ ID NO: 7; SEQ ID NO: 8, and SEQ ID NO:9 and a pharmaceutically acceptable carrier or excipient. The present invention further comprises a composition comprising a peptidomimetic selected from the group consisting of

SEQ ID NO: 23 having the formula

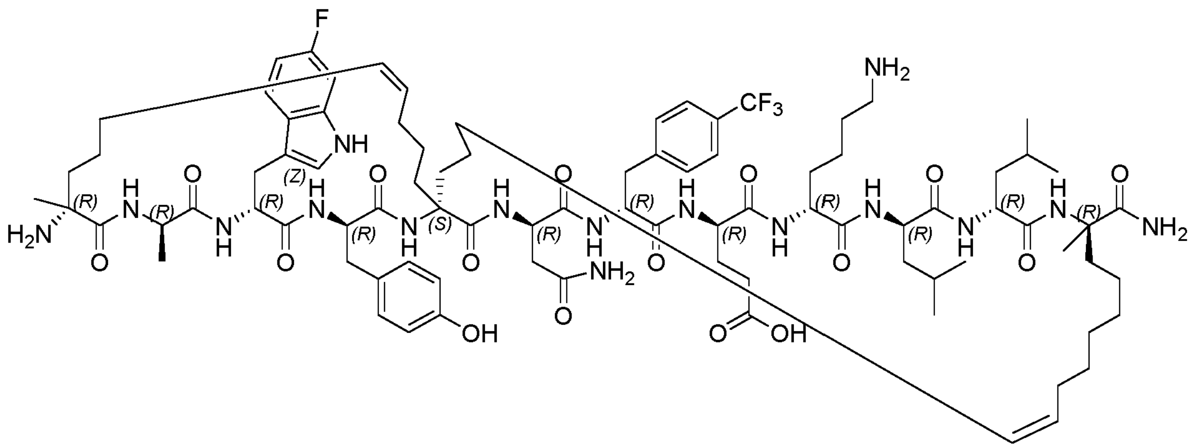

;

and, SEQ ID NO: 24 having the formula

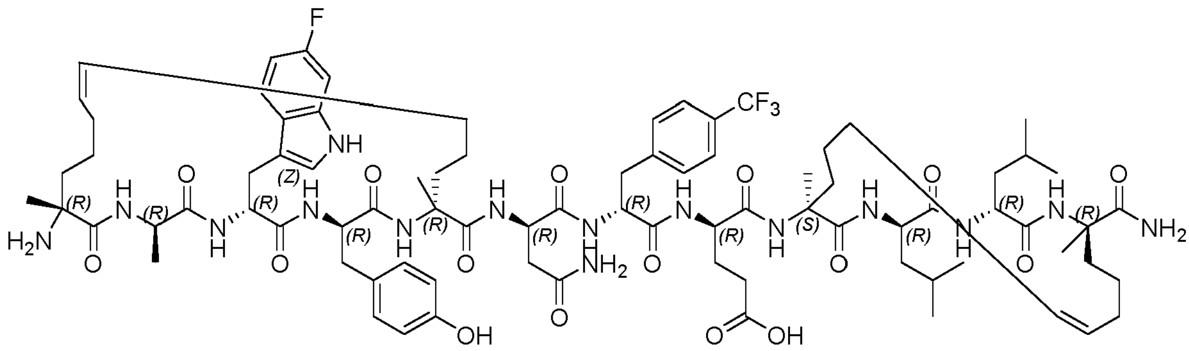

;

and, a pharmaceutically acceptable carrier or excipient.

BRIEF DESCRIPTION OF THE DRAWINGS

**Fig. 1A:** Crystal structure of p53-MDM2 (Protein Data Bank (PDB) ID:1YCR) complex (Baek et al., JACS 134: 103-106 92012)). MDM2 is shown as surface and bound peptide is shown as cartoon with interacting residues L-Phe19, L-Trp23, and L-Leu26 are highlighted in sticks. Hydrogen bond interactions are shown as dotted lines (black).

**Fig. 1B:** Crystal structure of (B) $^{D}$PMI-δ - MDM2 (PDB ID: 3PTX) complex (Zhang et al., J. Med. Chem. 55: 6237-6241 (2012). MDM2 is shown as surface and bound peptide is shown as cartoon with interacting residues $^{D}$Leu11, pCF$_3$-$^{D}$Phe7, and 6-F-$^{D}$Trp3 are highlighted in sticks. Hydrogen bond interactions are shown as dotted lines (black).

**Fig. 2A:** Probability distributions (the three lines represent three replica simulations) of the RMSD of $^{D}$PMI-δ sampled during the MD simulations of the $^{D}$PMI-δ - MDM2 complex.

**Fig. 2B:** Probability distributions (the three lines represent three replica simulations) of the RMSD of MDM2 sampled during the MD simulations of the $^{D}$PMI-δ - MDM2 complex; the RMSD is relative to the starting structure of the $^{D}$PMI-δ - MDM2 complex.

**Fig. 2C:** Probability distributions (the three lines represent three replica simulations) of the SASA of 6-F-$^{D}$W3, p-CF$_3$-$^{D}$F7, or $^{D}$Leu11 sampled during the molecular dynamics (MD) simulations of the $^{D}$PMI-δ - MDM2 complex; the RMSD is relative to the starting structure of the $^{D}$PMI-δ - MDM2 complex.

**Fig. 2D:** Probability distribution of root-mean-square-deviation (RMSD) of peptide conformations sampled during BPREMD simulations in the absence of MDM2.

**Fig. 2E:** CD spectra of $^{D}$PMI-δ peptide; note that this spectra is inverted, as expected for a peptide consisting only of D-amino acids.

**Fig. 3A:** Energetic analysis of the MD simulations of the $^{D}$PMI-δ - MDM2 complex. Binding free energy contributions of $^{D}$PMI-δ peptide residues.

**Fig. 3B:** Energetic analysis of the MD simulations of the $^{D}$PMI-δ - MDM2 complex. Computational alanine (D-ala) scan of $^{D}$PMI-δ peptide residues; values along y-axis represent the change in free energy upon mutation from wild type to

Ala of each residue in the peptide.

**Fig. 4:** A helical wheel representation of the $^{D}$PMI-δ template sequence used for the design of stapled peptides. Residues that are linked through all hydrocarbon linkers i,i+4 and i,i+7 are indicated. Sequences of $^{D}$PMI-δ and the six stapled $^{D}$PMI-δ peptides are also shown wherein $^{D}$PMI-δ has SEQ ID NO: 1; $^{D}$PMI-δ(1-5) has SEQ ID NO: 2; $^{D}$PMI-δ(2-6) has SEQ ID NO: 3; $^{D}$PMI-δ(2-9) has SEQ ID NO: 4; $^{D}$PMI-δ(5-9) has SEQ ID NO: 5; $^{D}$PMI-δ(5-12) has SEQ ID NO: 6; and, $^{D}$PMI-δ(6-10) has SEQ ID NO: 7. All amino acids are D-amino acids.

**Fig. 5A:** Binding of stapled $^{D}$PMI-δ peptides toward MDM2 protein measured by circular dichroism (CD). Note that the CD spectra is inverted, as expected for a peptide consisting of D-amino acids only.

**Fig. 5B:** Binding of stapled $^{D}$PMI-δ peptides toward MDM2 protein measured by fluorescence polarization (FP).

**Fig. 5C:** Binding of stapled $^{D}$PMI-δ peptides toward MDM2 protein measured by isothermal titration calorimetry (ITC).

**Fig. 5D:** Binding of stapled $^{D}$PMI-δ peptides toward MDM2 protein measured surface plasmon resonance (SPR).

**Fig. 6:** Structural representation of a snapshot of the $^{D}$PMI-δ(1-5) -MDM2 (left) and $^{D}$PMI-δE(5-12)-MDM2 (right) complexes taken from MD simulations. MDM2 is shown as surface and bound peptide is shown as cartoon with interacting residues highlighted in sticks. The hydrocarbon linker is light portion of peptide indicated by arrow. Hydrogen bond interactions are shown as dotted lines.

**Fig. 7A:** Stapled $^{D}$PMI-δ peptides titrated on to HCT116 p53 reporter cells and p53 transcriptional activation assessed in the absence of serum.

**Fig. 7B:** Stapled $^{D}$PMI-δ peptides titrated on to HCT116 cells and LDH release measured.

**Fig. 7C:** Activity of stapled $^{D}$PMI-δ peptides measured in a counter screen.

**Fig. 8A:** Sequences of $^{D}$PMI-δ and stapled and stitched $^{D}$PMI-δ peptides: $^{D}$PMI-δ has SEQ ID NO: 1; $^{D}$PMI-δ(1-5) has SEQ ID NO: 2; $^{D}$PMI-δ(5-12) has SEQ ID NO: 6; $^{D}$PMI-δ(1,5,12) has SEQ ID NO: 8; and, $^{D}$PMI-δ(1-5, 9-12) has SEQ ID NO: 9. A snapshot from an MD simulation of the stitched $^{D}$PMI-δ(1,5,12) -MDM2 complex. All amino acids are D-amino acids. MDM2 is shown as surface and bound peptide is shown as cartoon with interacting residues highlighted in sticks. Linkers 1-5 and 5-12 of the hydrocarbon stitch are indicated by the arrows. Hydrogen bond interactions are shown as dotted lines.

**Fig. 8B:** Surface plasmon resonance analysis of the binding of two stapled $^{D}$PMI-δ peptides and MDM2 protein.

**Fig. 8C:** Fluorescence polarization analysis of the binding of stapled $^{D}$PMI-δ peptides and MDM2 protein.

**Fig. 8D:** Stapled $^{D}$PMI-δ peptides titrated on to HCT116 p53 reporter cells and p53 transcriptional activation assessed in the absence of serum.

**Fig. 8D:** Stapled $^{D}$PMI-δ peptides titrated on to HCT116 p53 reporter cells and LDH release measured.

**Fig. 8F:** Activity of stapled $^{D}$PMI-δ peptides measured in a counter screen.

**Fig. 9A:** Sequence comparison of the *N*-terminal domains of MDM2 (SEQ ID NO: 10) and MDM4 (SEQ ID NO: 11). Identical residues are highlighted and binding pocket residues (residues that are within 6 Å of bound peptide) are also highlighted (*).

**Fig. 9C:** Snapshot from an MD simulation of the stitched $^{D}$PMI-δ(1,5,12) - MDM2 complex. MDM2 is shown as surface and bound peptide is shown as cartoon with interacting residues highlighted in sticks. Linkers 1-5 and 5-12 of the hydrocarbon stitch are indicated by the arrows. Hydrogen bond interactions are shown as dotted lines.

**Fig. 9C:** Fluorescence polarization binding analysis of stapled $^{D}$PMI-δ peptides and MDM4 protein.

**Fig. 10:** Metabolic stability of the stapled $^{D}$PMI-δ peptides quantified over four hours.

**Fig. 11:** Western blot analysis of HCT-116 cells treated with either vehicle control (1% (v/v) DMSO) or with 6.123 μM, 12.5 μM and 25 μM of the stated compound for either four or 24 hours. Compounds treatments contained a residual DMSO concertation of 1% (v/v) DMSO.

## DETAILED DESCRIPTION OF THE INVENTION

### *Definitions*

**"Administer"** and **"administering"** are used to mean introducing at least one peptidomimetic macrocycle, or a pharmaceutical composition comprising at least one peptidomimetic macrocycle, into a subject. When administration is for the purpose of treatment, the substance is provided at, or after the diagnosis of an abnormal cell growth, such as a tumor. The therapeutic administration of this substance serves to inhibit cell growth of the tumor or abnormal cell growth.

**"α-amino acid"** or simply **"amino acid"** refers to a molecule containing both an amino group and a carboxyl group bound to a carbon, which is designated the α-carbon, attached to a side chain (R group) and a hydrogen atom and may be represented by the formula shown for (R) and (S) α-amino acids

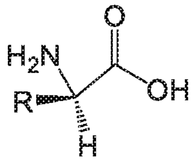
(R)-α-amino acid

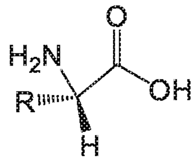
(S)-α-amino acid.

In general, L-amino acids have an (S) configuration except for cysteine, which has an (R) configuration, and glycine, which is achiral. Suitable α-amino acids for the all-D configuration peptides disclosed herein include only the D-isomers of the naturally-occurring amino acids and analogs thereof, as well as non-naturally occurring amino acids prepared by organic synthesis or other metabolic routes except for α,α-disubstituted amino acids, which may be L, D, or achiral. Unless the context specifically indicates otherwise, the term amino acid, as used herein, is intended to include amino acid analogs. As used herein, D amino acids are denoted by the superscript "D" (e.g., $^{D}$Leu) and L amino acids by "L" (e.g., L-Leu) or no L identifier (e.g., Leu).

**"α,α-disubstituted amino acid"** refers to a molecule or moiety containing both an amino group and a carboxyl group bound to the α-carbon that is attached to two natural or non-natural amino acid side chains, or combination thereof. Exemplary α,α-disubstituted amino are shown below. These α,α-disubstituted amino acids comprise a side chain with a terminal olefinic reactive group.

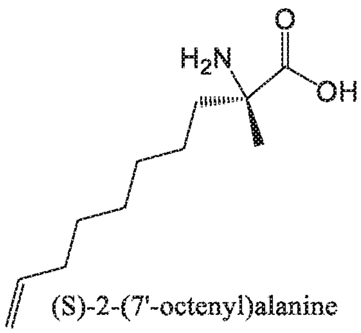
(S)-2-(7'-octenyl)alanine

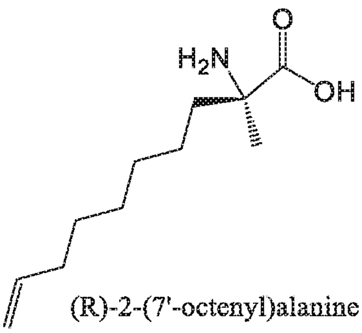
(R)-2-(7'-octenyl)alanine

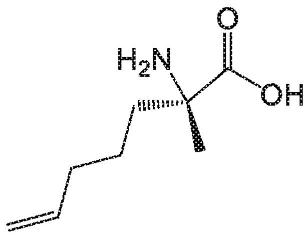
(S)-2-(4'-pentenyl)alanine

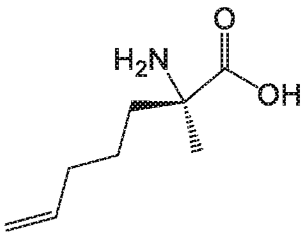
(R)-2-(4'-pentenyl)alanine

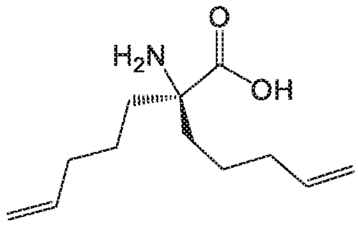
2,2-(4'-pentenyl)glycine

**"Amino acid analog" or "non-natural amino acid"** refers to a molecule which is structurally similar to an amino acid and which can be substituted for an amino acid in the formation of a peptidomimetic macrocycle. Amino acid analogs include, without limitation, compounds which are structurally identical to an amino acid, as defined herein, except for the inclusion of one or more additional methylene groups between the amino and carboxyl group (e.g., α-amino, β-carboxy acids), or for the substitution of the amino or carboxy group by a similarly reactive group (e.g., substitution of the primary amine with a secondary or tertiary amine, or substitution or the carboxy group with an ester).

**"Amino acid side chain"** refers to a moiety attached to the α-carbon in an amino acid. For example, the amino acid side chain for alanine is methyl, the amino acid side chain for phenylalanine is phenylmethyl, the amino acid side chain for cysteine is thiomethyl, the amino acid side chain for aspartate is carboxymethyl, the amino acid side chain for tyrosine is 4-hydroxyphenylmethyl, etc. Other non-naturally occurring amino acid side chains are also included, for example, those that occur in nature (e.g., an amino acid metabolite) or those that are made synthetically (e.g., an α,α-disubstituted amino acid).

**"Capping group"** refers to the chemical moiety occurring at either the carboxy or amino terminus of the polypeptide chain of the subject peptidomimetic macrocycle. The capping group of a carboxy terminus includes an unmodified carboxylic acid (i.e., -COOH) or a carboxylic acid with a substituent. For example, the carboxy terminus can be substituted with an amino group to yield a carboxamide at the C-terminus. Various substituents include but are not limited

to primary and secondary amines, including pegylated secondary amines. The capping group of an amino terminus includes an unmodified amine (i.e. $-NH_2$) or an amine with a substituent. For example, the amino terminus can be substituted with an acyl group to yield a carboxamide at the N-terminus. Various substituents include but are not limited to substituted acyl groups, including $C_1$-$C_6$ carbonyls, $C_7$-$C_{30}$ carbonyls, and pegylated carbamates.

**"Co-administer"** means that each of at least two different biological active compounds are administered to a subject during a time frame wherein the respective periods of biological activity overlap. Thus, the term includes sequential as well as co-extensive administration. When co-administration is used, the routes of administration need not be the same. The biological active compounds include peptidomimetic macrocycles, as well as other compounds useful in treating cancer, including but not limited to agents such as vinca alkaloids, nucleic acid inhibitors, platinum agents, interleukin-2, interferons, alkylating agents, antimetabolites, corticosteroids, DNA intercalating agents, anthracyclines, and ureas. Examples of specific agents in addition to those exemplified herein, include hydroxyurea, 5-fluorouracil, anthramycin, asparaginase, bleomycin, dactinomycin, dacabazine, cytarabine, busulfan, thiotepa, lomustine, mechlorehamine, cyclophosphamide, melphalan, mechlorethamine, chlorambucil, carmustine, 6-thioguanine, methotrexate, etc. The skilled artisan will understand that two different peptidomimetic macrocycles may be co-administered to a subject, or that a peptidomimetic macrocycle and an agent, such as one of the agents provided above, may be co-administered to a subject.

**"Combination therapy"** as used herein refers to treatment of a human or animal individual comprising administering a first therapeutic agent and a second therapeutic agent consecutively or concurrently to the individual. In general, the first and second therapeutic agents are administered to the individual separately and not as a mixture; however, there may be embodiments where the first and second therapeutic agents are mixed prior to administration.

**"Conservative substitution"** as used herein refers to substitutions of amino acids with other amino acids having similar characteristics (e.g. charge, side-chain size, hydrophobicity/hydrophilicity, backbone conformation and rigidity, etc.), such that the changes can frequently be made without altering the biological activity of the protein. Those of skill in this art recognize that, in general, single amino acid substitutions in non-essential regions of a polypeptide do not substantially alter biological activity (*see, e.g.,* Watson et al. Molecular Biology of the Gene, The Benjamin/Cummings Pub. Co., p. 224 (4th Ed.) (1987)). In addition, substitutions of structurally or functionally similar amino acids are less likely to disrupt biological activity. Exemplary conservative substitutions are set forth in **Table 1.**

**Table 1**

| Original residue | Conservative substitution | Original residue | Conservative substitution |
|---|---|---|---|
| Ala (A) | Gly; Ser | Leu (L) | Ile; Val |
| Arg (R) | Lys; His | Lys (K) | Arg; His |
| Asn (N) | Gln; His | Met (M) | Leu; Ile; Tyr |
| Asp (D) | Glu; Asn | Phe (F) | Tyr; Met; Leu |
| Cys (C) | Ser; Ala | Pro (P) | Ala |
| Gln (Q) | Asn | Ser (S) | Thr |
| Glu (E) | Asp; Gln | Thr (T) | Ser |
| Gly (G) | Ala | Trp (W) | Tyr; Phe |
| His (H) | Asn; Gln | Tyr (Y) | Trp; Phe |
| Ile (I) | Leu; Val | Val (V) | Ile; Leu |

**"Dose", "dosage", "unit dose", "unit dosage", "effective dose"** and related terms refer to physically discrete units that contain a predetermined quantity of active ingredient (e.g., peptidomimetic macrocycle) calculated to produce a desired therapeutic effect (e.g., death of cancer cells). These terms are synonymous with the therapeutically-effective amounts and amounts sufficient to achieve the stated goals of the methods disclosed herein.

**"Helical stability"** refers to the maintenance of $\alpha$-helical structure by the staples or stitch of a peptidomimetic macrocycle of the invention as measured by circular dichroism or NMR. For example, in some embodiments, the peptidomimetic macrocycles of the invention exhibit at least a 1.25, 1.5, 1.75 or 2-fold increase in $\alpha$-helicity as determined by circular dichroism compared to a corresponding uncross-linked macrocycle.

**"Macrocycle"** refers to a molecule having a chemical structure including a ring or cycle formed by at least nine covalently bonded atoms.

**"Macrocyclization reagent"** or **"macrocycle-forming reagent"** as used herein refers to any reagent which may be used to prepare a peptidomimetic macrocycle of the invention by mediating the reaction between two reactive

groups. Reactive groups may be, for example, an azide and alkyne, in which case macrocyclization reagents include, without limitation, Cu reagents such as reagents which provide a reactive Cu(I) species, such as CuBr, CuI or CuOTf, as well as Cu(II) salts such as $Cu(CO_2CH_3)_2$, $CuSO_4$, and $CuCl_2$ that can be converted in situ to an active Cu(I) reagent by the addition of a reducing agent such as ascorbic acid or sodium ascorbate.

Macrocyclization reagents may additionally include, for example, Ru reagents known in the art such as $Cp^*RuCl(PPh_3)_2$, $[Cp^*RuCl]_4$ or other Ru reagents which may provide a reactive Ru(II) species. In other cases, the reactive groups are terminal olefins. In such embodiments, the macrocyclization reagents or macrocycle-forming reagents are metathesis catalysts including, but not limited to, stabilized, late transition metal carbene complex catalysts such as Group VIII transition metal carbene catalysts. For example, such catalysts are Ru and Os metal centers having a +2 oxidation state, an electron count of 16 and pentacoordinated. Additional catalysts are disclosed in Grubbs et al., "Ring Closing Metathesis and Related Processes in Organic Synthesis" Acc. Chem. Res. 1995, 28, 446-452, and U.S. Pat. No. 5,811,515. In yet other cases, the reactive groups are thiol groups. In such embodiments, the macrocyclization reagent is, for example, a linker functionalized with two thiol-reactive groups such as halogen groups.

**"MDM2"** refers to the mouse double minute 2 protein also known as E3 ubiquitin-protein ligase. MDM2 is a protein that in humans is encoded by the *MDM2* gene. MDM2 protein is an important negative regulator of the p53 tumor suppressor. MDM2 protein functions both as an E3 ubiquitin ligase that recognizes the *N*-terminal trans-activation domain (TAD) of the p53 tumor suppressor and as an inhibitor of p53 transcriptional activation. As used herein, the term MDM2 refers to the human homolog. See GenBank Accession No.: 228952; GI:228952.

**"MDMX"** or **"MDM4"** refers to mouse double minute X or 4, a protein that shows significant structural similarity to MDM2. MDMX or MDM4 interacts with p53 via a binding domain located in the *N*-terminal region of the MDMX or MDM4 protein. As used herein, the term MDMX or MDM4 refers to the same human homolog. See GenBank Accession No.: 88702791; GI:88702791.

**"Member"** as used herein in conjunction with macrocycles or macrocycle-forming linkers refers to the atoms that form or can form the macrocycle, and excludes substituent or side chain atoms. By analogy, cyclodecane, 1,2-difluoro-decane and 1,3-dimethyl cyclodecane are all considered ten-membered macrocycles as the hydrogen or fluoro substituents or methyl side chains do not participate in forming the macrocycle.

**"Naturally occurring amino acid"** refers to any one of the twenty amino acids commonly found in peptides synthesized in nature, and known by the one letter abbreviations A, R, N, C, D, Q, E, G, H, I, L, K, M, F, P, S, T, W, Y and V.

**"Non-essential"** amino acid residue is a residue that can be altered from the wild-type sequence of a polypeptide without abolishing or substantially altering the polypeptide's essential biological or biochemical activity (e.g., receptor binding or activation). An **"essential"** amino acid residue is a residue that, when altered from the wild-type sequence of the polypeptide, results in abolishing or substantially abolishing the polypeptide's essential biological or biochemical activity.

**"Peptidomimetic macrocycle"** or **"crosslinked polypeptide"** refers to a compound comprising a plurality of amino acid residues joined by a plurality of peptide bonds and at least one macrocycle-forming linker, which forms a macrocycle between a first naturally-occurring or non-naturally-occurring amino acid residue (or analog) and a second naturally-occurring or non-naturally-occurring amino acid residue (or analog) within the same molecule. The peptidomimetic macrocycle include embodiments where the macrocycle-forming linker connects the α-carbon of the first amino acid residue (or analog) to the α-carbon of the second amino acid residue (or analog). Peptidomimetic macrocycles optionally include one or more non-peptide bonds between one or more amino acid residues and/or amino acid analog residues, and optionally include one or more non-naturally-occurring amino acid residues or amino acid analog residues in addition to any which form the macrocycle. A "corresponding non-crosslinked polypeptide" when referred to in the context of a peptidomimetic macrocycle is understood to relate to a polypeptide of the same amino acid sequence as the peptidomimetic macrocycle except for those amino acids involved in the staple or stitch crosslinks.

Unless otherwise stated, compounds and structures referred to herein are also meant to include compounds which differ only in the presence of one or more isotopically enriched atoms. For example, compounds having the present structures wherein hydrogen is replaced by deuterium or tritium, or wherein carbon atom is replaced by $^{13}C$- or $^{14}C$-enriched carbon, or wherein a carbon atom is replaced by silicon, are within the scope of this invention. The compounds of the present invention may also contain unnatural proportions of atomic isotopes at one or more of atoms that constitute such compounds. For example, the compounds may be radiolabeled with radioactive isotopes, such as for example tritium ($^3H$), iodine-125 ($^{125}I$) or carbon-14 ($^{14}C$). All isotopic variations of the compounds of the present invention, whether radioactive or not, are encompassed within the scope of the present invention.

**"Pharmaceutically acceptable derivative"** means any pharmaceutically acceptable salt, ester, salt of an ester, pro-drug or other derivative of a peptidomimetic macrocycle disclosed herein, which upon administration to an individual, is capable of providing (directly or indirectly) a peptidomimetic macrocycle disclosed herein. Particularly favored pharmaceutically acceptable derivatives are those that increase the bioavailability of the peptidomimetic macrocycle disclosed herein when administered to an individual (e.g., by increasing absorption into the blood of an orally administered peptidomimetic macrocycle disclosed herein) or which increases delivery of the active compound to a biological compartment (e.g., the brain or lymphatic system) relative to the parent species. Some pharmaceutically acceptable

derivatives include a chemical group which increases aqueous solubility or active transport across the gastrointestinal mucosa.

**"Polypeptide"** encompasses two or more naturally or non-naturally-occurring amino acids joined by a covalent bond (e.g., an amide bond). Polypeptides as described herein include full length proteins (e.g., fully processed proteins) as well as shorter amino acid sequences (e.g., fragments of naturally-occurring proteins or synthetic polypeptide fragments).

**"Stability"** refers to the maintenance of a defined secondary structure in solution by a peptidomimetic macrocycle of the invention as measured by circular dichroism, NMR or another biophysical measure, or resistance to proteolytic degradation in vitro or in vivo. Nonlimiting examples of secondary structures contemplated in this invention are α-helices, β-turns, and β-pleated sheets.

**"Therapeutically effective amount"** or **"Therapeutically effective dose"** as used herein refers to a quantity of a specific substance sufficient to achieve a desired effect in a subject being treated. For instance, this may be the amount of peptidomimetic macrocycle of the present invention necessary to activate p53 by inhibiting its binding to MDM2 and MDMX. It may also refer to the amount or dose of a chemotherapy agent or radiation administered to a subject that has cancer that is commonly administered to the subject to treat the cancer.

**"Treat"** or **"treating"** as used herein means to administer a therapeutic agent, such as a composition containing any of peptidomimetic macrocycles of the present invention, internally or externally to a subject or patient having one or more disease symptoms, or being suspected of having a disease, for which the agent has therapeutic activity or prophylactic activity. Typically, the agent is administered in an amount effective to alleviate one or more disease symptoms in the treated subject or population, whether by inducing the regression of or inhibiting the progression of such symptom(s) by any clinically measurable degree. The amount of a therapeutic agent that is effective to alleviate any particular disease symptom may vary according to factors such as the disease state, age, and weight of the patient, and the ability of the drug to elicit a desired response in the subject. Whether a disease symptom has been alleviated can be assessed by any clinical measurement typically used by physicians or other skilled healthcare providers to assess the severity or progression status of that symptom. The term further includes a postponement of development of the symptoms associated with a disorder and/or a reduction in the severity of the symptoms of such disorder. The terms further include ameliorating existing uncontrolled or unwanted symptoms, preventing additional symptoms, and ameliorating or preventing the underlying causes of such symptoms. Thus, the terms denote that a beneficial result has been conferred on a human or animal subject with a disorder, disease or symptom, or with the potential to develop such a disorder, disease or symptom.

**"Treatment"** as it applies to a human or veterinary individual, as used herein refers to therapeutic treatment, which encompasses contact of a peptidomimetic macrocycle of the present invention to a human or animal individual who is in need of treatment with the peptidomimetic macrocycle of the present invention.

*P53 Activating Peptidomimetic Macrocycles*

The present invention includes a hydrocarbon staple or stitch into an all-D configuration α-amino acid peptide inhibitor of the p53 - MDM2/MDMX interaction. $^{D}$PMI-δ, which is an all-D linear peptide having the amino acid sequence set forth in SEQ ID NO:1, was derived from a mirror image phage display screen reported by Liu et al. [41]. Specifically, they reported several 12-mer D-peptide antagonists of MDM2 (termed $^{D}$PMI-α, β, γ) that bind with affinities as low as 35 nM and are resistant to proteolytic degradation. $^{D}$PMI-δ is a corresponding analogue that was modified with two unnatural amino acids (6-F-$^{D}$Trp3 and *p*-$CF_3$-$^{D}$Phe7) to improve the MDM2 binding $K_d$ to 220 pM [51]. Crystal structures [51] of the complex between this peptide and the *N*-terminal domain of MDM2 showed that the peptide was bound in a conformation similar to that adopted by the wild-type p53 peptide (the all-L amino acid peptide derived from p53). The helix, as expected, was left-handed and projected the side chains of $^{D}$Trp2, *p*-$CF_3$-$^{D}$Phe7 and $^{D}$Leu11 into the hydrophobic pocket of MDM2, in conformations similar to those adopted by the side chains of Phe19, Trp23 and Leu26 in the wild-type p53 peptide [**Fig. 1A-1B**]. However, the peptide lacked cell permeability, but was able to activate p53 in cells when delivered using nano-carriers [42].

Particular stapling modifications of $^{D}$PMI-δ resulted in peptidomimetic macrocycles with improved binding to MDM2/MDMX and imparted cell permeability to the peptide, which enabled the stapled $^{D}$PMI-δ to enter the cell and disrupt the p53 MDM2/MDMX interaction and ultimately resulting in upregulation p53 activity. Other stapling modifications resulting in cell membrane disruption or failed to activate the p53 pathway intracellularly. Further, a bicyclic (stitched) peptidomimetic macrocycle embodiment of these all-D α-amino acids peptides demonstrates superior binding and cellular properties relative to the stapled α-amino acids peptide precursors.

Exemplary stapled $^{D}$PMI-δ peptides with binding to MDM2 and MDMX, cell permeability with no detectable cell membrane disruption, and intracellular p53 activation are represented by SEQ ID Nos: 23 and 24

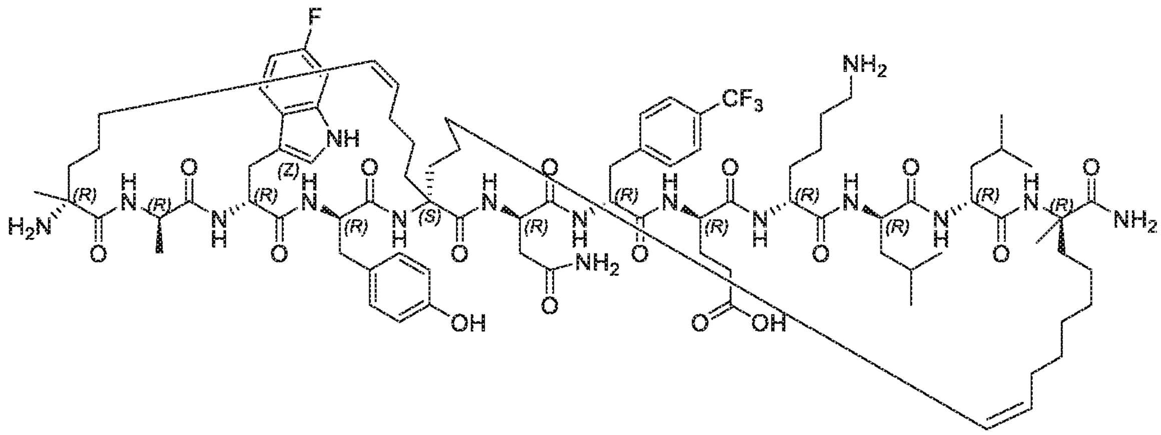

;

and

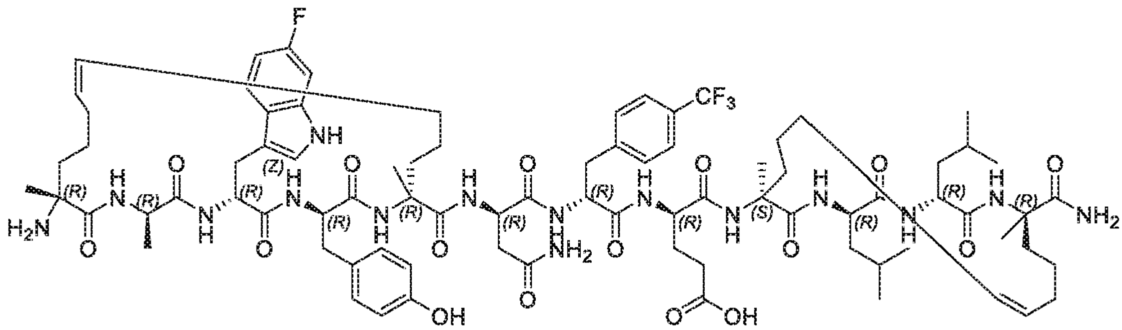

,

respectively.

The exemplary peptidomimetic macrocycles show that for $^{D}$PMI-δ and variants of the peptide, two staples or one stitch or a single staple linking positions 5 and 12 will provide a peptidomimetic macrocycle that binds MDM2 and MDMX, is protease resistant, has cellular permeability, no detectable cell membrane disruption as determined by a lactase dehydrogenase release assay (LDH) as disclosed in the Examples, and results in intracellular activation of p53.

*Pharmaceutical Compositions*

The present invention also provides pharmaceutical compositions comprising a peptidomimetic macrocycle of the present invention. The peptidomimetic macrocycle may be used in combination with any suitable pharmaceutical carrier or excipient. Such pharmaceutical compositions comprise a therapeutically effective amount of one or more peptidomimetic macrocycles, and pharmaceutically acceptable excipient(s) and/or carrier(s). The specific formulation will suit the mode of administration. In particular aspects, the pharmaceutical acceptable carrier may be water or a buffered solution.

Excipients included in the pharmaceutical compositions have different purposes depending, for example on the nature of the drug, and the mode of administration. Examples of generally used excipients include, without limitation: saline, buffered saline, dextrose, water-for-infection, glycerol, ethanol, and combinations thereof, stabilizing agents, solubilizing agents and surfactants, buffers and preservatives, tonicity agents, bulking agents, lubricating agents (such as talc or silica, and fats, such as vegetable stearin, magnesium stearate or stearic acid), emulsifiers, suspending or viscosity agents, inert diluents, fillers (such as cellulose, dibasic calcium phosphate, vegetable fats and oils, lactose, sucrose, glucose, mannitol, sorbitol, calcium carbonate, and magnesium stearate), disintegrating agents (such as crosslinked polyvinyl pyrrolidone, sodium starch glycolate, cross-linked sodium carboxymethyl cellulose), binding agents (such as starches, gelatin, cellulose, methyl cellulose or modified cellulose such as microcrystalline cellulose, hydroxypropyl cellulose, sugars such as sucrose and lactose, or sugar alcohols such as xylitol, sorbitol or maltitol, polyvinylpyrrolidone and polyethylene glycol), wetting agents, antibacterials, chelating agents, coatings (such as a cellulose film coating, synthetic polymers, shellac, corn protein zein or other polysaccharides, and gelatin), preservatives (including vitamin A, vitamin E, vitamin C, retinyl palmitate, and selenium, cysteine, methionine, citric acid and sodium citrate, and synthetic preservatives, including methyl paraben and propyl paraben), sweeteners, perfuming agents, flavoring agents, coloring agents, administration aids, and combinations thereof.

Carriers are compounds and substances that improve and/or prolong the delivery of an active ingredient to a subject in the context of a pharmaceutical composition. Carrier may serve to prolong the in vivo activity of a drug or slow the release of the drug in a subject, using controlled-release technologies. Carriers may also decrease drug metabolism in a subject and/or reduce the toxicity of the drug. Carrier can also be used to target the delivery of the drug to particular cells or tissues in a subject. Common carriers (both hydrophilic and hydrophobic carriers) include fat emulsions, lipids, PEGylated phospholipids, PEGylated liposomes, PEGylated liposomes coated via a PEG spacer with a cyclic RGD peptide c(RGD$^{DYK}$), liposomes and lipospheres, microspheres (including those made of biodegradable polymers or albumin), polymer matrices, biocompatible polymers, protein-DNA complexes, protein conjugates, erythrocytes, vesicles, nanoparticles, and side-chains for hydro-carbon stapling. The aforementioned carriers can also be used to increase cell membrane permeability of the peptidomimetic macrocycles of the invention. In addition to their use in the pharmaceutical compositions of the present invention, carriers may also be used in compositions for other uses, such as research uses in vitro (e.g., for delivery to cultured cells) and/or in vivo.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Suitable excipients for tablets or hard gelatin capsules include lactose, maize starch or derivatives thereof, stearic acid or salts thereof. Suitable excipients for use with soft gelatin capsules include for example vegetable oils, waxes, fats, semi-solid, or liquid polyols etc. For the preparation of solutions and syrups, excipients which may be used include for example water, polyols and sugars. For the preparation of suspensions oils, e.g. vegetable oils, may be used to provide oil-in-water or water in oil suspensions. In certain situations, delayed release preparations may be advantageous and compositions which can deliver the peptidomimetic macrocycles in a delayed or controlled release manner may also be prepared. Prolonged gastric residence brings with it the problem of degradation by the enzymes present in the stomach and so enteric-coated capsules may also be prepared by standard techniques in the art where the active substance for release lower down in the gastro-intestinal tract.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical compositions adapted for topical administration may be formulated as ointments, creams, suspensions, lotions, powders, solutions, pastes, gels, sprays, aerosols or oils. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e., by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurized aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water-for-injection, alcohols, polyols, glycerin and vegetable oils, for example. The compositions may be presented in unit-dose or multi-dose containers, for example sealed ampoules and vials, and may be stored in a freeze-dried (lyophilized) condition requiring only the addition of the sterile liquid carrier, for example water or saline for injections, immediately prior to use. Extemporaneous injection solutions and suspensions may be prepared from sterile powders, granules and tablets. The pharmaceutical compositions may contain preserving agents, solubilizing agents, stabilizing agents, wetting agents, emulsifiers, sweeteners, colorants, odorants, salts (substances of the present invention may themselves be provided in the form of a pharmaceutically acceptable salt), buffers, coating agents or antioxidants. They may also contain therapeutically-active agents in addition to the substance of the present invention.

The pharmaceutical compositions may be administered in a convenient manner such as by the topical, intravenous, intraperitoneal, intramuscular, intratumor, subcutaneous, intranasal or intradermal routes. The pharmaceutical compositions are administered in an amount which is effective for treating and/or prophylaxis of the specific indication. In general, the pharmaceutical compositions are administered in an amount of at least about 0.1 mg/kg to about 100 mg/kg body weight. In most cases, the dosage is from about 10 mg/kg to about 1 mg/kg body weight daily, taking into account the routes of administration, symptoms, etc.

Dosages of the peptidomimetic macrocycles of the present invention can vary between wide limits, depending upon the location, source, identity, extent and severity of the cancer, the age and condition of the individual to be treated, etc. A physician will ultimately determine appropriate dosages to be used.

The peptidomimetic macrocycles may also be employed in accordance with the present invention by expression of the antagonists in vivo, i.e., via gene therapy. The use of the peptides or compositions in a gene therapy setting is also considered to be a type of "administration" of the peptides for the purposes of the present invention.

Accordingly, the present invention also relates to methods of treating a subject having cancer, comprising administering to the subject a pharmaceutically-effective amount of one or more peptidomimetic macrocycle of the present invention, or a pharmaceutical composition comprising one or more of the antagonists to a subject needing treatment. The term "cancer" is intended to be broadly interpreted and it encompasses all aspects of abnormal cell growth and/or cell division. Examples include: carcinoma, including but not limited to adenocarcinoma, squamous cell carcinoma, adenosquamous carcinoma, anaplastic carcinoma, large cell carcinoma, small cell carcinoma, and cancer of the skin, breast, prostate, bladder, vagina, cervix, uterus, liver, kidney, pancreas, spleen, lung, trachea, bronchi, colon, small intestine, stomach, esophagus, gall bladder; sarcoma, including but not limited to chondrosarcoma, Ewing's sarcoma, malignant hemangioendothelioma, malignant schwannoma, osteosarcoma, soft tissue sarcoma, and cancers of bone, cartilage, fat, muscle, vascular, and hematopoietic tissues; lymphoma and leukemia, including but not limited to mature B cell neoplasms, such as chronic lymphocytic leukemia/small lymphocytic lymphoma, B-cell prolymphocytic leukemia, lymphomas, and plasma cell neoplasms, mature T cell and natural killer (NK) cell neoplasms, such as T cell prolymphocytic leukemia, T cell large granular lymphocytic leukemia, aggressive NK cell leukemia, and adult T cell leukemia/lymphoma, Hodgkin lymphomas, and immunodeficiency-associated lymphoproliferative disorders; germ cell tumors, including but not limited to testicular and ovarian cancer; blastoma, including but not limited to hepatoblastoma, medulloblastoma, nephroblastoma, neuroblastoma, pancreatoblastoma, leuropulmonary blastoma and retinoblastoma. The term also encompasses benign tumors.

In each of the embodiments of the present invention, the individual or subject receiving treatment is a human or non-human animal, e.g., a non-human primate, bird, horse, cow, goat, sheep, a companion animal, such as a dog, cat or rodent, or other mammal. In some embodiments, the subject is a human.

The invention also provides a kit comprising one or more containers filled with one or more of the ingredients of the pharmaceutical compositions of the invention, such as a container filled with a pharmaceutical composition comprising a peptidomimetic macrocycle of the present invention and a pharmaceutically acceptable carrier or diluent. Associated with such container(s) can be a notice in the form prescribed by a governmental agency regulating the manufacture, use or sale of pharmaceuticals or biological products, which notice reflects approval by the agency of manufacture, use or sale for human administration. In addition, the pharmaceutical compositions may be employed in conjunction with other therapeutic compounds.

*Combination therapy comprising chemotherapy*

The peptidomimetic macrocycle of the present invention may be administered to an individual having a cancer in combination with chemotherapy. The individual may undergo the chemotherapy at the same time the individual is administered the peptidomimetic macrocycle. The individual may undergo chemotherapy after the individual has completed a course of treatment with the peptidomimetic macrocycle. The individual may be administered the peptidomimetic macrocycle after the individual has completed a course of treatment with a chemotherapy agent. The combination therapy of the present invention may also be administered to an individual having recurrent or metastatic cancer with disease progression or relapse cancer and who is undergoing chemotherapy or who has completed chemotherapy.

The chemotherapy may include a chemotherapy agent selected from the group consisting of

(i) alkylating agents, including but not limited to, bifunctional alkylators, cyclophosphamide, mechlorethamine, chlorambucil, and melphalan;
(ii) monofunctional alkylators, including but not limited to, dacarbazine, nitrosoureas, and temozolomide (oral dacarbazine);
(iii) anthracyclines, including but not limited to, daunorubicin, doxorubicin, epirubicin, idarubicin, mitoxantrone, and valrubicin;
(iv) cytoskeletal disruptors (taxanes), including but not limited to, paclitaxel, docetaxel, abraxane, and taxotere;
(v) epothilones, including but not limited to, ixabepilone, and utidelone;
(vi) histone deacetylase inhibitors, including but not limited to, vorinostat, and romidepsin;

(vii) inhibitors of topoisomerase i, including but not limited to, irinotecan, and topotecan;
(viii) inhibitors of topoisomerase ii, including but not limited to, etoposide, teniposide, and tafluposide;
(ix) kinase inhibitors, including but not limited to, bortezomib, erlotinib, gefitinib, imatinib, vemurafenib, and vismodegib;
(x) nucleotide analogs and precursor analogs, including but not limited to, azacitidine, azathioprine, fluoropyrimidines (e.g., such as capecitabine, carmofur, doxifluridine, fluorouracil, and tegafur) cytarabine, , gemcitabine, hydroxyurea, mercaptopurine, methotrexate, and tioguanine (formerly thioguanine);
(xi) peptide antibiotics, including but not limited to, bleomycin and actinomycin; a platinum-based agent, including but not limited to, carboplatin, cisplatin, and oxaliplatin;
(xii) retinoids, including but not limited to, tretinoin, alitretinoin, and bexarotene;
and (xiii) vinca alkaloids and derivatives, including but not limited to, vinblastine, vincristine, vindesine, and vinorelbine.

Selecting a dose of the chemotherapy agent for chemotherapy depends on several factors, including the serum or tissue turnover rate of the entity, the level of symptoms, the immunogenicity of the entity, and the accessibility of the target cells, tissue or organ in the individual being treated. The dose of the additional therapeutic agent should be an amount that provides an acceptable level of side effects. Accordingly, the dose amount and dosing frequency of each additional therapeutic agent will depend in part on the particular therapeutic agent, the severity of the cancer being treated, and patient characteristics. Guidance in selecting appropriate doses of antibodies, cytokines, and small molecules are available. *See, e.g.*, Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602; Physicians' Desk Reference 2003 (Physicians' Desk Reference, 57th Ed); Medical Economics Company; ISBN: 1563634457; 57th edition (November 2002). Determination of the appropriate dose regimen may be made by the clinician, *e.g.*, using parameters or factors known or suspected in the art to affect treatment or predicted to affect treatment, and will depend, for example, the individual's clinical history (e.g., previous therapy), the type and stage of the cancer to be treated and biomarkers of response to one or more of the therapeutic agents in the combination therapy.

The present invention contemplates embodiments of the combination therapy that include a chemotherapy step comprising platinum-containing chemotherapy, pemetrexed and platinum chemotherapy or carboplatin and either paclitaxel or nab-paclitaxel. In particular embodiments, the combination therapy with a chemotherapy step may be used for treating at least NSCLC and HNSCC.

The combination therapy may be used for the treatment any proliferative disease, in particular, treatment of cancer. In particular embodiments, the combination therapy of the present invention may be used to treat melanoma, non-small cell lung cancer, head and neck cancer, urothelial cancer, breast cancer, gastrointestinal cancer, multiple myeloma, hepatocellular cancer, non-Hodgkin lymphoma, renal cancer, Hodgkin lymphoma, mesothelioma, ovarian cancer, small cell lung cancer, esophageal cancer, anal cancer, biliary tract cancer, colorectal cancer, cervical cancer, thyroid cancer, or salivary cancer.

In another embodiment, the combination therapy may be used to treat pancreatic cancer, bronchus cancer, prostate cancer, pancreatic cancer, stomach cancer, ovarian cancer, urinary bladder cancer, brain or central nervous system cancer, peripheral nervous system cancer, uterine or endometrial cancer, cancer of the oral cavity or pharynx, liver cancer, kidney cancer, testicular cancer, biliary tract cancer, small bowel or appendix cancer, adrenal gland cancer, osteosarcoma, chondrosarcoma, or cancer of hematological tissues.

In particular embodiments, the combination therapy may be used to treat one or more cancers selected from melanoma (metastatic or unresectable), primary mediastinal large B-cell lymphoma (PMBCL), urothelial carcinoma, MSIFIC, gastric cancer, cervical cancer, hepatocellular carcinoma (HCC), Merkel cell carcinoma (MCC), renal cell carcinoma (including advanced), and cutaneous squamous carcinoma.

*Additional Combination Therapies*

The peptidomimetic macrocycles disclosed herein may be used in combination with other therapies. For example, the combination therapy may include a composition comprising a peptidomimetic macrocycle co-formulated with, and/or co-administered with, one or more additional therapeutic agents, e.g., hormone treatment, vaccines, and/or other immunotherapies. In other embodiments, the peptidomimetic macrocycle is administered in combination with other therapeutic treatment modalities, including surgery, radiation, cryosurgery, and/or thermotherapy. Such combination therapies may advantageously utilize lower dosages of the administered therapeutic agents, thus avoiding possible

toxicities or complications associated with the various monotherapies.

By "in combination with," it is not intended to imply that the therapy or the therapeutic agents must be administered at the same time and/or formulated for delivery together, although these methods of delivery are within the scope described herein. The peptidomimetic macrocycle may be administered concurrently with, prior to, or subsequent to, one or more other additional therapies or therapeutic agents. The peptidomimetic macrocycle and the other agent or therapeutic protocol may be administered in any order. In general, each agent will be administered at a dose and/or on a time schedule determined for that agent. In will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually.

In certain embodiments, a peptidomimetic macrocycle described herein is administered in combination with one or more check point inhibitors or antagonists of programmed death receptor 1 (PD-1) or its ligand PD-L1 and PD-L2. The inhibitor or antagonist may be an antibody, an antigen binding fragment, an immunoadhesin, a fusion protein, or oligopeptide. In some embodiments, the anti-PD-1 antibody is chosen from nivolumab (OPDIVO, Bristol Myers Squibb, New York, New York), pembrolizumab (KEYTRUDA, Merck Sharp & Dohme Corp, Kenilworth, NJ USA), cetiplimab (Regeneron, Tarrytown, NY) or pidilizumab (CT-011). In some embodiments, the PD-1 inhibitor is an immunoadhesin (e.g., an immunocadhesin comprising an extracellular or PD-1 binding portion of PD-L1 or PD-L2 fused to a constant region (e.g., an Fc region of an immunoglobulin sequence)). In some embodiments, the PD-1 inhibitor is AMP-224. In some embodiments, the PD-L1 inhibitor is anti-PD-L1 antibody such durvalumab (IMFINZI, Astrazeneca, Wilmington, DE), atezolizumab (TECENTRIQ, Roche, Zurich, CH), or avelumab (BAVENCIO, EMD Serono, Billerica, MA). In some embodiments, the anti-PD-L1 binding antagonist is chosen from YW243.55.S70, MPDL3280A, MEDI-4736, MSB-0010718C, or MDX-1105,

The following examples are intended to promote a further understanding of the present invention.

## GENERAL METHODS

Available crystal structure of the linear $^{D}$PMI ($^{D}$PMI-$\delta$) peptide co-crystalized with MDM4 [pdb 3PTX] [51] was used to model the stapled (single and double) and stitched peptides. All these models were subjected to MD simulations for further refinement. MD simulations were carried out on the free peptide and peptide - MDM2 complexes. The Xleap module of AMBER16 [68] was used to prepare the system for the MD simulations. Hydrogen atoms were added and the *N*-terminus, *C*-terminus of the peptide was capped with the residue ACE and NHE. The parameters for the staple linkers were taken from our previous study [53]. All the simulation systems were neutralized with appropriate numbers of counter ions. The neutralized system was solvated in an octahedral box with TIP3P [69] water molecules, leaving at least 10 Å between the solute atoms and the borders of the box. MD simulations were carried out with the pemed module of the AMBER 16 package in combination with the ff14SB force field [70]. All MD simulations were carried out in explicit solvent at 300K. During all the simulations the long-range electrostatic interactions were treated with the particle mesh Ewald [71] method using a real space cut off distance of 9 Å. The settle [72] algorithm was used to constrain bond vibrations involving hydrogen atoms, which allowed a time step of 2 fs during the simulations. Solvent molecules and counter ions were initially relaxed using energy minimization with restraints on the protein and peptide atoms. This was followed by unrestrained energy minimization to remove any steric clashes. Subsequently the system was gradually heated from 0 to 300 K using MD simulations with positional restraints (force constant: 50 kcal $mol^{-1}$ $Å^{-2}$) on protein and peptides over a period of 0.25 ns allowing water molecules and ions to move freely followed by gradual removal of the positional restraints and a 2ns unrestrained equilibration at 300 K. The resulting systems were used as starting structures for the respective production phase of the MD simulations. For each case, three independent (using different initial random velocities) MD simulations were carried out starting from the well equilibrated structures. Each MD simulation was carried out for 250 ns and conformations were recorded every 4ps. To enhance the conformational sampling, each of these peptides were subjected to Biasing Potential Replica Exchange MD (BP-REMD) simulations. The BP-REMD technique is a type of Hamiltonian -REMD methods which includes a biasing potential that promote dihedral transitions along the replicas [55,56]. For each system, BP-REMD was carried with eight replicas including a reference replica without any bias. BP-REMD was carried out for 50ns with exchange between the neighbouring replicas were attempted for every 2ps and accepted or rejected according to the metropolis criteria. Conformations sampled at the reference replica (no bias) was used for further analysis. Simulation trajectories were visualized using VMD [73] and figures were generated using Pymol [74].

### Binding Energy calculations and energy decomposition analysis:

Molecular Mechanics Poisson Boltzmann Surface Area (MMPBSA) methods were used for the calculation of

binding free energies between the peptides and their partner proteins 250 conformations extracted from the last 50 ns of the simulations were used for the binding energy calculations. Entropy calculations are computationally intensive and do not converge easily and hence are ignored. The effective binding energies were decomposed into contributions of individual residues using the MMGBSA energy decomposition scheme. The MMGBSA calculations were carried out in the same way as in the MMPBSA calculations. The polar contribution to the solvation free energy was determined by applying the generalized born (GB) method (igb =2) [68], using mbondi2 radii. The non-polar contributions were estimated using the ICOSA method [68] by a solvent accessible surface area (SASA) dependent term using a surface tension proportionally constant of 0.0072 kcal/mol Å2. The contribution of peptide residues was additionally explored by carrying out in-silico alanine scanning in which each of the peptide residue is mutated to D-alanine in each conformation of the MD simulation and the change with respect to the binding energy of the wild type peptide is calculated using MMPBSA.

**Peptide Synthesis**

Peptides were synthesized using RINK Resin and Fmoc-protected amino acids, coupled sequentially with FIC/HOBT activating agents. Double coupling reactions were performed on the first amino acid and also at the stapling positions. At these latter positions, the activating reagents were switched to DIEA/HATU for better coupling efficiencies. Ring closing metathesis reactions were performed by first washing the resin 3 times with DCM, followed by addition of the 1$^{st}$ generation Grubbs Catalyst (35 mg dissolved into 5 mL DCM) and allowed to react for 2 hours (all steps with Grubbs Catalyst were performed in the dark). The ring-closing metathesis (RCM) reaction was repeated to ensure a complete reaction. After the RCM was complete a test cleavage was performed to ensure adequate yield. Peptides were cleaved and then purified with RP-HPLC.

**MDM2 Protein production**

A human MDM2 1-125 sequence was cloned into a pNIC-GST vector. The TV cleavage site was changed from ENLYFQS (SEQ ID NO: 13) to ENLYFQG (SEQ ID NO: 14) to give a fusion protein with the following sequence:

MSDKIIHSPILGYWKIKGLVQPTRLLLEYLEEKYEEHLYERDEGDKWRNKKFELGLEFPN
LPYYIDGDVKLTQSMAIIRYIADKHNMLGGCPKERAEISMLEGAVLDIRYGVSRIAYSKD
FETLKVDFLSKLPEMLKMFEDRLCHKTYLNGDHVTHPDFMLYDALDVVLYMDPMCLD
AFPKLVCFKKRIEAIPQIDKYLKSSKYIAWPLQGWQATFGGGDHPPKLEVLFQGHMHHH
HHHSSGVDLGTENLYFQGMCNTNMSVPTDGAVTTSQIPASEQETLVRPKPLLLKLLKSV
GAQKDTYTMKEVLFYLGQYIMTKRLYDEKQQHIVYCSNDLLGDLFGVPSFSVKEHRKI
YTMIYRNLVVVNQQESSDSGTSVSEN (SEQ ID NO: 12)

The corresponding plasmid was transformed into was BL21 (DE3) Rosetta T1R *E. coli* cells and grown under kanamycin selection. Bottles of 750 mL TERRIFIC BROTH supplemented with appropriate antibiotics and 100 μL of antifoam 204 (Sigma-Aldrich) were inoculated with 20 mL seed cultures grown overnight. The cultures were incubated at 37°C in the LEX system (Harbinger Biotech) with aeration and agitation through the bubbling of filtered air through the cultures. LEX system temperature was reduced to 18°C when culture OD600 reached 2, and the cultures were induced after 60 minutes with 0.5mM IPTG. Protein expression was allowed to continue overnight. Cells were harvested by centrifugation at 4000 g, at 15°C for 10min. The supernatants were discarded and the cell pellets were resuspended in lysis buffer (1.5 mL per gram of cell pellet). The cell suspensions were stored at -80°C before purification work. The re-suspended cell pellet suspensions were thawed and sonicated (Sonics Vibra-cell) at 70% amplitude, 3s on/off for 3 minutes, on ice. The lysate was clarified by centrifugation at 47000g, 4°C for 25 minutes. The supernatants were filtered through 1.2um syringe filters and loaded onto AKTA Xpress system (GE Healthcare). The purification regime is briefly described as follows. The lysates were loaded on to 1 mL Ni-NTA Superflow column (Qiagen) that had been equilibrated with 10 column volumes of wash 1 buffer. Overall buffer condition were as follows: IMAC wash 1 buffer: 20 mM HEPES, 500 mM NaCl, 10 mM Imidazole, 10 % (v/v) glycerol, 0.5 mM TCEP, pH 7.5; IMAC wash 2 buffer: 20 mM HEPES, 500 mM NaCl, 25 mM Imidazole, 10 % (v/v) glycerol, 0.5 mM TCEP, pH 7.5; IMAC Elution buffer: 20 mM HEPES, 500 mM NaCl, 500 mM Imidazole, 10 % (v/v) glycerol, 0.5 mM TCEP, pH 7.5. The sample was loaded until air was detected by air sensor, 0.8mL/minutes. The column was then washed with wash 1 buffer for 20 column volumes followed by 20 column volumes of wash 2 buffer. The protein was eluted with 5 column volumes of elution buffer. The eluted proteins were collected and stored in sample loops on the system and then injected into Gel Filtration (GF) columns. Elution peaks were collected in 2mL fractions and analyzed on SDS-PAGE gels. The entire purification was performed at 4° C. Relevant peaks were

pooled, TCEP was added to a total concentration of 2 mM. The protein sample was concentrated in Vivaspin 20 filter concentrators (VivaScience) at 15°C to approximately 15mg/mL. (< 18kDa - 5K MWCO, 19-49kDa - 10K MWCO, >50kDa - 30K MWCO). The final protein concentration was assessed by measuring absorbance at 280nm on Nanodrop ND-1000 (Nano-Drop Technologies). The final protein purity was assessed on SDS-PAGE gel. The final protein batch was then aliquoted into smaller fractions, frozen in liquid nitrogen and stored at -80°C.

**MDM4 protein production**

MDM4 protein was cloned into pNIC-GST vector and expressed in LEX system (Harbinger Biotech) at Protein Production Platform (PPP) at NTU School of Biological Sciences. Using glycerol stocks, inoculation cultures were started in 20 mL TERRIFIC BROTH with 8g/L glycerol supplemented with Kanamycin. The cultures were incubated at 37 °C, 200rpm overnight. The following morning, bottles of 750 mL Terrific Broth with 8g/L glycerol supplemented with Kanamycin and 100 μL of antifoam 204 (Sigma-Aldrich) were inoculated with the cultures. The cultures were incubated at 37 °C in the LEX system with aeration and agitation through the bubbling of filtered air through the cultures. When the OD600 reached ~2, the temperature was reduced to 18°C and the cultures were induced after 30 to 60 minutes with 0.5mM IPTG. Protein expression was allowed to continue overnight. The following morning, cells were harvested by centrifugation at 4200 rpm at 15°C for 10 minutes. The supernatants were discarded and the cells were re-suspended in lysis buffer (100 mM HEPES, 500 mM NaCl, 10 mM Imidazole, 10 % glycerol, 0.5 mM TCEP, pH 8.0 with Benzonase (4uL per 750mL cultivation) and 250U/μL Merck Protease Inhibitor Cocktail Set III, EDTA free (1000x dilution in lysis buffer) from Calbiochem) at 200 rpm, 4°C for approximately 30min and stored at -80°C. The re-suspended cell pellet suspensions were thawed and sonicated (Sonics Vibra-cell) at 70 % amplitude, 3s on/off for 3minutes, on ice. The lysate was clarified by centrifugation at 47000g, 4°C for 25 minutes. The supernatants were filtered through 1.2 μm syringe filters and loaded onto AKTA Xpress system (GE Healthcare) with a 1mL Ni-NTA Superflow (Qiagen) IMAC column. The column was washed with 20 column volume (CV) of wash buffer 1 (20 mM HEPES, 500 mM NaCl, 10 mM Imidazole, 10 % (v/v) glycerol, 0.5 mM TCEP, pH 7.5) and 20 CV of wash buffer 2 (20 mM HEPES, 500 mM NaCl, 25 mM Imidazole, 10 % (v/v) glycerol, 0.5 mM TCEP, pH 7.5) or until a stable baseline for 3 min and delta base 5mAU (0.8mL/min) was obtained respectively. MDM4 protein was eluted with elution buffer (20 mM HEPES, 500 mM NaCl, 500 mM Imidazole, 10 % (v/v) glycerol, 0.5 mM TCEP, pH 7.5) and eluted peaks (start collection: >50mAU, slope >200mAU/minutes, stop collection: <50mAU, stable plateau of 0.5min, delta plateau 5mAU) were collected and stored in sample loops on the system and then injected into equilibrated Gel Filtration (GF) column (HiLoad 16/60 Superdex 200 prep grade (GE Healthcare)) and eluted with 20 mM HEPES, 300 mM NaCl, 10% (v/v) glycerol, 0.5 mM TCEP, pH 7.5 at a flowrate of 1.2mL/minutes. Elution peaks (start collection: >20mAU, slope >10mAU/min, stop collection: < 20mAU, slope >10mAU/minutes, minimum peak width 0.5min) were collected in 2 mL fractions. The entire purification was performed at 4 °C. Relevant peaks were pooled and TCEP was added to a final concentration of 2 mM. The protein sample was concentrated in Vivaspin 20 filter concentrators (VivaScience) at 15°C to approximately 15mg/mL. The final protein concentration was assessed by measuring absorbance at 280nm on Nanodrop ND-1000 (Nano-Drop Technologies). The final protein purity was assessed by SDS-PAGE and purified MDM4 protein was frozen in liquid nitrogen and stored at - 80°C.

**Circular Dichroism (CD)**

Prior to the experiment, 5 μl of the 10 mM stock peptide was mixed with 45 μl of 100 % methanol, and dried for 2 hours in the SpeedVac concentrator (Thermo Scientific). The dried peptide was reconstituted in buffer, containing 1 mM HEPES pH 7.4 and 5 % methanol, to a concentration of 1 mM. The peptide sample was placed in a quartz cuvette with a path length of 0.2 cm and the CD spectrum was recorded from 300 to 190 nm at 25°C, using the Chirascan-plus qCD machine (Applied Photophysics). The actual concentration of the peptide was determined by the absorbance of the peptide at 280 nM. An estimate of the secondary structure components of the peptide was carried out by converting the CD spectrum to mean residue ellipticity, before deconvoluting using the CDNN software (distributed by Applied Photophysics). All experiments were done in duplicates.

**Isothermal Titration Calorimetry (ITC)**

All experiments were performed in duplicates using the MicroCal PEAQ-ITC Automated system. 100-200 μM of peptide was titrated into 20 μM of purified recombinant human MDM2 protein (amino acids 1-125), over 40 injections of 1 μL each. For peptides that are insoluble at high concentrations, reverse ITC was carried out by titrating 200 μM of MDM2 protein into 20 μM of peptide. All protein and peptides were dialyzed overnight in buffer containing 1 x phosphate-buffered saline (PBS) pH 7.2, 3 % DMSO, and 0.001 % Tween-20. Data analysis was carried out using the MicroCal PEAQ-ITC Analysis Software.

**Surface plasmon resonance (SPR)**

SPR experiments were performed with Biacore T100 (GE Healthcare) at 25°C. The site-specific mono-biotinylated MDM2 were prepared by sortase-mediated ligation. SPR buffer consisted of PBS pH 7.2, 1 mM DTT, 0.01% Tween 20, and 3% DMSO. The CM5 chip was first conditioned with 100 mM HCl, followed by 0.1% SDS, 50 mM NaOH and then water, all performed twice with 6 second injection at a flow rate of 100 μL/min. With the flow rate set to 10 μL/minutes, streptavidin (S4762, Sigma-Aldrich) was immobilized on the conditioned chip through amine coupling as described in the Biacore manual. Excess protein was removed by 30 second injection of the wash solution (50 mM NaOH + 1 M NaCl) for at least 8 times. The immobilized levels are 3400-3700 RU. The biotinylated MDM2 were captured to the streptavidin, to a level of ~400 RU for MDM2. Flow cell consisted of only streptavidin was used as the reference surface. Using a flow rate of 30 μL/minutes, peptides dissolved in the SPR buffer are injected for 180 sec. The dissociation was monitored for 300 seconds. For multi-cycle kinetics, each peptide injection is followed by a similar injection of SPR buffer to allow the surface to be fully regenerated. For single-cycle kinetics, five peptide concentrations were injected consecutively for 180 seconds, followed by a 4000 second dissociation. Similar injection of SPR buffer was followed to allow the surface to be regenerated, though not completely. After the run, responses from the target protein surface are transformed by (i) correcting with DMSO calibration curve, (ii) subtracting the responses obtained from the reference surface, and (iii) subtracting the responses of buffer injections from those of peptide injections. The last step is known as double referencing which corrects the systematic artefacts. The resulting responses were subjected to kinetic analysis by global fitting with 1:1 binding model.

**MDM2 Binding Assay**

Purified MDM2 (1-125) protein was titrated against 50 nM carboxyfluorescein (FAM)-labeled 12/1 peptide13 (FAM-RFMDYWEGL-NH2). Dissociation constants for titration of MDM2 against FAM-labeled 12/1 peptide were determined by fitting the experimental data to a 1:1 binding model equation shown below:

Equation 1:

$$r = r_o + (r_b - r_o) \times \frac{(K_d + [L]_t + [P]_t) - \sqrt{(K_d + [L]_t + [P]_t)^2 - 4[L]_t[P]_t}}{2[L]_t}$$

[P] is the protein concentration (MDM2), [L] is the labeled peptide concentration, r is the anisotropy measured, r0 is the anisotropy of the free peptide, rb is the anisotropy of the MDM2-FAM-labeled peptide complex, Kd is the dissociation constant, [L]t is the total FAM labeled peptide concentration, and [P]t is the total MDM2 concentration. The determined apparent Kd value of FAM-labeled 12/1 peptide (13.0 nM) was used to determine the apparent Kd values of the respective competing ligands in subsequent competition assays in fluorescence anisotropy experiments. Titrations were carried out with the concentration of MDM2 held constant at 250 nM and the labeled peptide at 50 nM. The competing molecules were then titrated against the complex of the FAM-labeled peptide and protein. Apparent Kd values were determined by fitting the experimental data to the equations shown below:

$$r = r_o + (r_b + r_o) \times \frac{2\sqrt{(d^2 - 3e)}\cos(\theta/3) - 9}{3K_{d1} + 2\sqrt{(d^2 - 3e)}\cos(\theta/3) - d}$$

$$d = K_{d1} + K_{d2} + [L]_{st} + [L]_t - [P]_t$$

$$e = ([L]_t - [P]_t)K_{d1} + ([L]_{st} - [P]_t)K_{d2} + K_{d1}K_{d2}$$

$$f = -K_{d1}K_{d2}[P]_t$$

$$\theta = ar\cos\left[\frac{-2d^3 + 9de - 27f}{2\sqrt{(d^2 - 3e)^3}}\right]$$

[L]st and [L]t denote labeled ligand and total unlabeled ligand input concentrations, respectively. Kd2 is the dissociation constant of the interaction between the unlabeled ligand and the protein. In all competition experiments, it is assumed that [P]t > [L]st, otherwise considerable amounts of free labeled ligand would always be present and would interfere with measurements. Kd1 is the apparent Kd for the labeled peptide used and has been experimentally determined as described in the previous paragraph. The FAM-labeled peptide was dissolved in dimethyl sulfoxide (DMSO) at 1 mM and diluted into experimental buffer. Readings were carried out with an Envision Multilabel Reader (PerkinElmer). Experiments were carried out in PBS (2.7 mM KCl, 137mM NaCl, 10 mM $Na_2HPO_4$ and 2 mM $KH_2PO_4$ (pH 7.4)) and 0.1% Tween 20 buffer. All titrations were carried out in triplicate. Curve-fitting was carried out using Prism 4.0 (GraphPad). To validate the fitting of a 1:1 binding model we carefully ensured that the anisotropy value at the beginning of the direct titrations between MDM2 and the FAM-labeled peptide did not differ significantly from the anisotropy value observed for the free fluorescently labeled peptide. Negative control titrations of the ligands under investigation were also carried out with the fluorescently labeled peptide (in the absence of MDM2) to ensure no interactions were occurring between the ligands and the FAM-labeled peptide. In addition, we ensured that the final baseline in the competitive titrations did not fall below the anisotropy value for the free FAM-labeled peptide, which would otherwise indicate an unintended interaction between the ligand and the FAM-labeled peptide to be displaced from the MDM2 binding site.

**p53 Beta-lactamase Reporter Gene Assay**

HCT116 cells were stably transfected with a p53 responsive β-lactamase reporter, were seeded into a 384-well plate at a density of 8,000 cells per well. Cells were maintained in McCoy's 5A Medium with 10% fetal bovine serum (FBS), Blasticidin and Penicillin/Streptomycin. The cells were incubated overnight and followed by removal of cell growth media and replaced with Opti-MEM either containing 0% FBS or 10% FBS. Peptides were then dispensed to each well using a liquid handler, ECHO 555 and incubated for 4/16 hours. Final working concentration of DMSO was 0.5%. β-lactamase activity was detected using the ToxBLAzer Dual Screen (Invitrogen) as per manufacturer's instructions. Measurements were done using Envision multiplate reader (Perkin-Elmer). Maximum p53 activity was defined as the amount of β-lactamase activity induced by 50 μM azide-ATSP-7041 (stapled p53 peptide; Aileron Therapeutics, Inc.). This was determined as the highest amount of p53 activity induced by azide-ATSP-7041 by titration on HCT116 cells.

**Lactate Dehydrogenase Release Assay**

HCT116 cells were seeded into a 384-well plate at a density of 8000 cells per well. Cells were maintained in McCoy's 5A Medium with 10% fetal bovine serum (FBS), Blasticidin and Penicillin Streptomycin. The cells were incubated overnight followed by removal of cell media and addition of Opti-MEM Medium without FBS. Cells were then treated with peptides for 4/16 hours in Opti-MEM either in 10% FBS or serum free. Final concentration of DMSO was 0.5%. Lactate dehydrogenase release was detected using CytoTox-ONE Homogenous Membrane Integrity Assay Kit (Promega) as per manufacturer's instructions. Measurements were carried out using Tecan plate reader. Maximum LDH release was defined as the amount of LDH released induced by the lytic peptide (iDNA79) and used to normalize the results.

**Tetracycline Beta-lactamase Reporter Gene Assay (Counterscreen)**

Based on Jump-In ™ T-REx™ CHO-K1 BLA cells and contain a stably integrated β-lactamase under the control of an inducible CMV promoter. Cells were seeded into a 384-well plate a density of 4000 cells per well. Cells were maintained in Dulbecco's Minimal Eagle Medium (DMEM) with 10% fetal bovine serum (FBS), Blasticidin and Penicillin/Streptomycin. The cells were incubated for 24 hours, followed by cell media removal and replacement with Opti-MEM either containing 10% FBS or 0% FBS. Peptides were then dispensed to each well using a liquid handler, ECHO 555 and incubated for 4/16 hours. Final working concentration of DMSO was 0.5%. β-lactamase activity was detected using the ToxBLAzer Dual Screen (Invitrogen) as per manufacturer's instructions. Measurements were carried out using Envision multiplate reader (Perkin-Elmer). Counterscreen activity was defined as the amount of β-lactamase activity induced by tetracycline.

**HCT-116 Western blot analysis**

**Preparation of compound Stock and working Solutions**: 10 mM or 1 mM stock solutions of compounds were prepared in 100% DMSO. Each compound was then serially diluted in 100% DMSO and further diluted 10-fold into HPLC grade sterile water to prepare 10X working solutions in 10% DMSO/water of each compound. Depending on the required volume used in the relevant assay, compounds were added to yield final concentrations as indicated in the relevant figure with a residual DMSO concentration of 1% v/v.

HCT116 cells (Thermo Fisher Scientific) were cultured in DMEM cell media, which was supplemented with 10% foetal calf serum (FBS) and penicillin/streptomycin. All cell lines were maintained in a 37°C humidified incubator with 5% $CO_2$ atmosphere. HCT116 cells were seeded into 96 well plates at a cell density of 60,000 cells per well and incubated overnight. Cells were also maintained in DMEM cell media with 10% fetal bovine serum (FBS) and penicillin/streptomycin. Cell media was then removed and replaced with cell media containing the various compounds/vehicle controls at the concentrations indicated in DMEM cell media with 2% FCS. After the stated incubation time (4 or 24 hours) cells were rinsed with PBS and then harvested in 100 $\mu$l of 1x NuPAGE LDS sample buffer supplied by Invitrogen (NP0008). Samples were then sonicated, heated to 90 °C for 5 minutes, sonicated twice for 10 seconds and centrifuged at 13, 000 rpm for 5 minutes. Protein concentrations were measured by BCA assay (Pierce). Samples were resolved on Tris-Glycine 4-20% gradient gels (BIORAD) according to the manufacturer's protocol. Western transfer was performed with an Immuno-blot PVDF membrane (Bio-Rad) using a Trans-Blot Turbo system (BIORAD). Western blot staining was then performed using antibodies against actin (AC-15, Sigma) as a loading control, p21 (118 mouse monoclonal), MDM2 (2A9 mouse monoclonal antibody) and p53 (DO-1 mouse monoclonal antibody).

EXAMPLE 1

**Conformational landscape of** $^D$PMI-$\delta$ **peptide in apo and MDM2-bound states.**

We sought to rationally design stapled $^D$PMI-$\delta$ analogues that would stabilize helical structure and preserve or enhance binding affinities. Accordingly, we applied molecular dynamics (MD) simulations to the published co-crystal structure of the MDM2-$^D$PMI-$\delta$ complex to understand its structural details critical for the maintenance of the binding motif. During the simulation, the bound conformation of the $^D$PMI-$\delta$ peptide remained stable with an RMSD of < 2Å relative to its starting conformation [**Fig. 2A**]. The bound $^D$PMI-$\delta$ peptide retained its crystallographic $\alpha$-helical conformation throughout the simulation (>95% $\alpha$-helicity). The peptide bound state of MDM2 also remained stable with an RMSD of < 2Å [**Fig. 2B**]. The bound conformation of the peptide is stabilized by hydrogen bonds and hydrophobic interactions. A hydrogen bond observed in the crystal structure between the side chain N of 6-F-$^D$Trp3 and the backbone O of Gln72 [**Fig. 1B**], is preserved in ~80% of the simulation. Other hydrogen bonds seen in the crystal structure and reflected in the simulations but for shorter durations included i) the side chains of Gln72(MDM2) and Thr1($^D$PMI-$\delta$), ii) the side chains of Lys94/His96(MDM2) and Glu8($^D$PMI-$\delta$), iii) the side chains of His96/Tyr100(MDM2) and the backbone carbonyl of Leu11($^D$PMI-$\delta$) [**Fig. 1B**]). As expected, the three critical residues of p53, 6-F-$^D$Trp3, p-$CF_3$-$^D$Phe7 and $^D$Leu10 from $^D$PMI-$\delta$ were buried into the hydrophobic binding pocket in MDM2 [**Fig. 2C**] throughout the simulation.

Peptide design was also informed by understanding the conformational landscape of the free $^D$PMI-$\delta$ peptide in solution. Simulations were carried out starting from the bound conformation of the peptide extracted from the crystal structure of the MDM2- $^D$PMI-$\delta$ complex. Biasing Potential Replica Exchange MD (BPREMD), a Hamiltonian Replica Exchange Method that has been used successfully to explore peptide landscapes [55, 56], was used to enhance the conformational sampling of the peptide. Unsurprisingly, the free peptide exhibited increased flexibility with RMSD ranging between 2-6 Å [**Fig. 2D**]. The two peaks (3-4 Å and 6 Å) correspond to the partially folded and unfolded states of the peptide, a rapid loss in $\alpha$-helicity is seen resulting in a state where only ~21% of the sampled conformations are alpha helical. This prediction was experimentally confirmed by circular dichroism (CD) spectroscopy, which showed the peptide was ~20.4% helical in solution [**Fig. 2E**]. This was also expected and consistent with the linear peptides derived from the natural p53 sequence.

EXAMPLE 2

**Design and Synthesis of stapled D-peptides.**

Relative to the above simulations, we sought to design stapled analogues of $^D$PMI-$\delta$ that would maximize helicity in solution and maintain target binding. To identify appropriate positions on the $^D$PMI-$\delta$ peptide for the introduction of the hydrocarbon linkers, we sought to determine residues that, upon mutation, would result in minimal perturbation to the peptide-MDM2 interaction. The overall binding energy of the peptide to MDM2 during the MD simulations is decomposed into the energetic contributions of each residue of the peptide. Unsurprisingly, 6-F-$^D$Trp3, p-$CF_3$-$^D$Phe7 and $^D$Leu11 are the

major contributors to the total binding energy followed by $^{D}$Tyr4 and Leu10 [**Fig. 3A**]. The contributions from the other seven residues are either negligible or even slightly destabilizing. We next carried out computational alanine scans of the residues of the peptide by mutating each residue to D-alanine and computing the change in the binding energy for each conformation sampled during the MD simulation and averaging the changes [**Fig. 3B**]. The results mirror the residue-wise contributions [**Fig. 3A**] in that the D-alanine mutations were most deleterious at positions that contributed most, i.e. 6-F-$^{D}$Trp3 and p-$CF_3$-$^{D}$Phe7 of $^{D}$PMI-δ (> ~ 10 kcal/mol) [**Fig. 3B**] while substitutions at positions $^{D}$Tyr4, $^{D}$Glu8 and $^{D}$Leu11 resulted in loss of ~2-5 kcal/mol in the overall binding energy [**Fig. 3B**]. In contrast the other positions were quite tolerant to D-Ala substitutions. Overall, these studies suggest 7 positions where staples could be incorporated without significant perturbations to target binding. The incorporation of staples requires careful selection of sidechains with appropriate stereochemistry. As stapling of the left-handed alpha-helices that are formed by all-D peptide has not been conducted previously, we first needed to select the appropriate stereocenters. We reasoned that the stereo-centers should be a mirror-image of the standard strategies that have proven effective for stapling right-handed alpha-helices (*i.e.,* S5 to S5 for (i, i+4) linkages, and R8 to S5 for (i, i+7) linkages). Accordingly, we choose to employ R5 to R5 and S8 to R5 linkages. Using these linkages and the simulation to guide staple placement, we designed several stapled versions of $^{D}$PMI-δ (details are shown in **Fig. 4**).

EXAMPLE 3

**Peptide stapling increases helicity.**

BP-REMD simulations suggested that all of the designed stapled $^{D}$PMI-δ analogues should have increased solution-based helicity. Specifically, we predicted solution helicities between 24-39%; values that were increased compared to the predicted and measured values of ~21% for the unstapled parent sequence (*vida supra).* The values for the stapled analogues agreed well with those obtained experimentally via CD spectroscopy (ranging from *24.5* % to 38%)[**Fig. 5A**]. This increase in helicity upon stapling mirrors what has been reported for stapling all-L amino acid peptides [57-58].

EXAMPLE 4

**Stability and binding affinity are improved upon peptide stapling.**

We next carried out MD simulations of the stapled $^{D}$PMI-δ peptides bound to MDM2. Using the linear $^{D}$PMI-δ peptide/MDM2 co-crystal structure as a starting point, staples were modelled into the all-D peptide at six sets of residues and subject to MD simulations. The stapled peptides remained stable during the MD simulations and remained largely (~95%) helical. The three critical residues 6-F-$^{D}$Trp3, p-$CF_3$-$^{D}$Phe and $^{D}$Leu11 remained buried in the hydrophobic pocket/binding site of MDM2 [**Fig. 6**]. The hydrocarbon linkers remained largely exposed to solvent without engaging the MDM2 surface; this contrasts with some of the L-amino acid stapled peptides where the staples contributed to the binding by engaging with the surface of MDM2 [52-53].

Next, the ability of these peptides to bind MDM2 was measured using fluorescence polarization [FP], surface plasmon resonance (SPR), and isothermal titration calorimetry [ITC] experiments. For the binding assays, MDM2 (residues 17-125) was used in conjunction with linear and stapled $^{D}$PMI-δ peptides. We used a stapled all-L-peptide, ATSP-7041 [59], a validated MDM2 binder, as a positive control and found that it binds strongly to MDM2 with Kd 2 nM in this version of our FP assay. In our hands, the linear $^{D}$PMI-δ peptide displayed strong affinity for MDM2 with Kd of 36 nM. Two of the six stapled $^{D}$PMI-δ peptides displayed strong affinity towards MDM2, two stapled peptides $^{D}$PMI-δ*(1-5)* and $^{D}$PMI-δ*(5-12)* binding with Kd of 13 and 39 nM respectively. In contrast, peptides $^{D}$PMI-δ*(2-6)*, $^{D}$PMI-δ*(5-9)*, $^{D}$PMI-δ*(6-10)* and $^{D}$PMI-δ*(2-9)* displayed no binding in the FP assay (**Fig. 5B**, Table 1). The SPR binding data mirrored the FP assay, with the linear and stapled $^{D}$PMI-δ (1-5 staple, and 5-12 staple) binding strongly with Kd of < 1nM, whereas the other stapled $^{D}$PMI-δ peptides displaying reduced affinity with $^{D}$PMI-δ (2-6) as a non-binder (**Table 1**).

**Table 1**
Secondary structure, binding and cellular activity of stapled, double stapled and stitched DPMI-δ peptides determined through various biophysical and biochemical methods.

| Peptide | CD (% helicity) | FP Kd (nM) MDM2 | FP Kd (nM) MDM4 | SPR Kd (nM) | P53 activity $EC_{50}$ (μM) (2% FBS) | LDH release $EC_{50}$ (μM) (2% FBS) | Counter-screen $EC_{50}$ (μM) (2% FBS) |
|---|---|---|---|---|---|---|---|
| | | | | | 16H | 16H | 16H |
| DPMI-δ | 20.4 | 36.2 | 43.8 | <1 | 50 | 50 | 50 |
| DPMI-δ(*1-5*) | 24.7 | 13.2 | 4.5 | <1 | 13.7 | 24 | 50 |
| DPMT-δ (*2-6*) | 46.7 | 10000 | 10000 | >500 | 50 | 17.7 | 13.9 |
| DPMI-(*2-9*) | 26.5 | 3350 | 10000 | 7.4 | 50 | 50 | 50 |
| DPMI-δ(*5-9*) | 38.0 | 10000 | 10000 | 29 | 50 | 46.5 | 47 |
| DPMI-δ(*6-10*) | 39.9 | 10000 | 10000 | 59 | 30.3 | 18.5 | 39 |
| DPMI-δ(*5-12*) | 31.8 | 38.75 | 29.5 | <1 | 21.3 | 50 | 50 |
| DPMI-δ(*1-5-12*) (*scrambled*) | 28 | 10000 | 10000 | >500 | 50 | 50 | 50 |
| DPMI-δ(*1-5-12*) | 52.1 | 4.25 | 12.5 | <1 | 4 | 50 | 50 |
| DPMI-δ(*1-5*, *9-12*) | 20 | 0.7 | 2.1 | <1 | 34.6 | 50 | 50 |
| ATSP-7041 | 49.6 | 49.2 | 4.5 | <1 | 1.8 | 50 | 50 |

Models provided an explanation for the lack of binding of DPMI-δ*(2-6) and* DPMI-δ*(2-9)*; a key hydrogen bond between the backbone of 6-floro-DTrp at position 3 and the sidechain of Q72 is lost when the residue at position 2 is replaced with a stapled linker as the alpha-methyl interferes. Our models are unable to demonstrate whether this is a kinetic effect or a thermodynamic effect; nevertheless, ATSP-7041 is also known to lose affinity when Thr2 is mutated to either amino-isobutyric (Aib) or N-methyl Threonine.

The 1:1 stoichiometric binding was further confirmed by Isothermal titration calorimetry (ITC) experiments. Both the linear and stapled DPMI-δ peptides bound to MDM2 with (ΔG = ~ -10 kcal/mol) [**Table 1**], with the enthalpy of binding (ΔH) ranges from -15.8 kcal/mol for the DPMI-δ*(1-5)* to -8.25 kcal/mol for the DPMI-δ*(5-12)*. Both the linear DPMI-δ peptide and DPMI-δ*(1-5)* stapled peptide less helical in solution, had a favorable enthalpy contribution for the binding. The favorable enthalpy compensates for the entropic penalty paid as the disordered peptide gets ordered during binding to MDM2. On the other hand, stapled peptides DPMI-δ*(5-12)* which had increased helicity (34% helicity) has favorable entropic contributions for the binding that compensate for the loss in favorable enthalpy contributions and therefore the binding is retained.

Next the proteolytic stabilities of the stapled and unstapled DPMI-δ peptides were investigated by incubating these molecules in whole cell homogenate. As expected, the all-D configuration α-amino acids composition rendered all peptides (linear and stapled) resistant to proteolytic degradation. Specifically, >90% of each peptide remained detectable in the homogenate during the 4-hour incubation time (**Fig. 10**). Small decreases in peptide concentrations over time were attributed to sample loss due to binding to labware and instrument surfaces rather than through proteolysis.

EXAMPLE 5

**Cellular uptake of stapled all-D configuration α-amino acid peptides.**

To investigate the effect of peptide stapling in the cellular context, linear and stapled DPMI-δ peptides were added to HCT116 cells with a stably transfected p53-responsive β-lactamase reporter gene. After 16 hours of peptide incubation, no p53 activation was observed for the linear DPMI-δ peptide, even at the high concentration tested (50μM). In contrast, three of the six stapled DPMI-δ peptides showed dose responsive increases in p53 activity, while the other three were inactive across the range of peptide concentrations tested (**Fig. 7A**).

Cellular activity correlated well with the biophysical and biochemical data. Stapled peptides DPMI-δ*(1-5)* and DPMI-δ*(5-12)* bound MDM2 well (with Kds of 13 and 39 nM respectively from FP assay) and also demonstrated measurable cellular activation of p53 with $EC_{50}$s of 17.5 and 23.2 μM respectively [**Table 1**]. While these peptides clearly cross the cell membrane in order to activate p53, peptides DPMI-δ*(5-9),* DPMI-δ*(2-6)* and DPMI-δ*(2-9)* were unable to

activate cellular p53, perhaps due their lack of binding affinity (Kd in μM range). Interestingly peptide $^{D}$PMI-δ*(6-10)* a non-binder of MDM2 (from FP assay) demonstrated measurable cellular activation with $EC_{50}$ of 30.3μM.

EXAMPLE 6

**Membrane distribution and counterscreen activity of stapled all-D configuration α-amino acid peptides.**

Macrocyclic peptides that are cell permeable are often hydrophobic in nature [60], a property that can impart an ability to disrupt the outer membrane and result in cellular leakage [61]. To assess whether the results from our p53 reporter assay [**Fig. 7A**] were potentially compromised by membrane damage, we carried out a membrane integrity assay (Lactate dehydrogenase release, LDH) **[Fig. 7B]** under identical conditions to our p53 cellular assay. The linear $^{D}$PMI-δ peptide which did not show any p53 cellular activity **[Fig. 7A]** also did not show any LDH leakage at concentrations as high as 50μM **[Fig. 7B].** The stapled peptides, $^{D}$PMI-δ*(2-9)* and $^{D}$PMI-δ*(5-9)* also did not cause LDH leakage, even at even at concentrations as high as 50μM. The stapled peptide $^{D}$PMI-δ*(*2-9) which was weak binder in biochemical assays and did not result in any cell activity also did not cause LDH release, suggesting that it is cell impermeable. Interestingly the most cell active stapled peptide $^{D}$PMI-δ*(5-12)*, didn't cause any LDH leakage **[Fig. 7B],** suggesting that the activity observed in the p53 receptor activation assay is through intracellular target engagement and or has the appropriate secondary structure to minimize cell lysis. Stapled peptides $^{D}$PMI-δ*(1-5)*, $^{D}$PMI-δ*(2-6)* and $^{D}$PMI-δ*(6-10)* all caused LDH leakage with $EC_{50}$ of 24μM, 17.7 μM and 18.5 μM respectively [see **Fig. 7B].** Peptide $^{D}$PMI-δ*(2-6)*, a non-binder of MDM2 and without any measurable cell activity, induced LDH leakage with $EC_{50}$ 17.7 μM. Peptides $^{D}$PMI-δ*(6-10)*, a non-binder of MDM2 and $^{D}$PMI-δ*(1-5)* a potent binder of MDM2 with cell efficacy, also resulted in considerable LDH leakage. In fact, the $EC_{50}$ observed in the p53 reporter activity assays is similar to the $EC_{50}$ observed for the LDH leakage assays, indicating that these two stapled peptides cause membrane disruption and the readout of the p53 reporter assay may not be due to intracellular target engagement but instead due to the nonspecific cytotoxicity resulting from plasma membrane lysis. Thus, we demonstrate that $^{D}$PMI-δ*(5-12)* enters the cells without membrane disruption, engages the MDM2:p53 complex, resulting in p53 reporter activity.

To further validate intracellular target engagement, we carried out a counterscreen assay with an identical reporter gene but one whose expression is independent of p53 activation. Most of the stapled $^{D}$PMI-δ peptides including the linear peptide had $EC_{50}$ values > 50μM **[Fig. 7C].** For the linear $^{D}$PMI-δ peptide and stapled peptides $^{D}$PMI-δ *(5-9)* and $^{D}$PMI-δ*(2-9)*, this result was unsurprising as these molecules appear to be cell impermeable. Interestingly, stapled peptides $^{D}$PMI-δ*(1-5)* and $^{D}$PMI-δ*(6-10)* each demonstrate significant activity in the reporter assay and LDH leakage assay and didn't exhibit activity or were weakly active ($^{D}$PMI-δ*(6-10)* 39μM) in counterscreen assay. $^{D}$PMI-δ*(5-12)* displayed negligible cytotoxicity with $EC_{50}$ > 50μM, suggesting that it is not cytotoxic and acts mechanistically through disruption of the intracellular MDM2-p53 complex.

EXAMPLE 7

**Design, synthesis, binding and cellular activity of double staple and stitched peptides.**

Encouraged by the results stapled $^{D}$PMI-δ peptides, with particular interest in $^{D}$PMI-δ*(5-12)* which showed on-target cellular activity without confounding activities in the LDH release or counterscreen assays, we wonder whether incorporation of an additional staple would confer further improvements in binding and cellular activity. Recent studies have highlighted the limitations of peptides carrying single staples including low cell permeability, low proteolytic stability and low cellular activity and have shown that these can be overcome with the introduction of an additional staple [39, 40, 62]. In such bicyclic arrangements, two pairs of hydrocarbon stapling residues are incorporated into a single peptide sequence. To avoid any cross reactivity during olefin metathesis, sufficient spacing between the two pairs of non-natural amino acid staple precursors are required, often resulting in a longer peptide sequence.

Several double-stapled peptides have been shown to successfully inhibit pathways in HIV-1 [39], Ral GTP-ase [63], Rab8a GTP-ase [64], estrogen receptor-α [65], Respiratory Syncytial Virus Entry [40, 62] and BCL9 [66]. All these peptides exhibited increased helicity, increased proteolytic resistance and increased binding as compared to the corresponding single stapled peptides. Some of these double-stapled peptides even demonstrated enhanced cell permeability. Double-stapled peptides can also be designed with a common attachment/anchoring point and peptides with such contiguous hydrocarbon staples are also referred to as "stitched" peptides [67]. Recently XYZ reported the synthesis of stitched peptides using RCM reactions that exhibited improvements in thermal and chemical stability, proteolytic stability and cell permeability [66]. From the six stapled peptides designed, we found that two, $^{D}$PMI-δ*(1-5)* and $^{D}$PMI-δ*(5-12)*, both exhibited improved binding and cellular properties. We introduced an addition staple between positions 9 and 12 (i+3) in $^{D}$PMI-δ*(1-5)* resulting in a $^{D}$PMI-δ*(1-5,9-12)* double stapled peptide **[Fig. 8A].** Combining $^{D}$PMI-δ*(1-5)* and $^{D}$PMI-δ*(5*-12) resulted in a stitched peptide, $^{D}$PMI-δ*(1,5,12)*, with the common attachment point for the

two staples localized at residue 5 **[Figs. 8A].** As expected the CD spectra of the stitched $^{D}$PMI-δ*(1,5,12)* showed increased helicity (52% helicity, **Table 1).** This agrees with reports on other peptides showing that the stitched and double stapled peptides often display increased helicity compared to the single stapled peptides [39, 40 , 62-67]. In contrast the double staple, peptide didn't enhance helicity in $^{D}$PMI-δ*(1-5,9-12)*.

Both the double-stapled $^{D}$PMI-δ*(1-5,9-12)* and stitched bicyclic peptides $^{D}$PMI-δ*(1,5,12)* bound MDM2 with Kd of 4 and 0.7 nM in FP assay, a 10- to 100-fold increase in affinity as compared to $^{D}$PMI-δ*(1-5)* and $^{D}$PMI-δ*(5-12)* **[Fig. 8C, Table 1].** This confirms that the additional staple enhances the target engagement of these peptides. $^{D}$PMI-δ*(1,5,12)* displayed enhanced cellular activity with $EC_{50}$ of 4 μM, at 16 hours, a five-fold increase compared to the single stapled peptides. Similar increases in cell permeability for a stitched peptide have been reported earlier [66]. However, the double-stapled peptide $^{D}$PMI-δ*(1-5,9-12)* didn't have significant cellular activity with an $EC_{50}$ of 34.6 μM, at 16 hours. Although recent studies have reported that the double-stapled peptides appear to follow the same trend as their single-stapled counterparts [39, 40, 62-65], lack of cell activity observed for the double-stapled peptide here, demonstrate that enhanced cellular activity is not uniform. Although the molecular mechanisms behind the increased cell permeability of the stitched and double-stapled D-peptides are unclear, it could be attributed to the increased conformational rigidity and/or increased hydrocarbon content of these peptides. No detectable LDH leakage was observed, even at concentrations as high as 50 μM [**Fig. 8E**], and there was negligible counter screen activity [**Fig.** 8F], confirming that the designed double stapled $^{D}$PMI-δ*(1-5,9-12)* and stitched peptides $^{D}$PMI-δ*(1,5,12)* enter cells without membrane disruption and result in the activation of p53 by inhibiting the MDM2-p53 complex. The intracellular target engagement of stitched $^{D}$PMI-δ*(1,5,12)* peptide as further validated using western-blot analysis **[Fig. 11].** Stabilization of MDM2 and activation of p53 was observed for $^{D}$PMI-δ(1,5,12) and ATSP-7041 (stapled p53 peptide; Aileron Therapeutics, Inc.), whereas the $^{D}$PMI-δ linear peptide failed to do so.

EXAMPLE 8

**Dual inhibition by stapled and stitched all-D configuration α-amino acid peptides.**

MDMX is homologous to MDM2 and is also a negative regulator of p53, often found overexpressed in some cancer cells. Studies have shown that dual inhibition (of MDM2 and MDMX) appears to be critical for full activation of p53-dependent tumor suppression. Thus, we were interested to know if the all-D configuration α-amino acid peptides had dual-inhibitory properties. As a control, the single stapled peptide ATSP-7041, a validated MDM2/MDMX binder, was observed to bind to MDMX with Kd 4.5 nM. The structure of MDMX is highly similar to MDM2 **[Fig. 9A**], so we generated a model of the $^{D}$PMI-δ peptide bound to a structure of MDMX using the $^{D}$PMI-δ:MDM2 structure as template. Models of the stapled/-stitched $^{D}$PMI-δ peptide bound to MDMX were generated by incorporating appropriate linkers in the $^{D}$PMI-δ:MDMX structure. We next carried out MD simulations of the (un)stapled/stitched $^{D}$PMI-δ peptides bound to MDMX. The stapled peptides remained stable during the MD simulations and remained largely (~95%) helical. The three critical residues 6-F-$^{D}$Trp3, p-$CF_3$-$^{D}$Phe and $^{D}$Leu11 remained buried in the hydrophobic pocket/binding site of MDMX [**Fig. 9B].** The hydrocarbon linkers remained largely exposed to solvent without engaging the MDMX surface. The binding of $^{D}$PMI-δ peptides with MDMX was further confirmed by FP assay [**Fig. 9C**].

The MDMX binding data mirrored the MDM2 binding FP assay, with the linear and stapled $^{D}$PMI-δ (1-5 staple, and 5-12 staple) binding strongly with Kd of 43.8 nM, 4.5 nM and 29.5 nM respectively. The other four stapled peptides $^{D}$PMI-δ *(2-6)*, $^{D}$PMI-δ*(5-9)*, $^{D}$PMI-δ *(2-9)*, $^{D}$PMI-δ*(6-10)* displayed no binding **(Table 2)** in the FP assay. Both the stitched $^{D}$PMI-δ (1,5,12) and double-stapled $^{D}$PMI-δ (1-5, 9-12) peptides displayed strong affinity towards MDMX, with Kd of 12.5 and 2. 1 nM respectively. In conclusion, the stitched $^{D}$PMI-δ (1,5,12) and double-stapled $^{D}$PMI-δ (1-5, 9-12) peptides are high affinity dual inhibitors of MDM2 and MDMX.

**Table 2:**

Binding of stapled & stitched $^{D}$PMI-δ peptides with MDM2 protein determined through SPR and ITC experiments.

| Peptide | SPR Kd (nM) | ITC (kcal/mol) | | |
|---|---|---|---|---|
| | | ΔH | TΔS | ΔG |
| $^{D}$PMI-δ | <1 | 13.8 | 3.04 | -10.8 |
| $^{D}$PMI-δ*(1-5)* | <1 | -15.8 | 3.04 | -11.6 |
| $^{D}$PMI-δ(*2-6*) | >500 | n.d. | n.d. | n.d. |
| $^{D}$PMI-(*2-9*) | 7.4 | n.d. | n.d. | n.d. |
| $^{D}$PMI-δ(*5-9*) | 29 | n.d. | n.d. | n.d. |

(continued)

| Table 2: Binding of stapled & stitched $^{D}$PMI-δ peptides with MDM2 protein determined through SPR and ITC experiments. | | | | |
|---|---|---|---|---|
| Peptide | SPR Kd (nM) | ITC (kcal/mol) | | |
| | | ΔH | TΔS | ΔG |
| $^{D}$PMI-δ*(5-12)* | <1 | -8.25 | -1.83 | -10.1 |
| $^{D}$PMI-δ(*6-10*) | 59 | -9.35 | -1.86 | -11.2 |
| $^{D}$PMI-δ(*1-5-12*) | <1 | n.d. | n.d. | n.d. |
| $^{D}$PMI-δ(*1-5, 9-12*) | <1 | n.d. | n.d. | n.d. |
| ATSP-7041 | <1 | n.d. | n.d. | n.d. |

EXAMPLE 9

**Summary of results shown in Examples 1-8.**

Peptide based inhibitors are remerging as next generation therapeutic modalities because of high target specificity, high biocompatibility and low toxicity. However, liabilities such as conformational stability, proteolytic sensitivity and cell permeability hinder their potential. Peptide stapling to constrain peptides in its active/bound conformation results in several benefits such as improved stability, target binding and cell permeability. Although stapling L-amino acid peptides can confer resistance to protease-mediated degradation, the effect is often not complete, especially for residues located outside of the staple. On the other hand, D-amino acid peptides show complete resistance to proteolysis, increased stability and bioavailability, hence appear to be suitable for oral administration. Unfortunately, just like most linear peptides, all-D peptides generally lack membrane permeability and cellular activity.

We reasoned that a combination of the two strategies (i.e., all-D configuration α-amino acids and stapling) might provide a robust molecule satisfying all the required criteria for intracellular target engagement. Accordingly, we embarked on introducing a hydrocarbon staple into an all-D α-amino acid peptide inhibitor of the p53:MDM2/MDMX interaction that had been discovered using mirror - image phage display [41]. Guided by the available crystal structure of $^{D}$PMI-δ bound to the *N*-terminal domain of MDM2, we designed six stapled $^{D}$PMI-δ peptides using a combination of modelling and molecular simulations. All six stapled peptides displayed helicity ranging from 24% to 45% which compared with ~21% for the linear counterpart. Two peptides demonstrated increased affinity for MDM2 in biophysical and biochemical experiments. These peptides also showed enhanced cell uptake without detectable membrane disruption and disrupted the MDM2-p53 complex, leading to activation of p53. No correlation was apparent between helicity and binding as was also reported for L-amino acid peptides or with cell activity.

We next decided to introduce a second staple generating a double stapled peptide and a stitched peptide. The stitched peptide displayed the highest helicity (52%) while double stapled peptide remained unchanged at 20%), similar to the linear peptide. Nevertheless, both these peptides displayed increased binding to MDM2, suggesting that the binding mechanism of these peptides are different from each other. However only the stitched peptide displayed increased cellular activity, probably due to increased cell permeability while the double stapled peptide appears unable to cross the cell membrane. Although stapling resulted in increased helicity, increased affinity and more importantly enables cell permeability, it is not clear how this latter is achieved. Increased hydrophobicity resulting from the hydrocarbon linker of the stitched peptide could be a major driving force, however the lack of permeability for the double stapled peptide which has a longer hydrophobic linker casts doubt on the hydrophobicity - permeability link. Therefore, understanding cell permeability warrants further studies to systematically investigate the factors enabling cell permeability of these peptides.

While stapling appeared to impart cell permeability, it also resulted in membrane disruption by some of the peptides. Curiously, while all the stapled and stitched peptides displayed reporter activity at 4 hours, only four peptides (1-5, 5-12, 6-10 and 1,5,12 stitched) continued to show activity at 16 hours. However, the 6-10 stapled peptide is known to not bind MDM2 and is also known to cause membrane disruption (from LDH release assays) and hence it likely results in activation of p53 due to membrane disruption. At the same time, the 1-5 stapled peptide, which is a potent binder of MDM2, also causes LDH leakage and hence it is unclear what results in p53 activation: target engagement or membrane disruption, likely some combination of the two.

A counterscreen assay, with an identical read out to the primary cellular screen but that is independent of p53 activation, was carried out to find peptides with off-target effects and we found that 6-10 which is a non-binder of MDM2, yet activated p53 even at 16 hours, appears to have off-target effects. The 2-6 stapled peptide, also a non-binder of MDM2, caused membrane disruption, showed counter screen activity (which could result from membrane disruption or off-target

activity). In contrast the 5-12 stapled and the 1,5,12 stitched peptide showed no membrane disruption and off target activity, activating p53 through intracellular target engagement. The on-target engagement of the stapled and stitched peptide was further validated in western blot assays. Thus, it is important to use a combination of LDH leakage, counter screen assays and target engagement/reporter activation to rule out false positives (that result from off-target engagement and membrane disruption).

Stapling also enabled the peptide to bind to MDMX with high affinity, The MDMX binding data mirrored the MDM2 data, resulting in a cell permeable dual inhibitor of MDM2/MDMX. It is possible that in the activation assays, binding to MDMX likely contributes to p53 activation. Several studies have shown that dual inhibition of MDM2 and MDMX appears to be critical for full activation of p53-dependent tumour suppression.

In conclusion, by stapling all-D configuration α-amino acids peptides, the examples demonstrate that stapling can enhance both binding and cellular properties of all-D linear peptides, as has been reported for the L-amino acid peptides. The use of all-D-peptides leveraging intrinsic stability and macrocyclization as described here imparts enhanced target binding and cellular activity to advance a novel stapled peptide modality having significant therapeutic potential for p53-dependent cancers.

REFERENCES

1. Petta I., Lievens S., Libert C., Tavernier J., De Bosscher K. Modulation of Protein Protein Interactions for the Development of Novel Therapeutics. Mol. Ther. 2015;24:707-718.

2. Macalino S.J.Y., Basith S., Clavio N.A.B., Chang H., Kang S., Choi S. Evolution of In Silico Strategies for Protein-Protein Interaction Drug Discovery. Molecules. 2018;23:1963

3. Scott, D.E., Bayly, A.R., Abell, C. and Skidmore, J. (2016) Small molecules, big targets: drug discovery faces the protein-protein interaction challenge. Nat. Rev. Drug Discov. 15, 533-550

*4.* Nevola L, Giralt E. Modulating protein protein interactions: the potential of peptides. Chem Commun. 2015;51:3302-15.

5. Lau, J. L.; Dunn, M. K. Therapeutic peptides: Historical perspectives, current development trends, and future directions Bioorg. Med. Chem. 2017, 26, 2700-2707

6. Fosgerau, K.; Hoffmann, T. Peptide therapeutics: current status and future directions Drug Discovery Today 2015, 20, 122- 128

7. Bakail M., Ochsenbein F. Targeting protein-protein interactions, a wide open field for drug design. Comptes Rendus Chimie. 2016, 19, 19-27

8. A. Henninot, J.C. Collins, J.M. Nuss. The current state of peptide drug discovery: back to the future J. Med. Chem., 61 (2018), pp. 1382-1414

9. Morrison C., Constrained peptides' time to shine?. Nature Reviews Drug Discovery volume 17, pages 531-533 (2018)

10. Valeur E., et al. New Modalities for Challenging Targets in Drug Discovery. Angew. Chem. Int. Ed. 2017, 56, 10294 - 10323

11. Cary, D. R.; Ohuchi, M.; Reid, P. C.; Masuya, K. Constrained Peptides in Drug Discovery and Development. Yuki Gosei Kagaku Kyokaishi 2017, 75, 1171- 1178,

12. Vinogradov A., Macrocyclic Peptides as Drug Candidates: Recent Progress and Remaining Challenges. J. Am. Chem. Soc., 2019, 141 (10), pp 4167-4181.

13. Sawyer, T. K. Macrocyclic α helical peptide therapeutic modality: A perspective of learnings and challenges. Bioorg. Med. Chem. 2018, 26, 2807- 2815

14. L.D. Walensky, G.H. Bird Hydrocarbon-stapled peptides: principles, practice, and progress J Med Chem, 57 (2014), pp. 6275-6288,

15. Y.S. Tan, D.P. Lane, C.S. Verma Stapled peptide design: principles and roles of computation Drug Discov Today, 21 (2016), pp. 1642-1653

16. Ali AM., et al. Stapled Peptides Inhibitors: A New Window for Target Drug Discovery. Computational and Structural Biotechnology Journal, 2019, 17, 263-281.

17. Klein M., Stabilized helical peptides: overview of the technologies and its impact on drug discovery. Expert Opinion on Drug Discovery. 2017, 12, 1117-1125.

18. Lerge J et al. Stapled peptides as a new technology to investigate protein-protein interactions in human platelets. Chem Sci, 2108, 9, 4638-4643.

19. Xu W., Macrocyclized Extended Peptides: Inhibiting the Substrate-Recognition Domain of Tankyrase. J. Am. Chem. Soc., 2017, 139 (6), pp 2245-2256

20. Wiedmann, MM., et al. Development of Cell-Permeable ,Non-Helical Constrained Peptides to Target a Key

Protein-Protein Interaction in Ovarian Cancer. Angew .Chem. Int.Ed. 2017 , 56 ,524 -529
21. L. D. Walensky, A. L. Kung, I. Escher, T. J. Malia, S. Barbuto, R. D. Wright, G. Wagner, G. L. Verdine and S. J. Korsmeyer. Activation of apoptosis in vivo by a hydrocarbon-stapled BH3 helix. Science, 2004, 305, 1466-1470.
22. S. A. Kawamoto, A. Coleska, X. Ran, H. Yi, C. Y. Yang and S. Wang. Design of triazole-stapled BCL9 α-helical peptides to target the β-catenin/B-cell CLL/lymphoma 9 (BCL9) protein-protein interaction, J.Med. Chem., 2012, 55, 1137-1146
23. K. Takada, Di Zhu, G. H. Bird, K. Sukhdeo, J.-J. Zhao, M. Mani, M. Lemieux, D. E. Carrasco, J. Ryan,
24. D. Horst, M. Fulciniti, N. C. Munshi, W. Xu, A. L. Kung, R. A. Shivdasani, L. D. Walensky and D. R. Carrasco. Targeted disruption of the BCL9/β-catenin complex inhibits oncogenic Wnt signaling. Sci. Transl. Med., 2012, 4, 148ra117.
25. L Dietrich, B Rathmer, K Ewan, T Bange, S Heinrichs, TC Dale, D Schade, TN Grossmann. Cell permeable stapled peptide inhibitor of Wnt signaling that targets β-catenin protein-protein interactions. Cell Chem. Biol., 24, 958-968 (2017).
26. Spiegel J, Cromm PM, Itzen A, Goody RS, Grossmann TN, Waldmann H. Direct targeting of Rab-GTPase-effector interactions. Angew Chem Int Ed Engl. 2014 Feb 24:53(9):2498-503
27. Xie M., et al. Structural Basis of Inhibition of ERα-Coactivator Interaction by High-Affinity N-Terminus Isoaspartic Acid Tethered Helical Peptides. J. Med. Chem., 2017, 60 (21), pp 8731-8740.
28. Paola de., et al. Cullin3-BTB interface: a novel target for stapled peptides. PLoS One. 2015 Apr 7;10(4):e0121149,
29. Misawa T., et al. Structural development of stapled short helical peptides as vitamin D receptor (VDR)-coactivator interaction inhibitors. Bioorg Med Chem. 2015 Mar 1;23(5):1055-61,
30. Lama D., et al, Structural insights reveal a recognition feature for tailoring hydrocarbon stapled-peptides against the eukaryotic translation initiation factor 4E protein. Chemical Science. 2019, 10, 2489-2500
31. F. Bemal, A. F. Tyler, S. J. Korsmeyer, L. D. Walensky and G. L. Verdine. Reactivation of the p53 tumor suppressor pathway by a stapled p53 peptide. J. Am. Chem. Soc., 2007, 129, 2456-2457.
32. L. K. Henchey, J. R. Porter, I. Ghosh and P. S. Arora. High specificity in protein recognition by hydrogen-bond-surrogate a-helices: selective inhibition of the p53/MDM2 complex. Chembiochem., 2010, 11, 2104-2107
33. C. J. Brown, S. T., Quah, J. Jong, A. M. Goh, P. C., Chiam, K. H. Khoo, M. L. Choong, M. A. Lee, L. Yurlova, K. Zolghadr, T. L. Joseph, C. S. Verma and D. P. Lane. Stapled peptides with improved potency and specificity that activate p53. ACS Chem. Biol., 2013, 8, 506-512
34. Y. S. Chang, B. Graves, V. Guerlavais, C. Tovar, K. Packman, T. To, K. Olson, K. Kesavan, P. Gangurde, A. Mukherjee, T. Baker, K. Darlak, C. Elkin, Z. Filipovic, F. Z Qureshi, H. Cai, P. Berry, E. Feyfant, X. E. Shi, J. Horstick, A. Annis, N. Fotouhi, T. Manning, H. Nash, L. T. Vassilev and T. K. Sawyer. Stapled a-helical peptide drug development: a potent dual inhibitor of MDM2 and MDMX for p53-dependent cancer therapy. Proc. Natl. Acad. Sci. U.S.A, 2013, 110, E3445-E3455.
35. A. Burgess, K. M. Chia, S. Haupt, D. Thomas, Y.Haupt and E. Lim. Clinical overview of MDM2/Xtargeted therapies. Front. Oncol., 2016, 6, 1-7.
36. K. Kojima, J. Ishizawa and M. Andreeff. Pharmacological activation of wild-type p53 in the therapy of leukemia. Exp. Hematol., 2016, 44, 791-798.
37. V. Tisato, R. Voltan, A. Gonelli, P. Secchiero and G. Zauli. MDM2/X inhibitors under clinical evaluation: Perspectives for the management of hematological malignancies and pediatric cancer. J. Hematol. Oncol., 2017, 10, 133.
38. Cromm PM. Et al. Protease-Resistant and Cell-Permeable Double-Stapled Peptides Targeting the Rab8a GTPase. ACS Chem. Biol., 2016, 11 (8), pp 2375-2382
39. Bird GH., et al., Hydrocarbon double-stapling remedies the proteolytic instability of a lengthy peptide therapeutic. PNAS August 10, 2010 107 (32) 14093-14098
40. Gaillard V., et al. A Short Double-Stapled Peptide Inhibits Respiratory Syncytial Virus Entry and Spreading. Antimicrobial Agents and Chemotherapy Mar 2017, 61 (4) e02241-16
41. M. Liu, M. Pazgier, C. Li, W. Yuan, C. Li, W. Lu Angew. Chem., Int. Ed., 49 (2010), p. 3649
42. M. Liu, C. Li, M. Pazgier, C. Li, Y. Mao, Y. Lv, B. Gu, G. Wei, W. Yuan, C. Zhan, W.Y. Lu, W. Lu Proc. Natl. Acad. Sci. USA, 107 (2010), p. 14321
43. K. Mandal, M. Uppalapati, D. Ault-Riché, J. Kenney, J. Lowitz, S.S. Sidhu, S.B. Kent Proc. Natl. Acad. Sci. USA, 109 (2012), p. 14779
44. H.N. Chang, B.Y. Liu, Y.K. Qi, Y. Zhou, Y.P. Chen, K.M. Pan, W.W. Li, X.M. Zhou, W.W. Ma, C.Y. Fu, Y.M. Qi, L. Liu, Y.F. Gao Angew. Chem., Int. Ed., 54 (2015), p. 11760
45. Welch BD, Francis JN, Redman JS, Paul S, Weinstock MT, Reeves JD, Lie YS, Whitby FG, Eckert DM, Hill CP, Root MJ, Kay MS. Design of a potent D-peptide HIV-1 entry inhibitor with a strong barrier to resistance. J Virol. 2010;84(21):11235-44,
46. Lane DP p53, guardian of the genome Nature. 1992 Jul 2;358(6381):15-6.

47. ami-Schmidt, O., M. Lokshin, and C. Prives . 2016. The roles of MDM2 and MDMX in cancer. Annu. Rev. Pathol. 11:617-644
48. Dongsheng Pei,1,2 Yanping Zhang, 1,2 and Junnian Zheng1 Regulation of p53: a collaboration between MDM2 and MDMX. Oncotarget. 2012 Mar; 3(3): 228-235.
49. Tisato V1, Voltan R2, Gonelli A2, Secchiero P2, Zauli G2. MDM2/X inhibitors under clinical evaluation: perspectives for the management of hematological malignancies and pediatric cancer. J Hematol Oncol. 2017 Jul 3;10(1):133
50. F. Funda Meric-Bernstam, M. S. Saleh, J. R. Infante, S. Goel, G. S. Falchook, G. Shapiro, K. Y. Chung, R. M. Conry, D. S. Hong, J. S. Wang, U. Steidl, L. D. Walensky, V. Guerlavais, M. Payton, D. A. Annis, M. Aivado, M. R. Patel Phase I trial of a novel stapled peptide ALRN-6924 disrupting MDMX- and MDM2- mediated inhibition of WT p53 in patients with solid tumors and lymphomas. J. Clin. Oncol. 35, 2505-2505 (2017)
51. Zhan, C.; Zhao, L.; Wei, X.; Wu, X.; Chen, X.; Yuan, W.; Lu, W. Y.; Pazgier, M.; Lu, W. An ultrahigh affinity d-peptide antagonist of MDM2 J. Med. Chem. 2012, 55, 6237-6241
52. CJ Brown, ST Quah, J Jong, AM Goh, PC Chiam, KH Khoo, ML Choong, et al. Stapled peptides with improved potency and specificity that activate p53. ACS chemical biology 8 (3), 506-512
53. Tan, Y. S. et al. Benzene Probes in Molecular Dynamics Simulations Reveal Novel Binding Sites for Ligand Design. J Phys Chem Lett 7, 3452-3457,
54. D. Thean, J. S. Ebo, T. Luxton, Xue'Er Cheryl Lee, T. Y. Yuen, F. J. Ferrer, C. W. Johannes, D. P. Lane & C. J. Brown. Enhancing Specific Disruption of Intracellular Protein Complexes by Hydrocarbon Stapled Peptides Using Lipid Based Delivery. Scientific Reportsvolume 7, Article number: 1763 (2017).
55. Kannan S, Zacharias M (2007) Enhanced sampling of peptide and protein conformations using replica exchange simulations with a peptide backbone biasing-potential. Proteins. 66: 697-7006.
56. Ostermeir K, Zacharias M. Hamiltonian replica-exchange simulations with adaptive biasing of peptide backbone and side chain dihedral angles. J. Comput. Chem., 35 (2014), pp
57. Schafmeister, C. E.; Po, J.; Verdine, G. L. An all-hydrocarbon cross-linking system for enhancing the helicity and metabolic stability of peptides. J. Am. Chem. Soc. 2000, 122, 5891 - 5892.. 150-158
58. LD Walensky, GH bird, Hydrocarbon-Stapled Peptides: Principles, Practice, and Progress. J.Med.Chem. 2014, 57, 6275 - 6288
59. Y.S. Chang, B. Graves, V. Guerlavais, C. Tovar, K. Packman, T. To, K. Olson, K. Kesavan, P. Gangurde, A. Mukherjee, T. Baker, K. Darlak, C. Elkin, Z. Filipovic, F. Z Qureshi, H. Cai, P. Berry, E. Feyfant, X. E. Shi, J. Horstick, A. Annis, N. Fotouhi, T. Manning, H. Nash, L. T.Vassilev and T. K. Sawyer. Stapled a-helical peptide drug development: a potent dual inhibitor of MDM2 and MDMX for p53-dependent cancer therapy. Proc. Natl. Acad. Sci. U.S.A, 2013, 110, E3445-E3455.
60. A. Furukawa, C.E. Townsend, J. Schwochert, C.R. Pye, M.A. Bednarek, R.S. Lokey. Passive membrane permeability in cyclic peptomer scaffolds is robust to extensive variation in side chain functionality and backbone geometry. J Med Chem, 59 (2016), pp. 9503-9512
61. Li YC, et al. A versatile platform to analyze low-affinity and transient protein-protein interactions in living cells in real time. Cell Rep. 2014;9:1946-58
62. Bird GH, et al. Mucosal delivery of a double-stapled RSV peptide prevents nasopulmonary infection. J Clin Invest. 2014;124:2113-24
63. Thomas JC, Cooper JM, Clayton NS, Wang C, White MA, Abell C, Owen D, and Mott HR (2016) Inhibition of Ral GTPases using a stapled peptide approach. J Biol Chem 291:18310-18325
64. P. M. Cromm , J. Spiegel , P. Küchler , L. Dietrich , J. Kriegesmann , M. Wendt, R. S. Goody , H. Waldmann and T. N. Grossmann , ACS Chem. Biol., 2016, 11, 2375 -2382
65. Speltz T.E., Mayne C.G., Fanning S.W., Siddiqui Z., Tajkhorshid E., Greene G.L. A "cross-stitched" peptide with improved helicity and proteolytic stability. Org Biomol Chem. 2018;16:3702-3706
66. Kawamoto S.A., Coleska A., Ran X., Yi H., Yang C.Y., Wang S. Design of triazole-stapled BCL9 a-helical peptides to target the B-catenin/B-cell CLL/lymphoma 9 (BCL9) protein-protein interaction. J Med Chem. 2012;55:1137-1146.
67. Hilinski G.J., Kim Y.-W., Hong J., Kutchukian P.S., Crenshaw C.M., Berkovitch S.S. et al. 2014. Stitched $\alpha$-helical peptides via bis ring-closing metathesis. J Am Chem Soc, 2014 Sep 3:136(35):12314-22.

| Table of Sequences | | |
|---|---|---|
| SEQ ID NO: | Description | Sequence |
| 1 | $^{D}$PMI-$\delta$<br>Xaa3 is 6-F-Trp | TAXYANXEKLLR |

(continued)

| Table of Sequences | | |
|---|---|---|
| SEQ ID NO: | Description | Sequence |
| | Xaa7 is p-$CF_3$-Phe<br>Xaa1-Xaa12 all alpha-amino acids thereof have a D configuration<br>Xaa12 optionally has a C-terminal amide | |
| 2 | $^{D}$PMT-δ(1-5)<br>Xaa1 is R5<br>Xaa3 is 6-F-Trp<br>Xaa5 is R5<br>Xaa7 is p-$CF_3$-Phe<br>Xaa1-Xaa12 all alpha-amino acids thereof have a D configuration<br>Xaa12 optionally has a C-terminal amide<br>Xaa1 optionally has a *N*-terminal acyl | XAXYXNXEKLLR |
| 3 | $^{D}$PMI-δ(2-6)<br>Xaa2 is R5<br>Xaa3 is 6-F-Trp<br>Xaa6 is R5<br>Xaa7 is p-$CF_3$-Phe<br>Xaa1-Xaa12 all alpha-amino acids thereof have a D configuration<br>Xaa12 optionally has a C-terminal amide<br>Xaa1 optionally has a N-terminal acyl | TXXYAXXEKLLR |
| 4 | $^{D}$PMI-δ(2-9)<br>Xaa2 is S8<br>Xaa3 is 6-F-Trp<br>Xaa7 is p-$CF_3$-Phe<br>Xaa9 is R5<br>Xaa1-Xaa12 all alpha-amino acids thereof have a D configuration<br>Xaa12 optionally has a C-terminal amide<br>Xaa1 optionally has a N-terminal acyl | TXXYANXEXLLR |
| 5 | $^{D}$PMI-δ(5-9)<br>Xaa3 is 6-F-Trp<br>Xaa5 is R5<br>Xaa7 is p-$CF_3$-Phe<br>Xaa9 is R5<br>Xaa1-Xaa12 all alpha-amino acids thereof have a D configuration<br>Xaa12 optionally has a C-terminal amide<br>Xaa1 optionally has a N-terminal acyl | TAXYXNXEXLLR |
| 6 | $^{D}$PMT-δ(5-12)<br>Xaa3 is 6-F-Trp<br>Xaa5 is S8<br>Xaa7 is p-$CF_3$-Phe<br>Xaa12 is R5 | TAXYXNXEKLLX |

(continued)

| Table of Sequences | | |
|---|---|---|
| SEQ ID NO: | Description | Sequence |
| | Xaa1-Xaa12 all alpha-amino acids thereof have a D configuration<br>Xaa12 optionally has a C-terminal amide<br>Xaa1 optionally has a N-terminal acyl | |
| 7 | DPMI-δ(6-10)<br>Xaa3 is 6-F-Trp<br>Xaa6 is R5<br>Xaa7 is p-$CF_3$-Phe<br>Xaa10 is R5<br>Xaa1-Xaa12 all alpha-amino acids thereof have a D configuration<br>Xaa12 optionally has a C-terminal amide<br>Xaa1 optionally has a N-terminal acyl | TAXYAXXEKXLR |
| 8 | DPMI-δ(1,5,12)<br>Xaa1 is R5<br>Xaa3 is 6-F-Trp<br>Xaa5 is B5<br>Xaa7 is p-$CF_3$-Phe<br>Xaa12 is R8<br>Xaa1-Xaa12 all alpha-amino acids thereof have a D configuration<br>Xaa12 optionally has a C-terminal amide<br>Xaa1 optionally has a *N*-terminal acyl | XAXYXNXEKLLX |
| 9 | DPMI-δ(1-5, 9-12)<br>Xaa1 is R5<br>Xaa3 is 6-F-Trp<br>Xaa5 is R5<br>Xaa7 is p-$CF_3$-Phe<br>Xaa9 is S5<br>Xaa12 is R8<br>Xaa1-Xaa12 all alpha-amino acids thereof have a D configuration<br>Xaa12 optionally has a C-terminal amide<br>Xaa1 optionally has a N-terminal acyl | XAXYXNXEXLLX |
| 10 | N-terminal domain of MDM2<br>All-L amino acids | MCNTNMSVPTDGAVTTSQIPASEQETLVRPK PLLLKLLKSVGAQKDTYTMKEVLFYLGQYI MTKRLYDEKQQHIVYCSNDLLGDLFGVPSFS VKEHRKIYTMIYRNLVVVNQQESSDSGTSVS EN |
| 11 | N-terminal domain of MDM4 (MDMX)<br>All alpha-amino acids have an L configuration | MTSFSTSAQCSTSDSACRISPGQINQVRPKLP LLKILHAAGAQGEMFTVKEVMHYLGQYIMV KQLYDQQEQHMVYCGGDLLGELLGRQSFSV KDPSPLYDMLRKNLVTLATATTDAAQTLAL AQD |

(continued)

| Table of Sequences | | |
|---|---|---|
| SEQ ID NO: | Description | Sequence |
| 12 | Human MDM2 1-125 sequence<br>All alpha-amino acids have an L configuration | MSDKIIHSPILGYWKIKGLVQPTRLLLEYLEE<br>KYEEHLYERDEGDKWRNKKFELGLEFPNLP<br>YYIDGDVKLTQSMAIIRYIADKHNMLGGCPK<br>ERAEISMLEGAVLDIRYGVSRIAYSKDFETLK<br>VDFLSKLPEMLKMFEDRLCHKTYLNGDHVT<br>HPDFMLYDALDVVLYMDPMCLDAFPKLVC<br>FKKRIEAIPQIDKYLKSSKYIAWPLQGWQAT<br>FGGGDHPPKLEVLFQGHMHHHHHHSSGVDL<br>GTENLYFQGMCNTNMSVPTDGAVTTSQIPA<br>SEQETLVRPKPLLLKLLKSVGAQKDTYTMK<br>EVLFYLGQYIMTKRLYDEKQQHIVYCSNDLL<br>GDLFGVPSFSVKEHRKIYTMIYRNLVVVNQQ<br>ESSDSGTSVSEN |
| 13 | TV cleavage site<br>All alpha-amino acids have an L configuration | ENLYFQS |
| 14 | TV cleavage site with S7G substitution<br>All alpha-amino acids have an L configuration | ENLYFQG |
| 15 | Human p53 protein<br>All alpha-amino acids have an L configuration | MEEPQSDPSVEPPLSQETFSDLWKLLPENNV<br>LSPLPSQAMDDLMLSPDDIEQWFTEDPGPDE<br>APRMPEAAPRVAPAPAAPTPAAPAPAPSWPL<br>SSSVPSQKTYQGSYGFRLGFLHSGTAKSVTC<br>TYSPALNKMFCQLAKTCPVQLWVDSTPPPG<br>TRVRAMAIYKQSQHMTEVVRRCPHHERCSD<br>SDGLAPPQHLIRVEGNLRVEYLDDRNTFRHS<br>VVVPYEPPEVGSDCTTIHYNYMCNSSCMGG<br>MNRRPILTIITLEDSSGNLLGRNSFEVHVCAC<br>PGRDRRTEEENLRKKGEPHHELPPGSTKRAL<br>SNNTSSSPQPKKKPLDGEYFTLQIRGRERFEM |
| | | FRELNEALELKDAQAGKEPGGSRAHSSHLKS<br>KKGQSTSRHKKLMFKTEGPDSD |
| 16 | $^{D}$PMI-δ variant<br>Xaa1 is an alpha-amino or an alpha, alpha-disubstituted amino acid<br>Xaa3 is 6-F-Trp<br>Xaa5 is an alpha-amino or an alpha,alpha-disubstituted amino acid<br>Xaa7 is p-$CF_3$-Phe<br>Xaa9 is an alpha-amino or an alpha, alpha-disubstituted amino acid<br>Xaa12 is an alpha-amino or an alpha,alpha-disubstituted amino acid<br>Xaa1-Xaa12 all alpha-amino acids thereof have a D configuration<br>Xaa12 optionally has a C-terminal amide | XAXYXNXEXLLX |

(continued)

| Table of Sequences | | |
|---|---|---|
| SEQ ID NO: | Description | Sequence |
| | Xaa1 optionally has a N-terminal acyl | |
| 17 | DPMI-δ(1-5)<br>Xaa1 is R5<br>Xaa3 is 6-F-Trp<br>Xaa5 is R5<br>Xaa7 is p-$CF_3$-Phe<br>Xaa1-Xaa12 all alpha-amino acids thereof have a D configuration<br>Xaa12 has a C-terminal amide | XAXYXNXEKLLR |
| 18 | DPMI-δ(2-6)<br>Xaa2 is R5<br>Xaa3 is 6-F-Trp<br>Xaa6 is R5<br>Xaa7 is p-$CF_3$-Phe<br>Xaa1-Xaa12 all alpha-amino acids thereof have a D configuration<br>Xaa12 has a C-terminal amide | TXXYAXXEKLLR |
| 19 | DPMI-δ(2-9)<br>Xaa2 is S8<br>Xaa3 is 6-F-Trp<br>Xaa7 is p-$CF_3$-Phe<br>Xaa9 is R5<br>Xaa1-Xaa12 all alpha-amino acids thereof have a D configuration<br>Xaa12 has a C-terminal amide | TXXYANXEXLLR |
| 20 | DPMI-δ(5-9)<br>Xaa3 is 6-F-Trp<br>Xaa5 is R5<br>Xaa7 is p-$CF_3$-Phe<br>Xaa9 is R5<br>Xaa1-Xaa12 all alpha-amino acids thereof have a D configuration<br>Xaa12 has a C-terminal amide | TAXYXNXEXLLR |
| 21 | DPMI-δ(5-12)<br>Xaa3 is 6-F-Trp<br>Xaa5 is S8<br>Xaa7 is p-$CF_3$-Phe<br>Xaa12 is R5<br>Xaa1-Xaa12 all alpha-amino acids thereof have a D configuration<br>Xaa12 has a C-terminal amide | TAXYXNXEKLLX |
| 22 | DPMI-δ(6-10)<br>Xaa3 is 6-F-Trp<br>Xaa6 is R5<br>Xaa7 is p-$CF_3$-Phe<br>Xaa10 is R5 | TAXYAXXEKXLR |

(continued)

| Table of Sequences | | |
|---|---|---|
| SEQ ID NO: | Description | Sequence |
| | Xaa1-Xaa12 all alpha-amino acids thereof have a D configuration<br>Xaa12 has a C-terminal amide | |
| 23 | DPMI-δ(1,5,12)<br>Xaa1 is R5<br>Xaa3 is 6-F-Trp<br>Xaa5 is B5<br>Xaa7 is p-$CF_3$-Phe<br>Xaa12 is R8<br>Xaa1-Xaa12 all alpha-amino acids thereof have a D configuration<br>Xaa12 has a C-terminal amide | XAXYXNXEKLLX |
| 24 | DPMI-δ(1-5, 9-12)<br>Xaa1 is R5<br>Xaa3 is 6-F-Trp<br>Xaa5 is R5<br>Xaa7 is p-$CF_3$-Phe<br>Xaa9 is S5<br>Xaa12 is R8<br>Xaa1-Xaa12 all alpha-amino acids thereof have a D configuration<br>Xaa12 has a C-terminal amide | XAXYXNXEXLLX |
| | | |
| | | |
| R5 is (R)-2-(4'-pentenyl)-alanine<br>R8 is (R)-2-(7'-octenyl)-alanine<br>S5 is (S)-2-(4'-pentenyl)-alanine<br>S8 is (S)-2-(7'-octenyl)-alanine<br>B5 is 2,2-(4'-penenyl)-glycine | | |

## Claims

**1.** A peptidomimetic macrocycle comprising:
a peptide of D configuration α-amino acids having the amino acid sequence set forth in SEQ ID NO:16 and two staples or one stitch, wherein each staple comprises a hydrocarbon crosslinker linking the α-carbons of two α,α-disubstituted amino acids separated by at least two α-amino acids and each stitch comprises two hydrocarbon crosslinkers linking the α-carbons of two α,α-disubstituted amino acids to the α-carbon of a common α,α-disubstituted amino acid situated between the two α,α-disubstituted amino acids.

**2.** The peptidomimetic macrocycle of claim 1, wherein each α,α-disubstituted amino acid comprises one or two α-carbon-linked reactive groups wherein the reactive group of a first α,α-disubstituted amino acid is capable of reacting with the reactive group of a second α,α-disubstituted amino acid to form a crosslinker.

**3.** The peptidomimetic macrocycle of claim 2, wherein the reactive groups each comprises a terminal olefin group.

**4.** The peptidomimetic macrocycle of claim 1, wherein the peptide comprises a stitch in which a first crosslinker links the α-carbon of an α,α-disubstituted amino acid at position 1 to the α-position of a common α,α-disubstituted amino acid

at position 5 and a second crosslinker links the α-position of an α,α-disubstituted amino acid at position 12 to the α-position of the common α,α-disubstituted amino acid at position 5.

**5.** The peptidomimetic macrocycle of claim 4, wherein the α,α-disubstituted amino acid at position 1 is (R)-2-(4'-pentenyl)alanine, at position 12 is (R)-2-(7'-octenyl)alanine, and at position 5 is 2,2-(4'-pentenyl)glycine.

**6.** The peptidomimetic macrocycle of claim 1, wherein the peptidomimetic macrocycle comprises the amino acid sequence set forth in SEQ ID NO: 8.

**7.** The peptidomimetic macrocycle of claim 1, wherein the peptidomimetic macrocycle comprises the formula

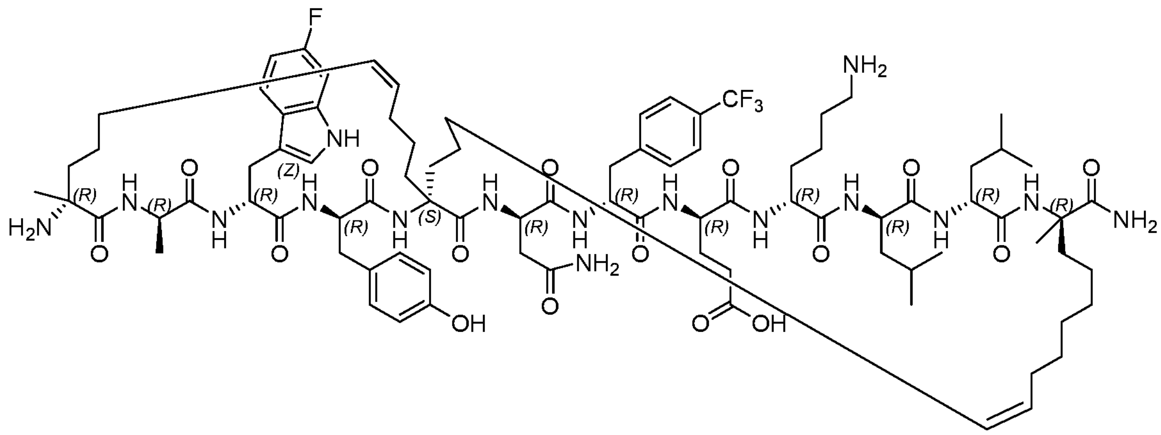

(SEQ ID NO:23).

**8.** The peptidomimetic macrocycle of claim 1, wherein the peptide comprises two staples wherein the first staple comprises a crosslinker that links the α-position of an α,α-disubstituted amino acid at position 1 to the α-position of an α,α-disubstituted amino acid at position 5 and the second staple comprises a crosslinker that links the α-position of an α,α-disubstituted amino acid at position 9 to the α-position of an α,α-disubstituted amino acid at position 12.

**9.** The peptidomimetic macrocycle of claim 8, wherein the α,α-disubstituted amino acids at positions 1 and 5 are each (R)-2-(4'-pentenyl)alanine and the amino acids at positions 9 and 12 are (S)-2-(4'-pentenyl)alanine and (R)-2-(7'-octenyl)alanine, respectively.

**10.** The peptidomimetic macrocycle of claim 1, wherein the peptidomimetic macrocycle comprises the amino acid sequence set forth in SEQ ID NO: 9.

**11.** The peptidomimetic macrocycle of claim 1, wherein the peptidomimetic macrocycle comprises the formula

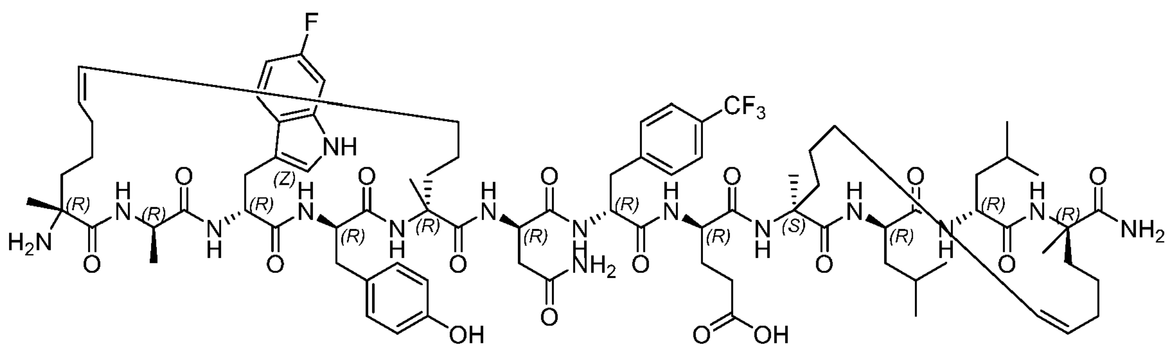

(SEQ ID NO: 24).

**12.** The peptidomimetic macrocycle of claim 1, wherein at least one α,α-disubstituted amino acid of the peptidomimetic macrocycle has a D configuration.

**13.** The peptidomimetic macrocycle of claim 1, wherein the peptidomimetic macrocycle binds both mouse double minute 2 (MDM2) and mouse double minute X (MDMX), is protease resistant and cell permeable with no detectable disruption of the cell membrane as determined by a lactate dehydrogenase (LDH) release assay, and activates p53 intracellularly.

**14.** A composition comprising the peptidomimetic macrocycle of any one of claims 1-13 and a pharmaceutically acceptable carrier or excipient.

**15.** A peptidomimetic macrocycle of any one of claims 1-13 for use in a method for treating cancer.

**16.** The peptidomimetic macrocycle for use of claim 15, wherein the cancer is selected from the group consisting of melanoma, non-small cell lung cancer, head and neck cancer, urothelial cancer, breast cancer, gastrointestinal cancer, multiple myeloma, hepatocellular cancer, non-Hodgkin lymphoma, renal cancer, Hodgkin lymphoma, mesothelioma, ovarian cancer, small cell lung cancer, esophageal cancer, anal cancer, biliary tract cancer, colorectal cancer, cervical cancer, thyroid cancer, salivary cancer, pancreatic cancer, bronchus cancer, prostate cancer, pancreatic cancer, stomach cancer, ovarian cancer, urinary bladder cancer, brain or central nervous system cancer, peripheral nervous system cancer, uterine or endometrial cancer, cancer of the oral cavity or pharynx, liver cancer, kidney cancer, testicular cancer, biliary tract cancer, small bowel or appendix cancer, adrenal gland cancer, osteosarcoma, chondrosarcoma, and cancer of hematological tissues.

**17.** A peptidomimetic macrocycle of any one of claims 1-13 and a therapeutically effective dose of a chemotherapy agent or radiation for use in a combination therapy for treating cancer.

**18.** The combination therapy for use of claim 17, wherein the chemotherapy agent is selected from the group consisting of actinomycin, all-trans retinoic acid, alitretinoin, azacitidine, azathioprine, bexarotene, bleomycin, bortezomib, carmofur, carboplatin, capecitabine, cisplatin, chlorambucil, cyclophosphamide, cytarabine, dacarbazine, daunorubicin, docetaxel, doxifluridine, doxorubicin, epirubicin, epothilone, etoposide, fluorouracil, gemcitabin, hydroxyurea, idarubicin, imatinib, ixabepilone, irinotecan, mechlorethamine, melphalan, mercaptopurine, methotrexate, mitoxantrone, nitrosoureas, oxaliplatin, paclitaxel, pemetrexed, romidepsin, tegafur, temozolomide(oral dacarbazine), teniposide, tioguanine, topotecan, utidelone, valrubicin, vemurafenib, vinblastine, vincristine, vindesine, vinorelbine, and vorinostat.

**19.** A peptidomimetic macrocycle of any one of claims 1-13 and a therapeutically effective amount of a checkpoint inhibitor for use in a combination therapy for treating cancer.

**20.** The combination therapy for use of claim 19, wherein the checkpoint inhibitor is an anti-PD1 antibody or an anti-PD-L1 antibody.

**21.** The combination therapy for use of claim 19, wherein the therapy further includes administering to the subject a therapeutically effective dose of a chemotherapy agent or radiation.

## Patentansprüche

**1.** Ein peptidomimetischer Makrocyclus, umfassend:
ein Peptid aus α-Aminosäuren in der D-Konfiguration mit der in SEQ ID NO: 16 angegebenen Aminosäuresequenz und zwei Klammern oder einer Stitch-Verbrückung, wobei jede Klammer einen Kohlenwasserstoff-Crosslinker umfasst, der die α-Kohlenstoffe von zwei α,α-disubstituierten Aminosäuren verknüpft, die durch wenigstens zwei α-Aminosäuren getrennt sind, und jede Stitch-Verbrückung zwei Kohlenwasserstoff-Crosslinker umfasst, die die α-Kohlenstoffe von zwei α,α-disubstituierten Aminosäuren mit dem α-Kohlenstoff einer gemeinsamen α,α-disubstituierten Aminosäure verknüpfen, die sich zwischen den zwei α,α-disubstituierten Aminosäuren befindet.

**2.** Der peptidomimetische Makrocyclus nach Anspruch 1, wobei jede α,α-disubstituierte Aminosäure ein oder zwei α-Kohlenstoff-verbrückte reaktive Gruppen umfasst, wobei die reaktive Gruppe einer ersten α,α-disubstituierten Aminosäure in der Lage ist, mit der reaktiven Gruppe einer zweiten α,α-disubstituierten Aminosäure zu reagieren, um einen Crosslinker zu bilden.

**3.** Der peptidomimetische Makrocyclus nach Anspruch 2, wobei die reaktiven Gruppen jeweils eine terminale Olefingruppe umfassen.

4. Der peptidomimetische Makrocyclus nach Anspruch 1, wobei das Peptid eine Stitch-Verbrückung umfasst, bei der ein erster Crosslinker das α-Kohlenstoff einer α,α-disubstituierten Aminosäure an Position 1 mit der α-Position einer gemeinsamen α,α-disubstituierten Aminosäure an Position 5 verknüpft und ein zweiter Crosslinker die α-Position einer α,α-disubstituierten Aminosäure an Position 12 mit der α-Position der gemeinsamen α,α-disubstituierten Aminosäure an Position 5 verknüpft.

5. Der peptidomimetische Makrocyclus nach Anspruch 4, wobei die α,α-disubstituierte Aminosäure an Position 1 (R)-2-(4'-Pentenyl)alanin ist, an Position 12 (R)-2-(7'-Octenyl)alanin ist, und an Position 5 2,2-(4'-Pentenyl)glycin ist.

6. Der peptidomimetische Makrocyclus nach Anspruch 1, wobei der peptidomimetische Makrocyclus die in SEQ ID NO: 8 angegebene Aminosäuresequenz umfasst.

7. Der peptidomimetische Makrocyclus nach Anspruch 1, wobei der peptidomimetische Makrocyclus folgende Formel umfasst:

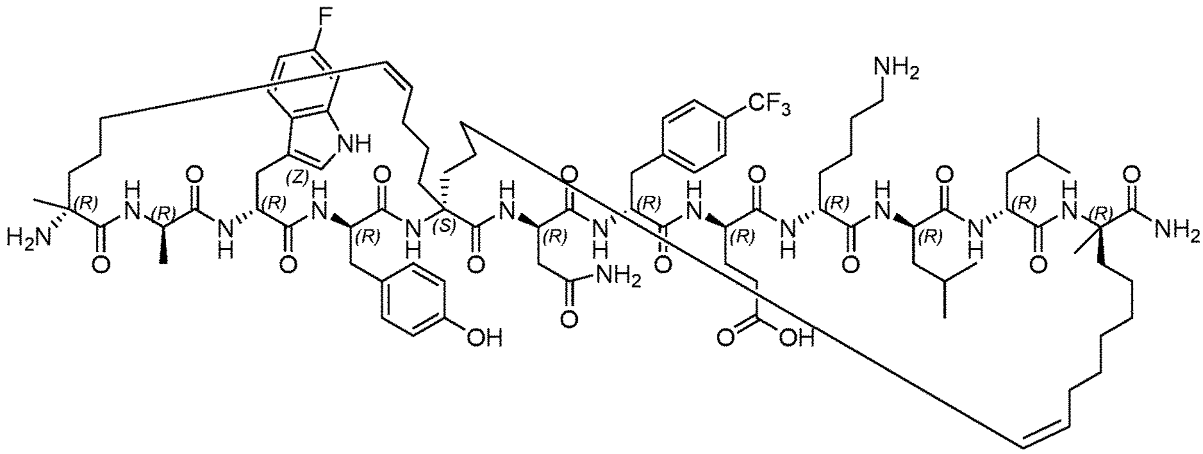

(SEQ ID NO:23).

8. Der peptidomimetische Makrocyclus nach Anspruch 1, wobei das Peptid zwei Klammern umfasst, wobei die erste Klammer einen Crosslinker umfasst, der die α-Position einer α,α-disubstituierten Aminosäure an Position 1 mit der α-Position einer α,α-disubstituierten Aminosäure an Position 5 verknüpft, und die zweite Klammer einen Crosslinker umfasst, der die α-Position einer α,α-disubstituierten Aminosäure an Position 9 mit der α-Position einer α,α-disubstituierten Aminosäure an Position 12 verknüpft.

9. Der peptidomimetische Makrocyclus nach Anspruch 8, wobei die α,α-disubstituierten Aminosäuren an den Positionen 1 und 5 jeweils (R)-2-(4'-Pentenyl)alanin sind und die Aminosäuren an den Positionen 9 und 12 (S)-2-(4'-Pentenyl)alanin bzw. (R)-2-(7'-Octenyl)alanin sind.

10. Der peptidomimetische Makrocyclus nach Anspruch 1, wobei der peptidomimetische Makrocyclus die in SEQ ID NO: 9 angegebene Aminosäuresequenz umfasst.

11. Der peptidomimetische Makrocyclus nach Anspruch 1, wobei der peptidomimetische Makrocyclus folgende Formel umfasst:

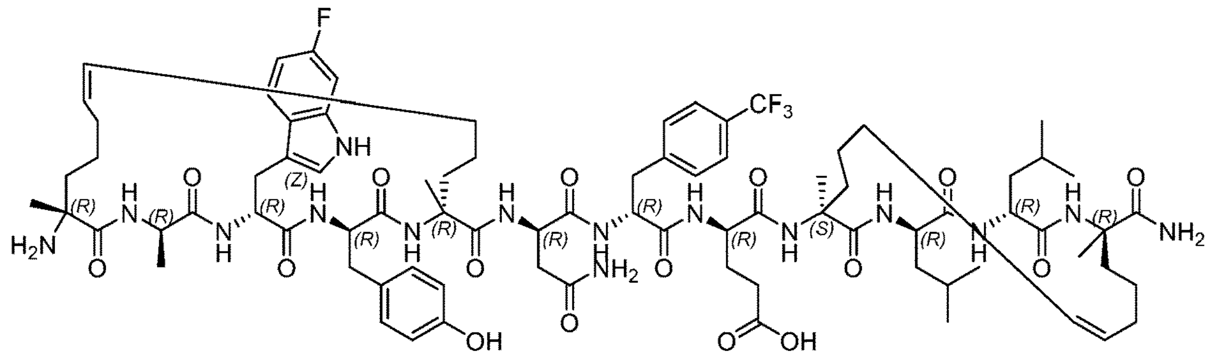

(SEQ ID NO: 24).

**12.** Der peptidomimetische Makrocyclus nach Anspruch 1, wobei wenigstens eine α,α-disubstituierte Aminosäure des peptidomimetischen Makrocyclus eine D-Konfiguration besitzt.

**13.** Der peptidomimetische Makrocyclus nach Anspruch 1, wobei der peptidomimetische Makrocyclus sowohl Maus-Double-Minute 2 (MDM2) als auch Maus-Double-Minute X (MDMX) bindet, proteaseresistent und zellpermeabel ohne nachweisbare Disruption der Zellmembran, ermittelt durch ein Laktatdehydrogenase (LDH)-Release-Assay, ist, und p53 intrazellulär aktiviert.

**14.** Eine Zusammensetzung, die den peptidomimetischen Makrocyclus nach einem der Ansprüche 1 - 13 und einen pharmazeutisch annehmbaren Träger oder Hilfsstoff umfasst.

**15.** Ein peptidomimetischer Makrocyclus nach einem der Ansprüche 1 - 13 zur Verwendung bei einem Verfahren zur Behandlung von Krebs.

**16.** Der peptidomimetische Makrocyclus zur Verwendung nach Anspruch 15, wobei der Krebs ausgewählt ist aus der Gruppe bestehend aus Melanom, nicht-kleinzelligem Lungenkarzinom, Kopf-Hals-Tumoren, Urothelkarzinom, Brustkrebs, gastrointestinalen Tumoren, multiplem Myelom, hepatozellulärem Karzinom, Nicht-Hodgkin-Lymphom, Nierenkarzinom, Hodgkin-Lymphom, Mesotheliom, Ovarialkarzinom, kleinzelligem Lungenkarzinom, Ösophaguskarzinom, Analkarzinom, Gallengangskarzinom, kolorektalem Karzinom, Gebärmutterhalskrebs, Schilddrüsenkarzinom, Speicheldrüsenkarzinom, Bauchspeicheldrüsenkarzinom, Bronchialkarzinom, Prostatakarzinom, Bauchspeicheldrüsenkarzinom, Magenkrebs, Ovarialkarzinom, Harnblasenkarzinom, Gehirntumor oder Tumoren des zentralen Nervensystems, Tumoren des peripheren Nervensystems, Uterus- oder Endometriumkarzinom, Tumoren der Mundhöhle oder des Rachens, Leberkrebs, Nierenkrebs, Hodenkrebs, Gallengangskarzinom, Dünndarm- oder Appendixkarzinom, Nebennierenkarzinom, Osteosarkom, Chondrosarkom und Karzinomen von hämatologischen Geweben.

**17.** Ein peptidomimetischer Makrocyclus nach einem der Ansprüche 1 - 13 und eine therapeutisch wirksame Dosis eines chemotherapeutischen Mittels oder Strahlung zur Verwendung bei einer Kombinationstherapie zur Behandlung von Krebs.

**18.** Die Kombinationstherapie zur Verwendung nach Anspruch 17, wobei das chemotherapeutische Mittel ausgewählt ist aus der Gruppe bestehend aus Actinomycin, all-trans-Retinsäure, Alitretinoin, Azacitidin, Azathioprin, Bexaroten, Bleomycin, Bortezomib, Carmofur, Carboplatin, Capecitabin, Cisplatin, Chlorambucil, Cyclophosphamid, Cytarabin, Dacarbazin, Daunorubicin, Docetaxel, Doxifluridin, Doxorubicin, Epirubicin, Epothilon, Etoposid, Fluorouracil, Gemcitabin, Hydroxyharnstoff, Idarubicin, Imatinib, Ixabepilon, Irinotecan, Mechlorethamin, Melphalan, Mercaptopurin, Methotrexat, Mitoxantron, Nitrosoharnstoffen, Oxaliplatin, Paclitaxel, Pemetrexed, Romidepsin, Tegafur, Temozolomid (orales Dacarbazin), Teniposid, Tioguanin, Topotecan, Utidelon, Valrubicin, Vemurafenib, Vinblastin, Vincristin, Vindesin, Vinorelbin und Vorinostat.

**19.** Ein peptidomimetischer Makrocyclus nach einem der Ansprüche 1 - 13 und eine therapeutisch wirksame Menge eines Checkpoint-Inhibitors zur Verwendung bei einer Kombinationstherapie zur Behandlung von Krebs.

**20.** Die Kombinationstherapie zur Verwendung nach Anspruch 19, wobei der Checkpoint-Inhibitor ein Anti-PD1-Antikörper oder ein Anti-PD-L1-Antikörper ist.

21. Die Kombinationstherapie zur Verwendung nach Anspruch 19, wobei die Therapie ferner das Verabreichen einer therapeutisch wirksamen Dosis eines chemotherapeutischen Mittels oder Strahlung an das Subjekt umfasst.

## Revendications

1. Macrocycle peptidomimétique comprenant :
un peptide d'acides aminés α de configuration D possédant la séquence d'acides aminés présentée dans la SEQ ID NO :16 et deux attaches ou une couture, dans lequel chaque attache comprend un agent de réticulation hydrocarbure reliant les carbones α de deux acides aminés α,α-disubstitués séparés par au moins deux acides aminés α et chaque couture comprend deux agents de réticulation hydrocarbures reliant les carbones α de deux acides aminés α,α-disubstitués au carbone α d'un acide aminé α,α-disubstitué commun situé entre les deux acides aminés α,α-disubstitués.

2. Macrocycle peptidomimétique selon la revendication 1, dans lequel chaque acide aminé α,α-disubstitué comprend un ou deux groupes réactifs reliés par un carbone α, dans lequel le groupe réactif d'un premier acide aminé α,α-disubstitué est capable de réagir avec le groupe réactif d'un deuxième acide aminé α,α-disubstitué pour former un agent de réticulation.

3. Macrocycle peptidomimétique selon la revendication 2, dans lequel les groupes réactifs comprennent chacun un groupe oléfinique terminal.

4. Macrocycle peptidomimétique selon la revendication 1, dans lequel le peptide comprend une couture dans lequel un premier agent de réticulation relie le carbone α d'un acide aminé α,α-disubstitué en position 1 à la position α d'un acide aminé α,α-disubstitué commun en position 5 et un deuxième agent de réticulation relie la position α d'un acide aminé α,α-disubstitué en position 12 à la position α d'un acide aminé α,α-disubstitué commun en position 5.

5. Macrocycle peptidomimétique selon la revendication 4, dans lequel l'acide aminé α,α-disubstitué en position 1 est une (R)-2-(4'-penténylalanine, en position 12 est une (R)-2-(7'-octényl)alanine et en position 5 est une 2,2-(4'-pentényl)glycine.

6. Macrocycle peptidomimétique selon la revendication 1, où le macrocycle peptidomimétique comprend la séquence d'acides aminés présentée dans la SEQ ID NO :8.

7. Macrocycle peptidomimétique selon la revendication 1, où le macrocycle peptidomimétique comprend la formule :

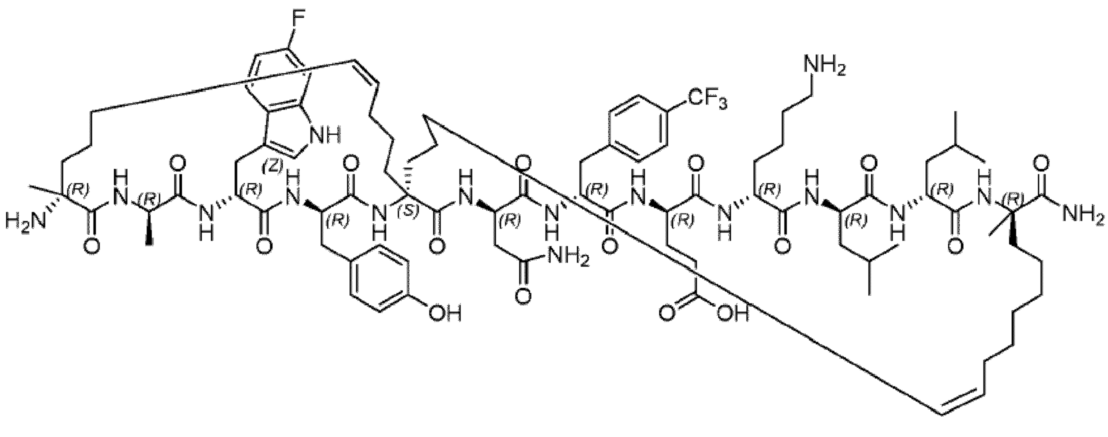

(SEQ ID NO :23).

8. Macrocycle peptidomimétique selon la revendication 1, dans lequel le peptide comprend deux attaches, dans lequel la première attache comprend un agent de réticulation qui relie la position α d'un acide aminé α,α-disubstitué en position 1 à la position α d'un acide aminé α,α-disubstitué en position 5 et la deuxième attache comprend un agent de réticulation qui relie la position α d'un acide aminé α,α-disubstitué en position 9 à la position α d'un acide aminé α,α-disubstitué en position 12.

9. Macrocycle peptidomimétique selon la revendication 8, dans lequel les acides aminés α,α-disubstitués en positions 1 et 5 sont chacun une (R)-2-(4'-penténynl)alanine et les acides aminés en positions 9 et 12 sont une (S)-2-(4'-penténynl)alanine et une (R)-2-(7'-octényl)alanine, respectivement.

10. Macrocycle peptidomimétique selon la revendication 1, où le macrocycle peptidomimétique comprend la séquence d'acides aminés présentée dans la SEQ ID NO :9.

11. Macrocycle peptidomimétique selon la revendication 1, où le macrocycle peptidomimétique comprend la formule :

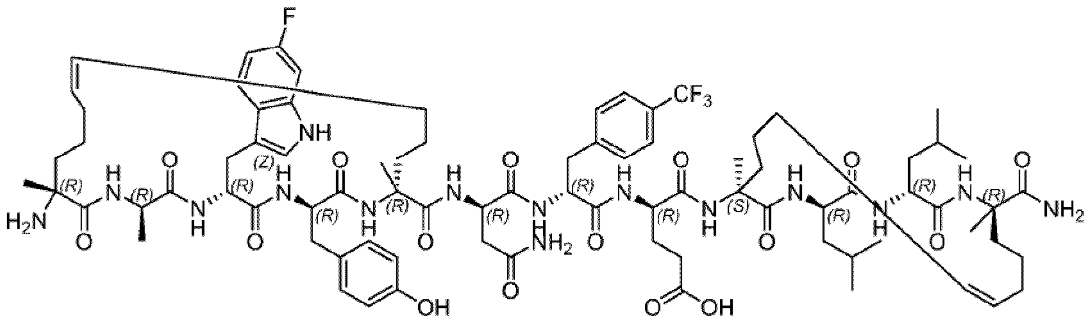

(SEQ ID NO :24).

12. Macrocycle peptidomimétique selon la revendication 1, dans lequel au moins un acide aminé α,α-disubstitué du macrocycle peptidomimétique a une configuration D.

13. Macrocycle peptidomimétique selon la revendication 1, où le macrocycle peptidomimétique relie à la fois un minuscule double de souris 2 (MDM2) et un minuscule double de souris X (MDMX), est résistant à une protéase et perméable à une membrane cellulaire sans rupture détectable de la membrane cellulaire tel que déterminé par un essai de libération de lactate déshydrogénase (LDH) et active p53 de façon intracellulaire.

14. Composition comprenant le macrocycle peptidomimétique selon l'une quelconque des revendications 1-13 et un véhicule ou excipient pharmaceutiquement acceptables.

15. Macrocycle peptidomimétique selon l'une quelconque des revendications 1 à 13 pour utilisation dans une méthode de traitement d'un cancer.

16. Macrocycle peptidomimétique pour utilisation selon la revendication 15, où le cancer est choisi dans le groupe constitué de : mélanome, cancer pulmonaire non à petites cellules, cancer de la tête et du cou, cancer urothélial, cancer du sein, cancer gastro-intestinal, myélome multiple, cancer hépatocellulaire, lymphome non hodgkinien, cancer rénal, lymphome hodgkinien, mésothéliome, cancer ovarien, cancer pulmonaire à petites cellules, cancer œsophagien, cancer anal, cancer du tractus biliaire, cancer colorectal, cancer du col de l'utérus, cancer de la thyroïde, cancer salivaire, cancer pancréatique, cancer des bronches, cancer de la prostate, cancer pancréatique, cancer de l'estomac, cancer ovarien, cancer de la vessie, cancer du cerveau ou du système nerveux central, cancer du système nerveux périphérique, cancer de l'utérus ou de l'endomètre, cancer de la cavité buccale ou du pharynx, cancer du foie, cancer du rein, cancer testiculaire, cancer du tractus biliaire, cancer de l'intestin grêle ou de l'appendice, cancer des glandes surrénales, ostéosarcome, chondrosarcome et cancer de tissus hématologiques.

17. Macrocycle peptidomimétique selon l'une quelconque des revendications 1 à 13 et une dose thérapeutiquement efficace d'un agent de chimiothérapie ou de rayons pour utilisation dans une thérapie de combinaison pour le traitement d'un cancer.

18. Thérapie de combinaison pour utilisation selon la revendication 17, où l'agent de chimiothérapie est choisi dans le groupe constitué de : actinomycine, acide rétinoïque totalement trans, alitrétinoïne, azacitidine, azathioprine, bexarotène, bléomycine, bortézomib, carmofur, carboplatine, capécitabine, cisplatine, chlorambucil, cyclophosphamide, cytarabine, dacarbazine, daunorubicine, docétaxel, doxifluridine, doxorubicine, épirubicine, épothilone, étoposide, fluorouracile, gemcitabine, hydroxyurée, idarubicine, imatinib, ixabépilone, irinotécan, méchloréthamine, melphalan, mercaptopurine, méthotrexate, mitoxantrone, nitrosourées, oxaliplatine, paclitaxel, pémétrexed, romidepsine, tégafur, témozolomide (dacarbazine orale), téniposide, tioguanine, topotécan, utidélone, valrubicine,

vémurafénib, vinblastine, vincristine, vindésine, vinorelbine et vorinostat.

**19.** Macrocycle peptidomimétique selon l'une quelconque des revendications 1 à 13 et une quantité thérapeutiquement efficace d'un inhibiteur de point de contrôle pour utilisation dans une thérapie de combinaison pour le traitement d'un cancer.

**20.** Thérapie de combinaison pour utilisation selon la revendication 19, où l'inhibiteur de point de contrôle est un anticorps anti-PD1 ou un anticorps anti-PD-L1.

**21.** Thérapie de combinaison pour utilisation selon la revendication 19, où la thérapie inclut en outre l'administration au sujet d'une dose thérapeutiquement efficace d'un agent de chimiothérapie ou de rayons.

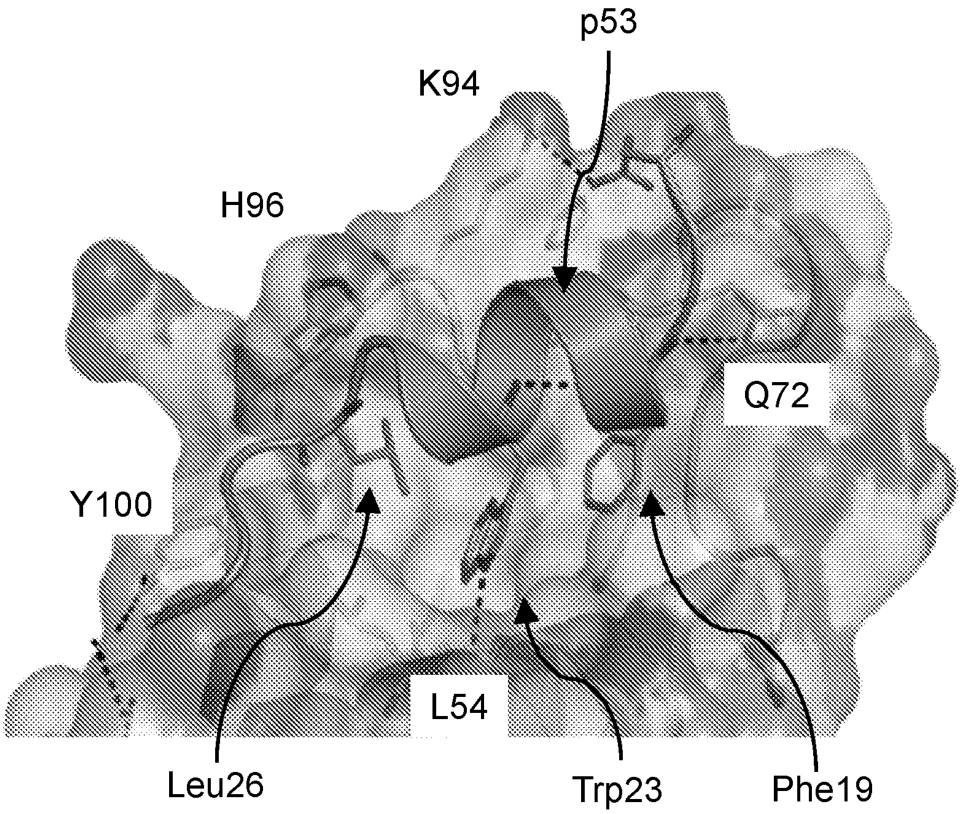
p53
K94
H96
Q72
Y100
L54
Leu26
Trp23
Phe19

FIG.1A

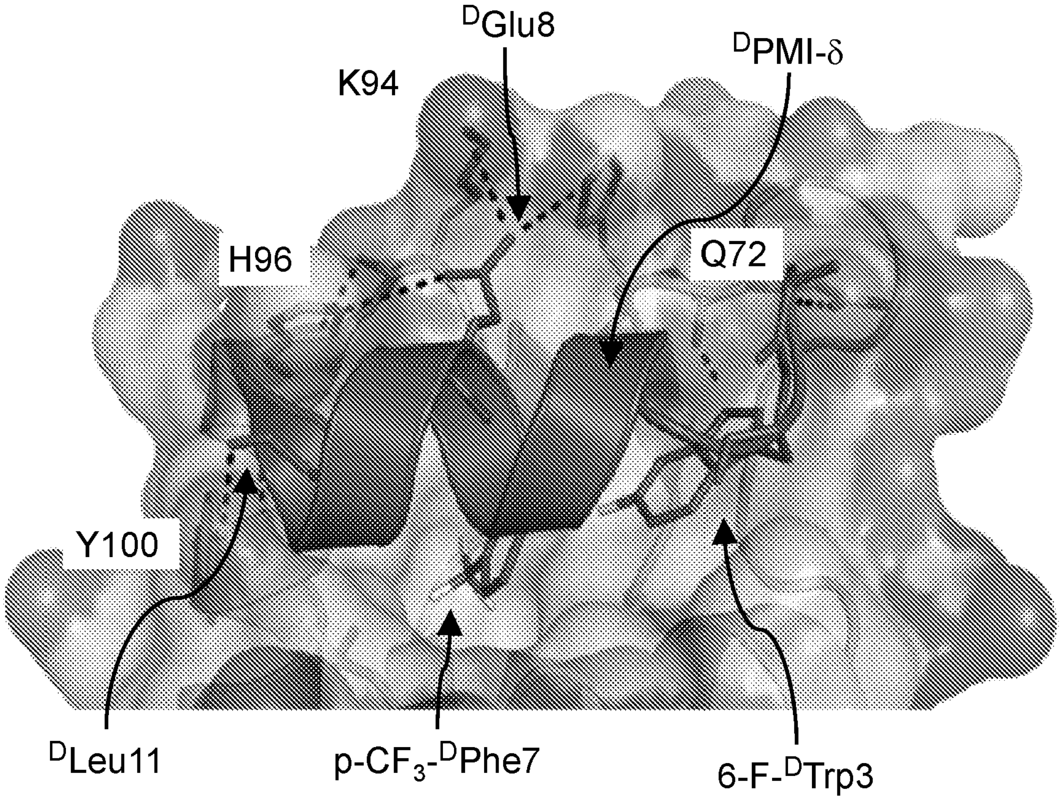
DGlu8
DPMI-δ
K94
H96
Q72
Y100
DLeu11
p-CF3-DPhe7
6-F-DTrp3

FIG.1B

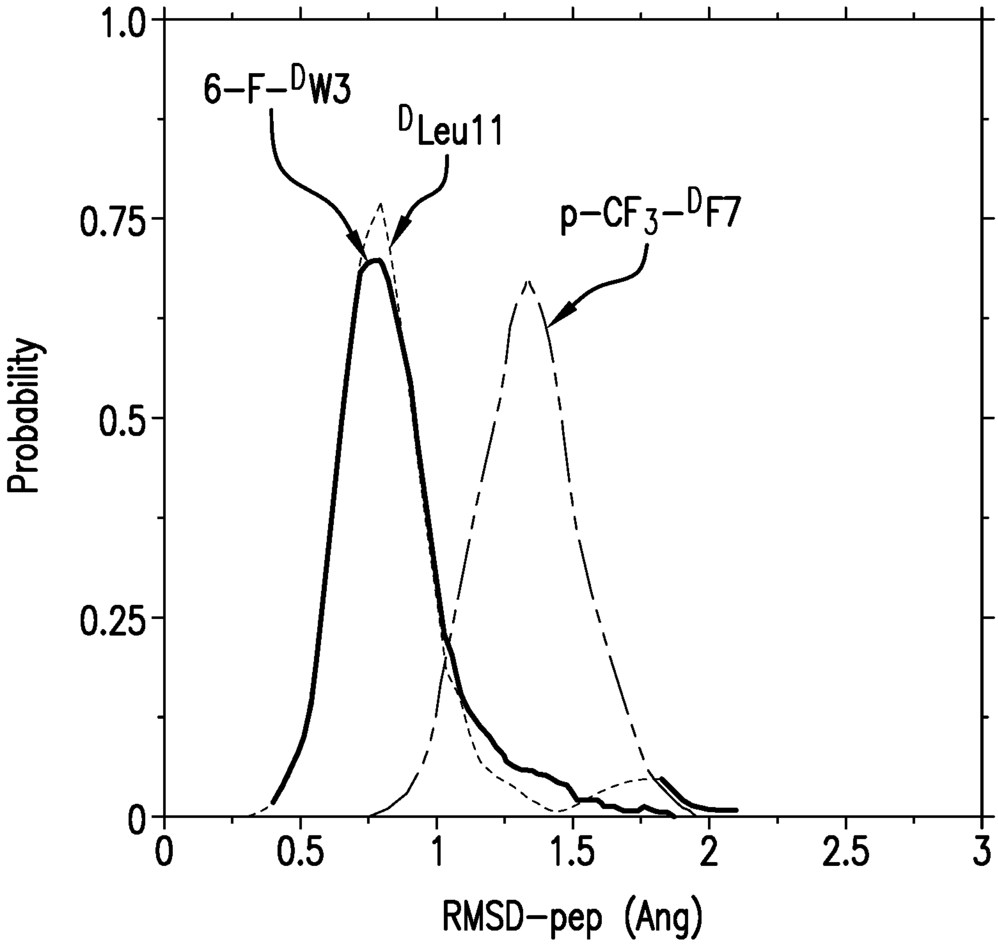
1.0
0.75
0.5
0.25
0
Probability
6-F-DW3
DLeu11
p-CF3-DF7
0
0.5
1
1.5
2
2.5
3
RMSD-pep (Ang)

FIG.2A

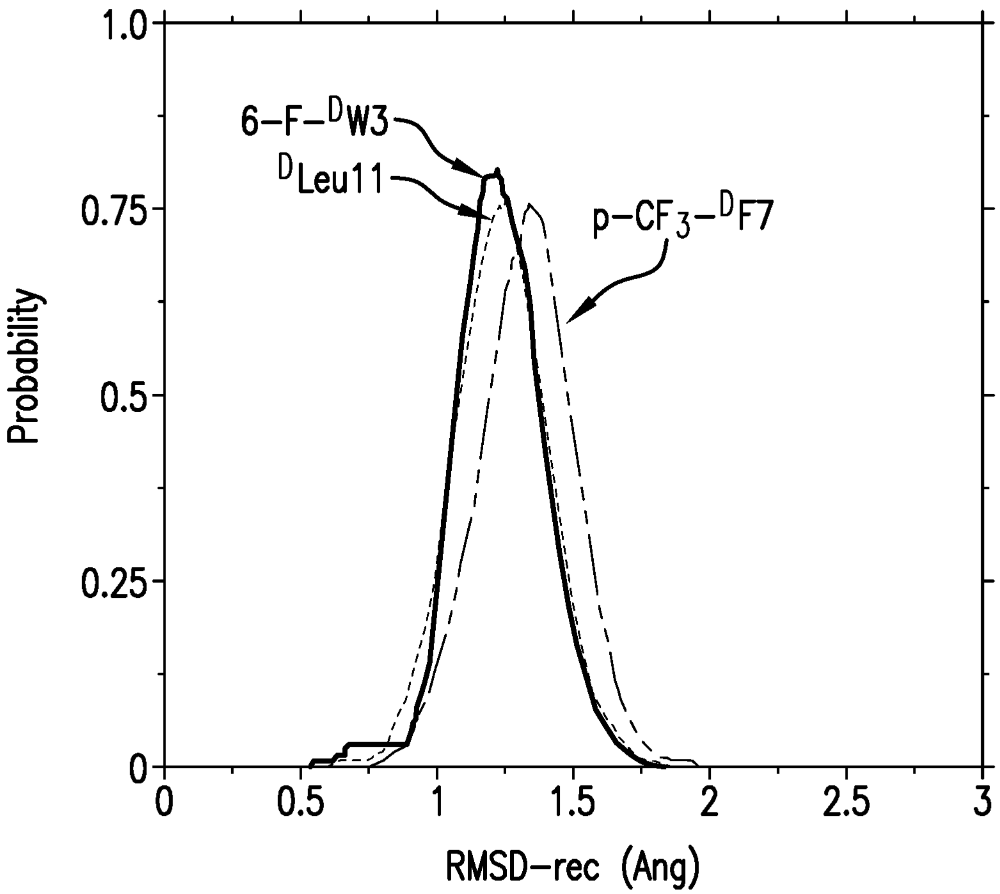
1.0
0.75
0.5
0.25
0
Probability
6-F-DW3
DLeu11
p-CF3-DF7
0
0.5
1
1.5
2
2.5
3
RMSD-rec (Ang)

FIG.2B

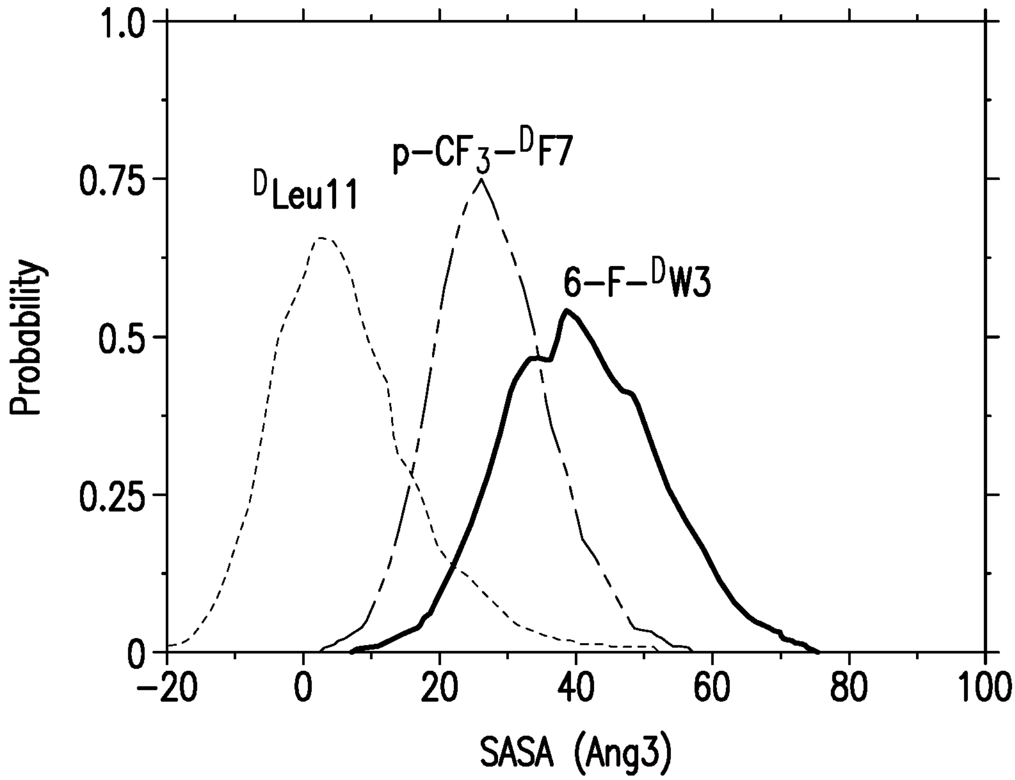
1.0
0.75
0.5
0.25
0
Probability
−20
0
20
40
60
80
100
SASA (Ang3)
DLeu11
p−CF3−DF7
6−F−DW3

FIG.2C

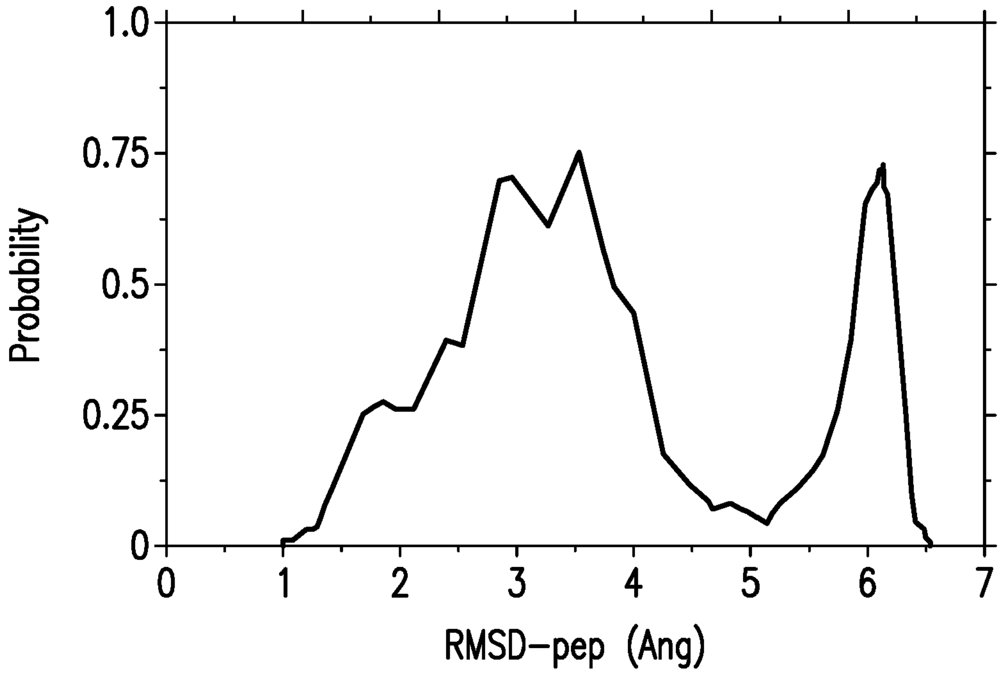


FIG.2D

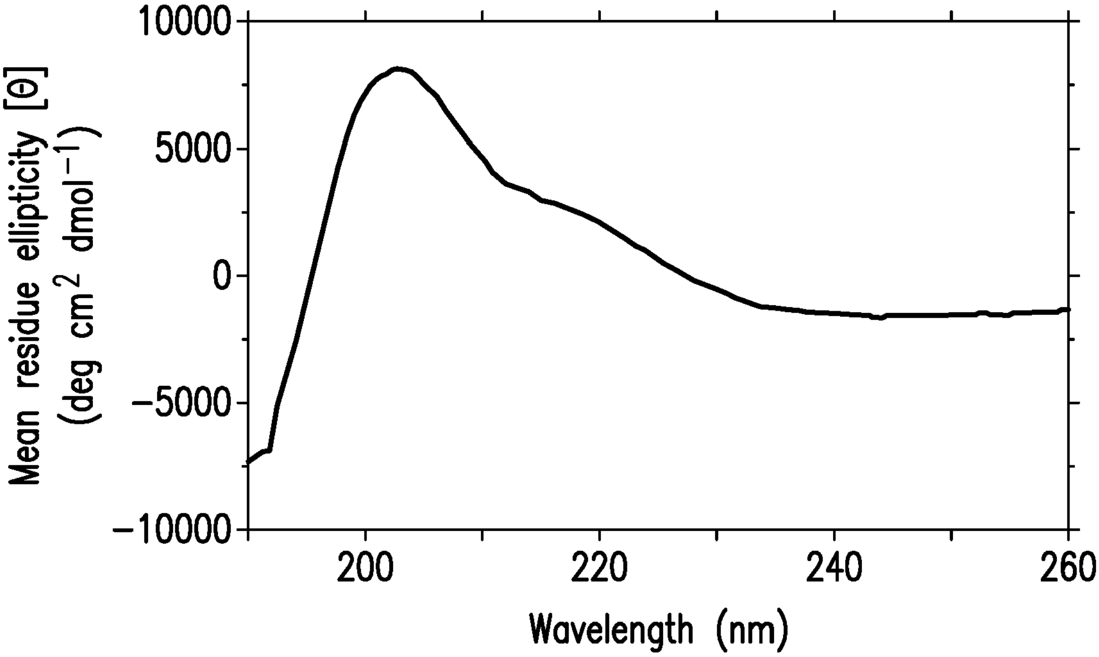
10000
5000
0
−5000
−10000
200
220
240
260
Wavelength (nm)
Mean residue ellipticity [θ]
(deg cm$^2$ dmol$^{-1}$)

FIG.2E

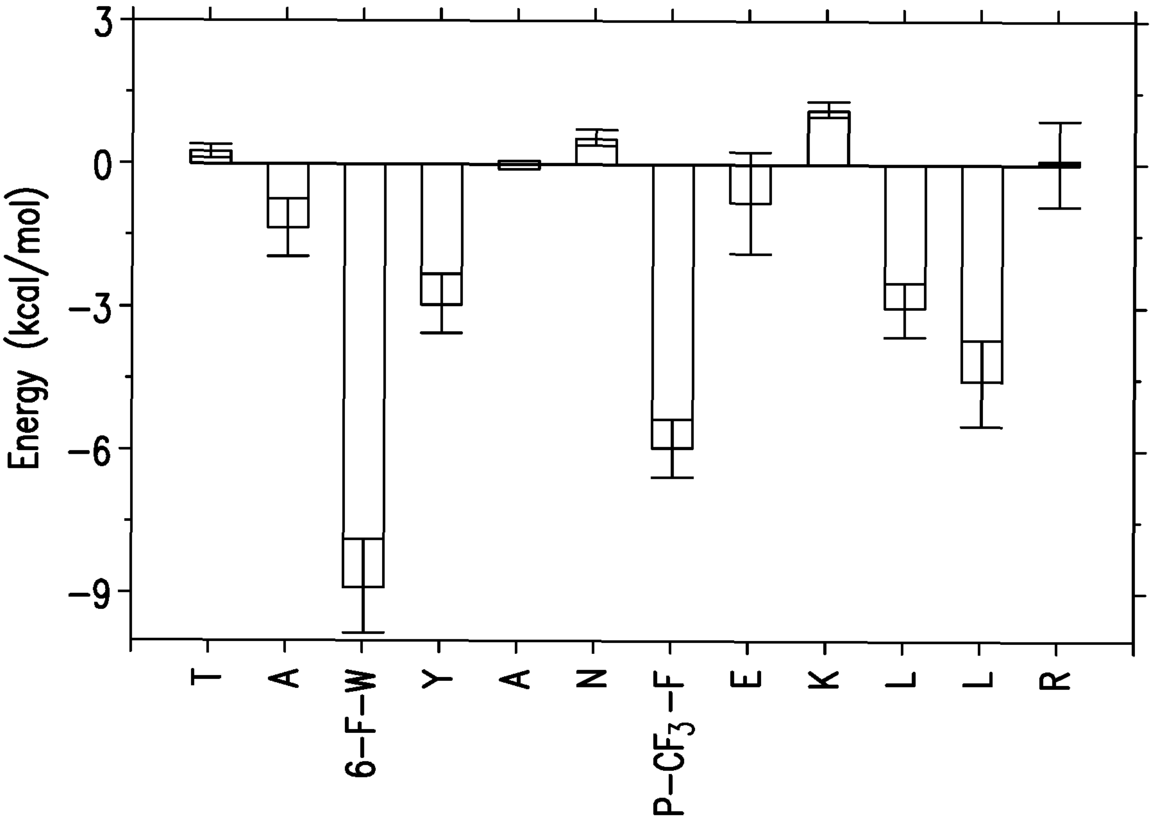


FIG.3A

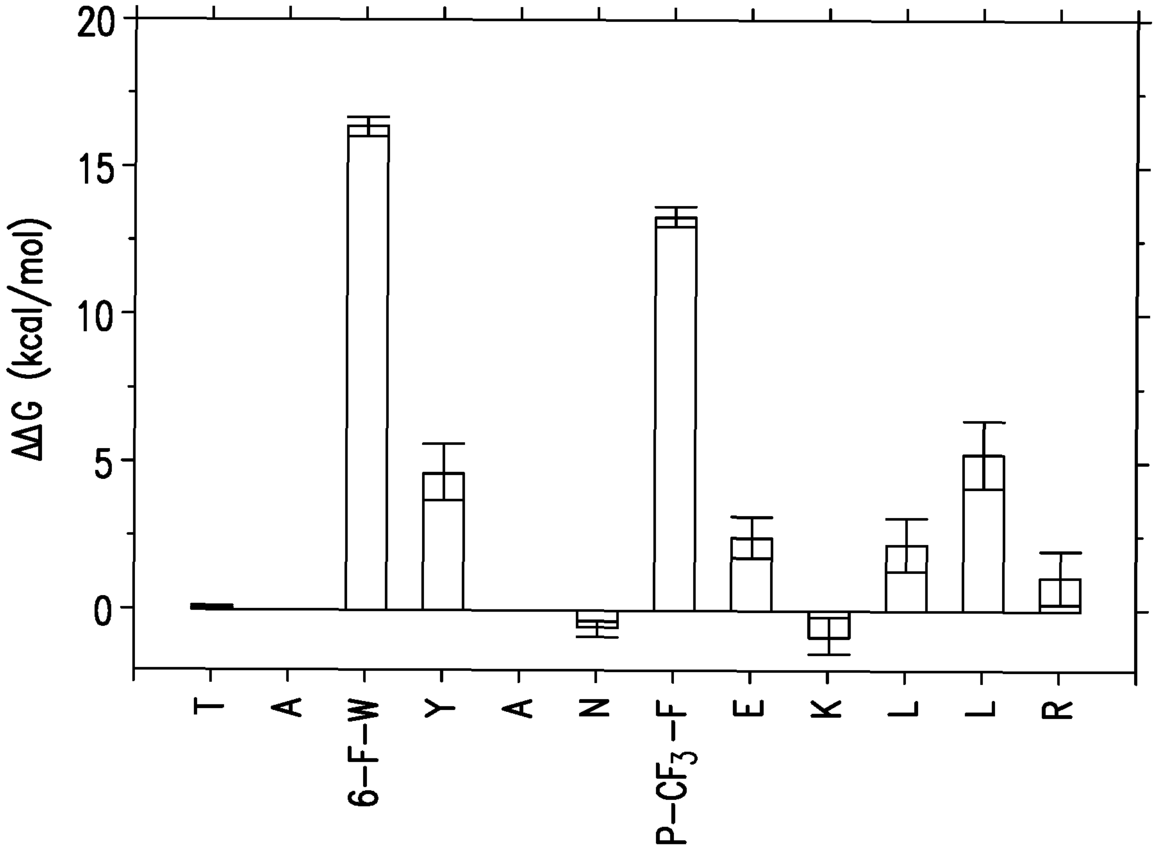


FIG.3B

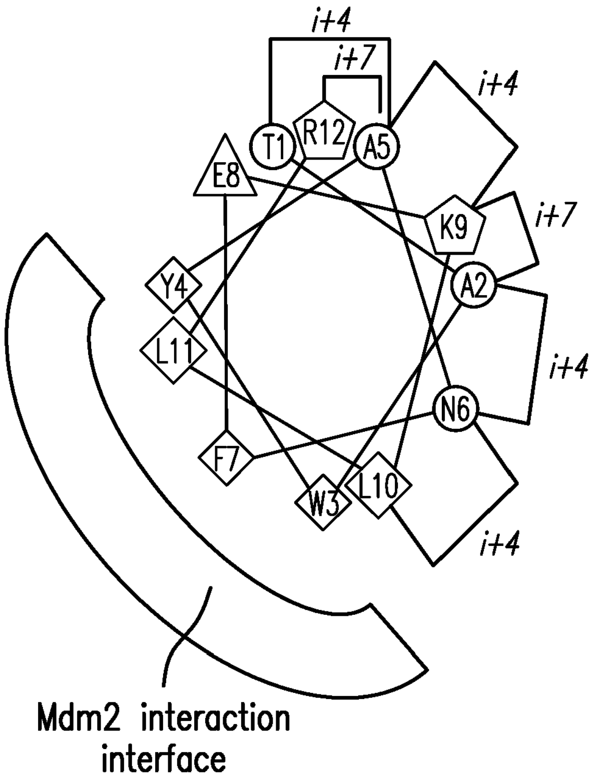


| Peptide | Sequence | | | | | | | | | | | |
|---|---|---|---|---|---|---|---|---|---|---|---|---|
| $^{D}$PMI-δ | T | A | 6-F-W | Y | A | N | *p*-$CF_3$-F | E | K | L | L | R |
| $^{D}$PMI-δ(1-5) | $R_5$ | A | 6-F-W | Y | $R_5$ | N | *p*-$CF_3$-F | E | K | L | L | R |
| $^{D}$PMI-δ(2-6) | T | $R_5$ | 6-F-W | Y | A | $R_5$ | *p*-$CF_3$-F | E | K | L | L | R |
| $^{D}$PMI-δ(2-9) | T | $S_8$ | 6-F-W | Y | A | N | *p*-$CF_3$-F | E | $R_5$ | L | L | R |
| $^{D}$PMI-δ(5-9) | T | A | 6-F-W | Y | $R_5$ | N | *p*-$CF_3$-F | E | $R_5$ | L | L | R |
| $^{D}$PMI-δ(5-12) | T | A | 6-F-W | Y | $S_8$ | N | *p*-$CF_3$-F | E | K | L | L | $R_5$ |
| $^{D}$PMI-δ(6-10) | T | A | 6-F-W | Y | A | $R_5$ | *p*-$CF_3$-F | E | K | $R_5$ | L | R |

FIG.4

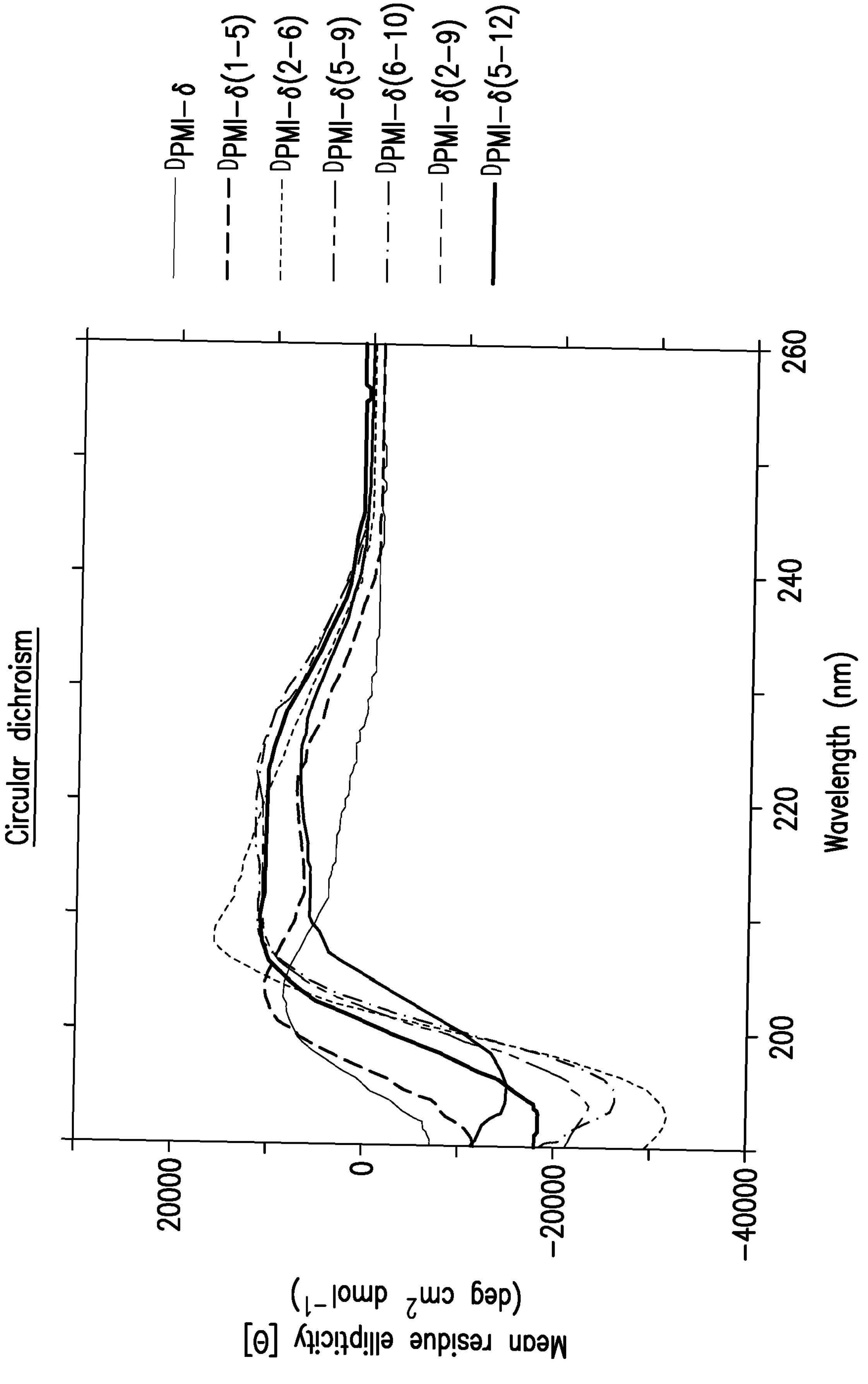
Circular dichroism
Mean residue ellipticity [Θ] (deg cm$^2$ dmol$^{-1}$)
20000
0
-20000
-40000
200
220
240
260
Wavelength (nm)
$^{D}$PMI-δ
$^{D}$PMI-δ(1-5)
$^{D}$PMI-δ(2-6)
$^{D}$PMI-δ(5-9)
$^{D}$PMI-δ(6-10)
$^{D}$PMI-δ(2-9)
$^{D}$PMI-δ(5-12)

FIG.5A

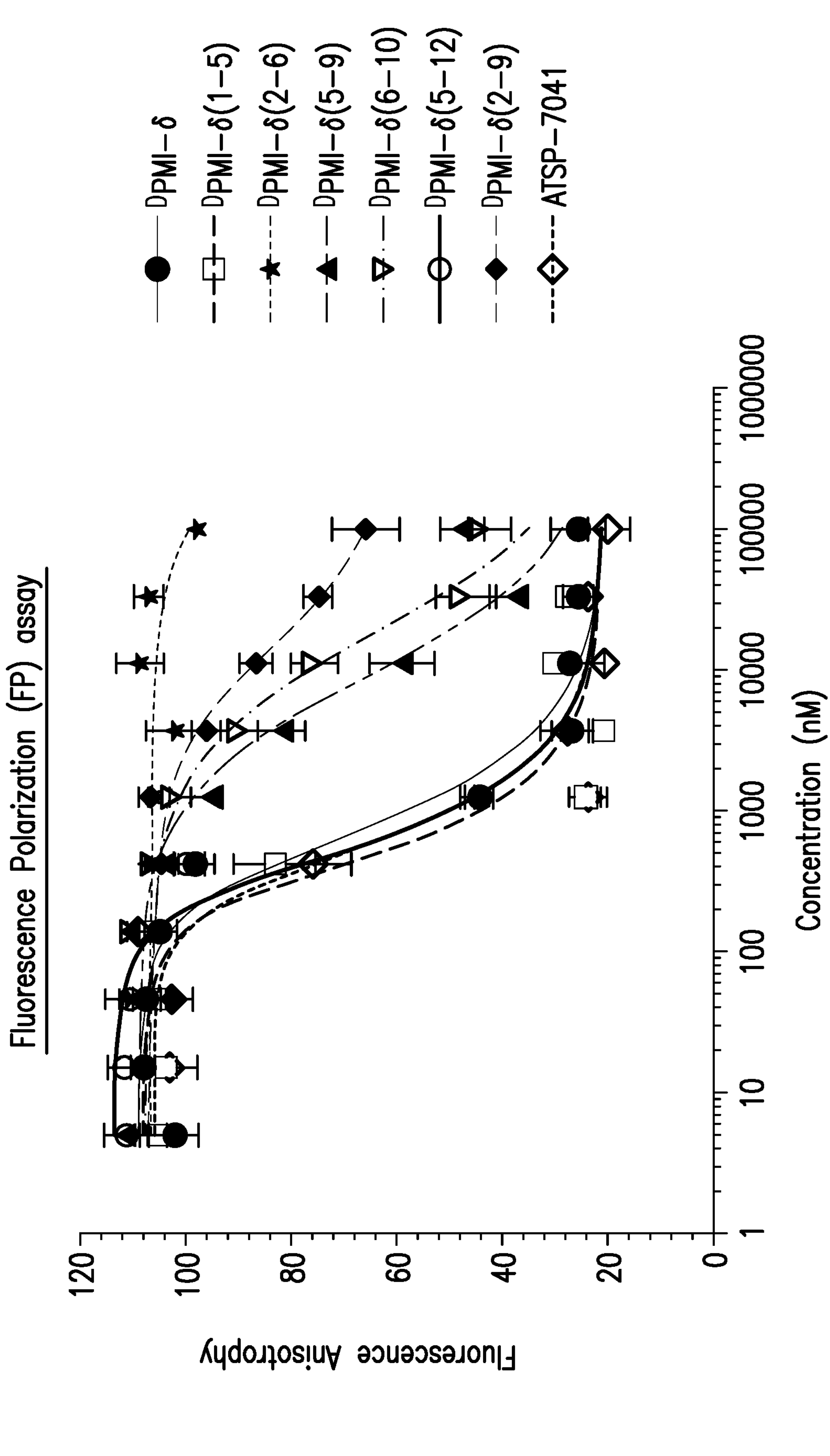
Fluorescence Polarization (FP) assay
Fluorescence Anisotropy
120
100
80
60
40
20
0
1
10
100
1000
10000
100000
1000000
Concentration (nM)
DPMI-δ
DPMI-δ(1-5)
DPMI-δ(2-6)
DPMI-δ(5-9)
DPMI-δ(6-10)
DPMI-δ(5-12)
DPMI-δ(2-9)
ATSP-7041

FIG.5B

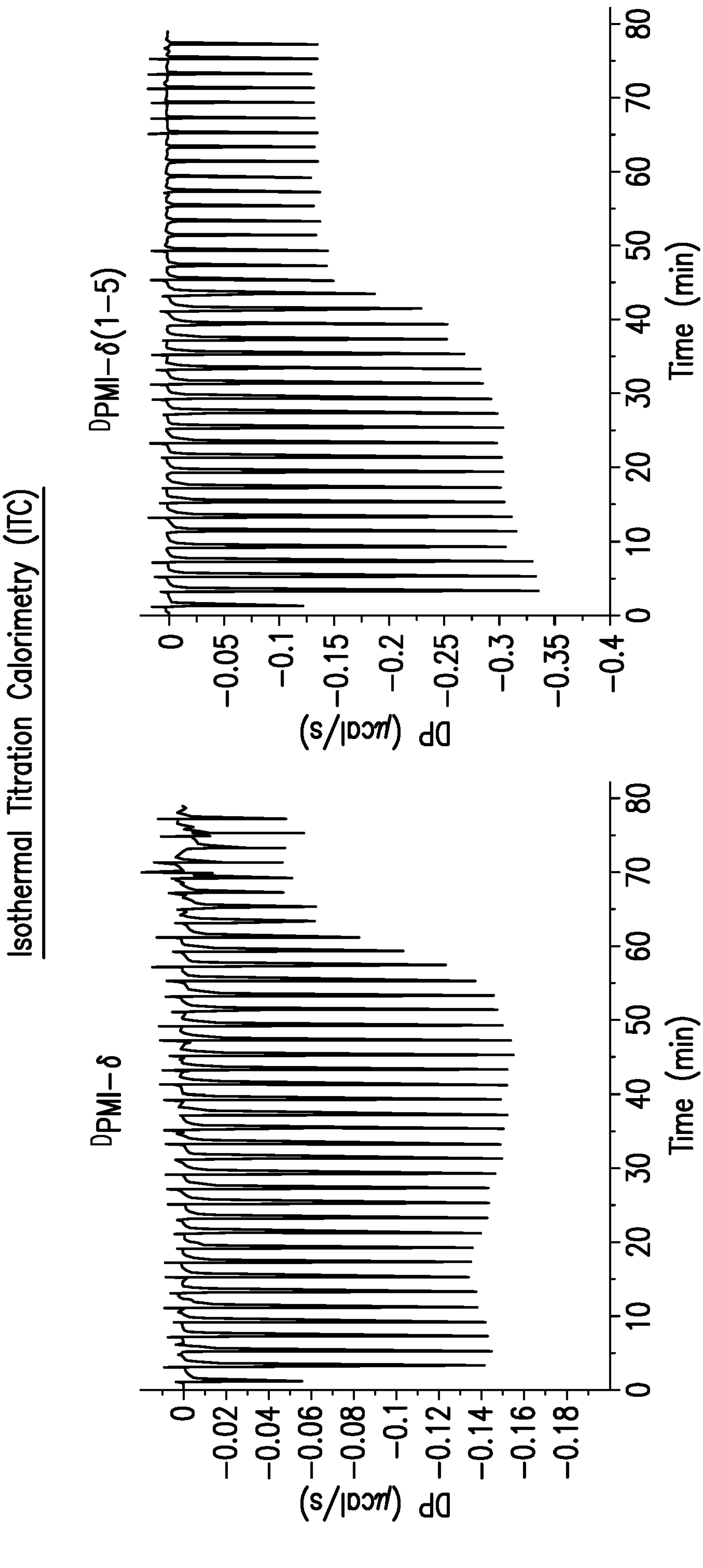
Isothermal Titration Calorimetry (ITC)
DPMI-δ
DP (μcal/s)
0
-0.02
-0.04
-0.06
-0.08
-0.1
-0.12
-0.14
-0.16
-0.18
0
10
20
30
40
50
60
70
80
Time (min)
DPMI-δ(1-5)
DP (μcal/s)
0
-0.05
-0.1
-0.15
-0.2
-0.25
-0.3
-0.35
-0.4
0
10
20
30
40
50
60
70
80
Time (min)

FIG.5C

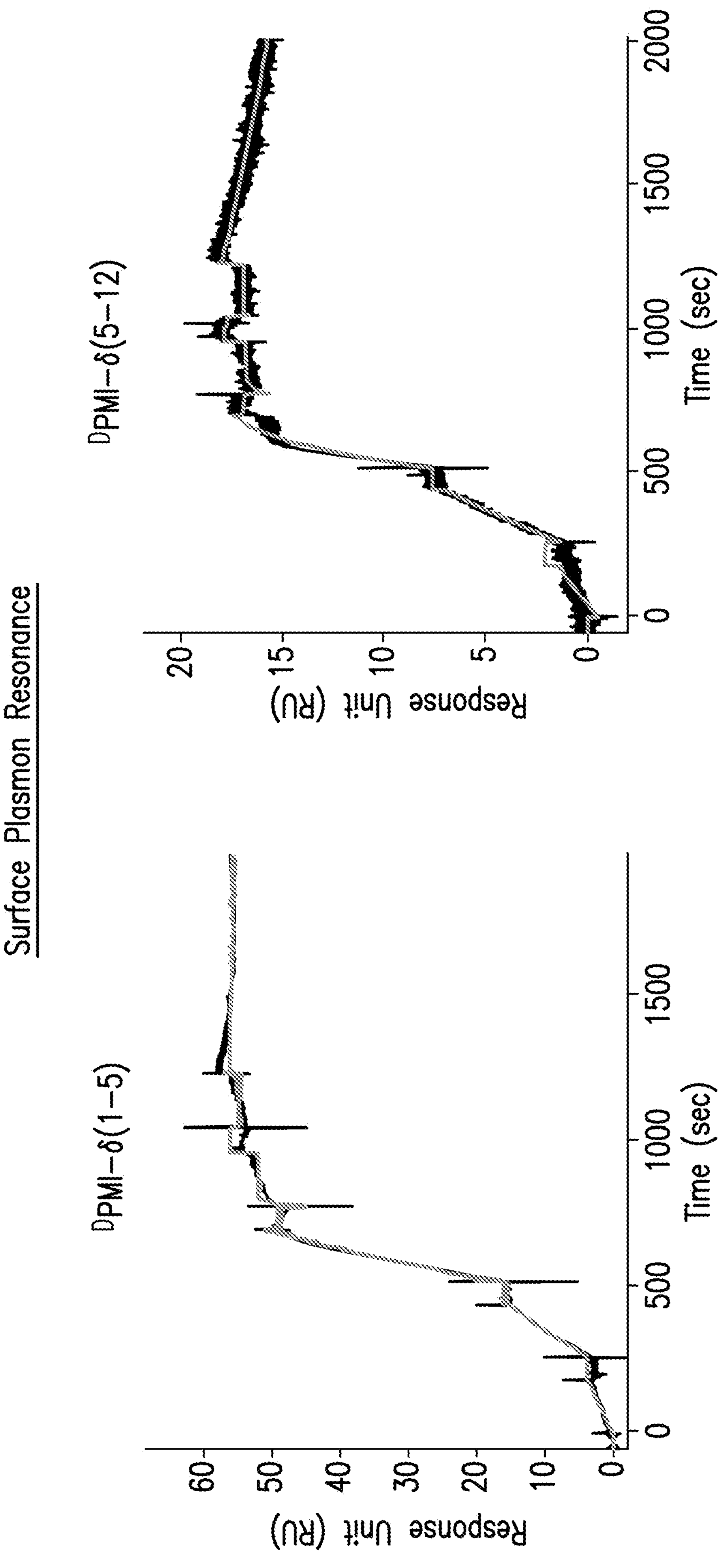
Surface Plasmon Resonance
DPMI-δ(1-5)
DPMI-δ(5-12)
Response Unit (RU)
Time (sec)
0
500
1000
1500
2000
0
10
20
30
40
50
60
5
15

FIG.5D

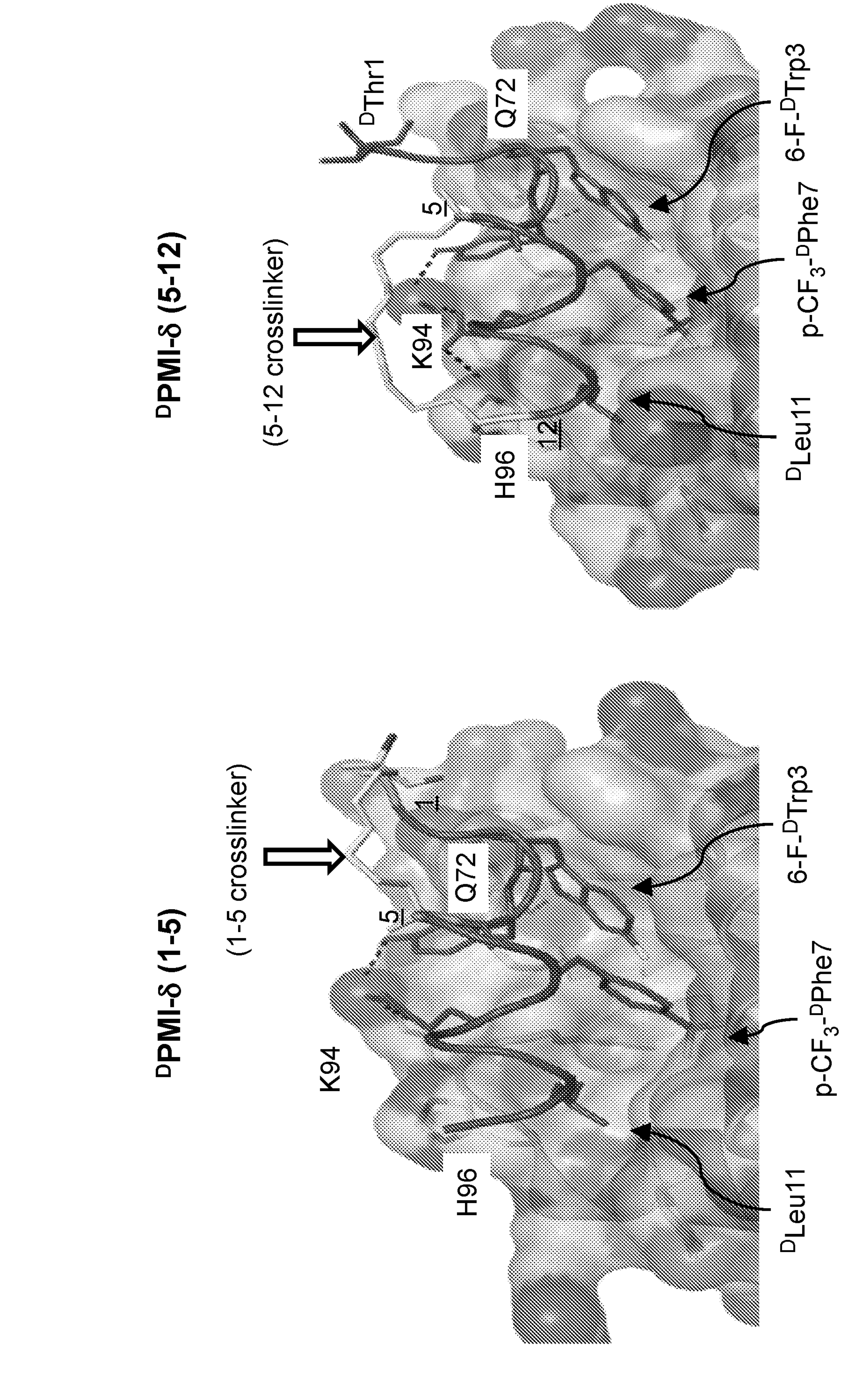

$^{D}$PMI-δ (1-5)
(1-5 crosslinker)
K94
H96
Q72
5
1
6-F-$^{D}$Trp3
p-CF$_3$-$^{D}$Phe7
$^{D}$Leu11
$^{D}$PMI-δ (5-12)
(5-12 crosslinker)
$^{D}$Thr1
Q72
5
K94
12
H96
6-F-$^{D}$Trp3
p-CF$_3$-$^{D}$Phe7
$^{D}$Leu11


FIG.6

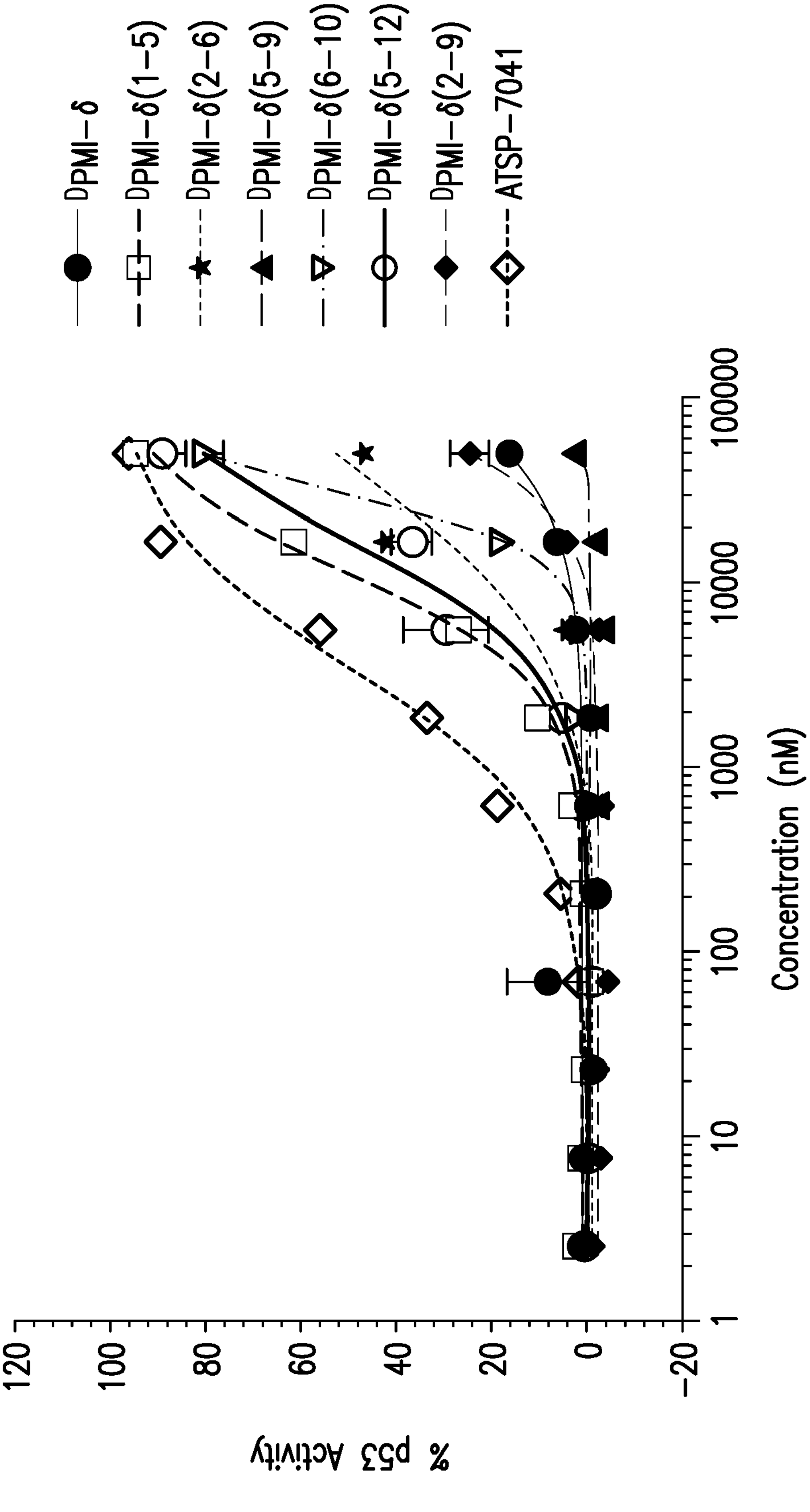
DPMI-δ
DPMI-δ(1-5)
DPMI-δ(2-6)
DPMI-δ(5-9)
DPMI-δ(6-10)
DPMI-δ(5-12)
DPMI-δ(2-9)
ATSP-7041
% p53 Activity
120
100
80
60
40
20
0
-20
1
10
100
1000
10000
100000
Concentration (nM)

FIG.7A

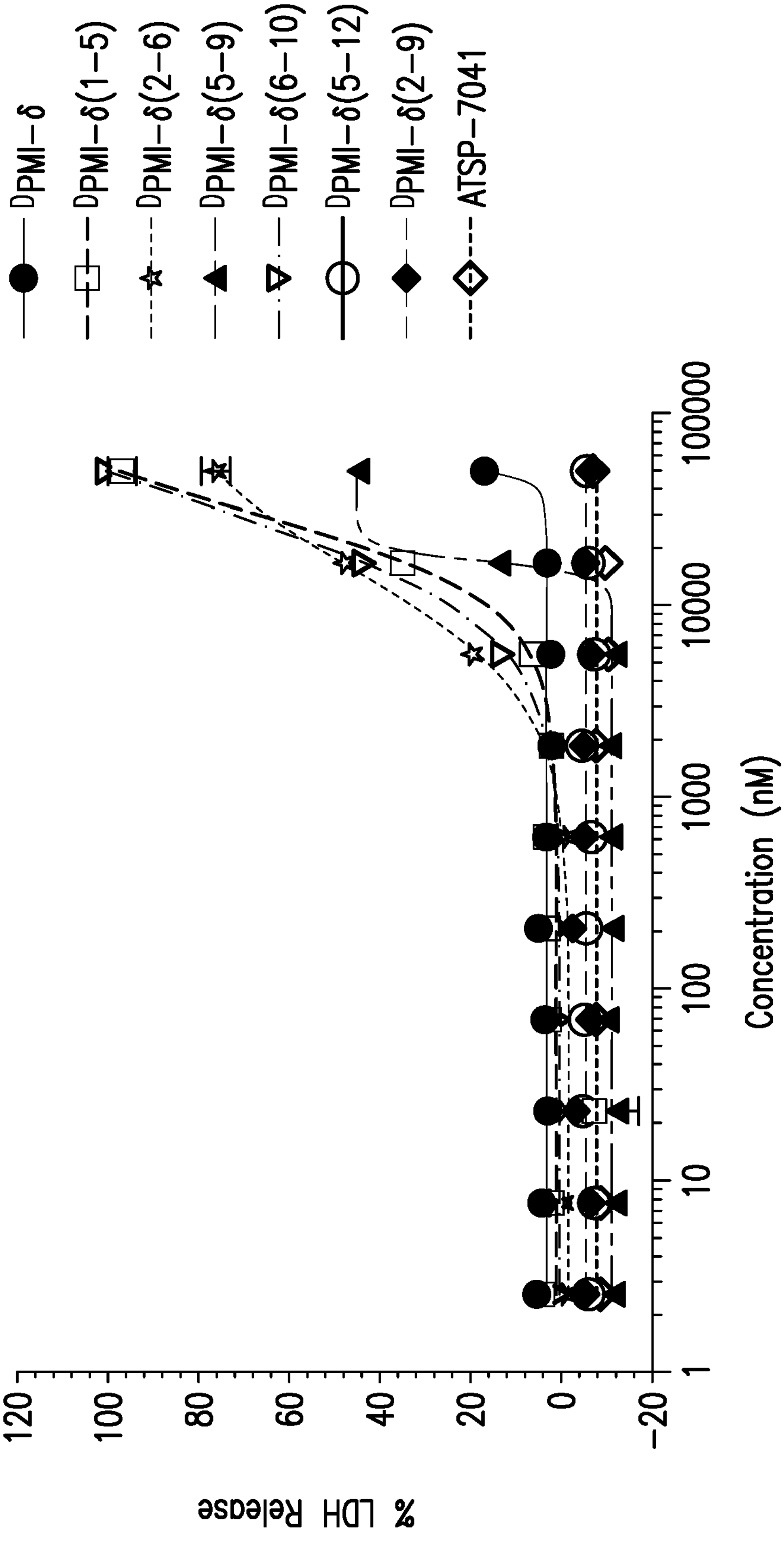
% LDH Release
120
100
80
60
40
20
0
−20
1
10
100
1000
10000
100000
Concentration (nM)
DPMI−δ
DPMI−δ(1−5)
DPMI−δ(2−6)
DPMI−δ(5−9)
DPMI−δ(6−10)
DPMI−δ(5−12)
DPMI−δ(2−9)
ATSP−7041

FIG.7B

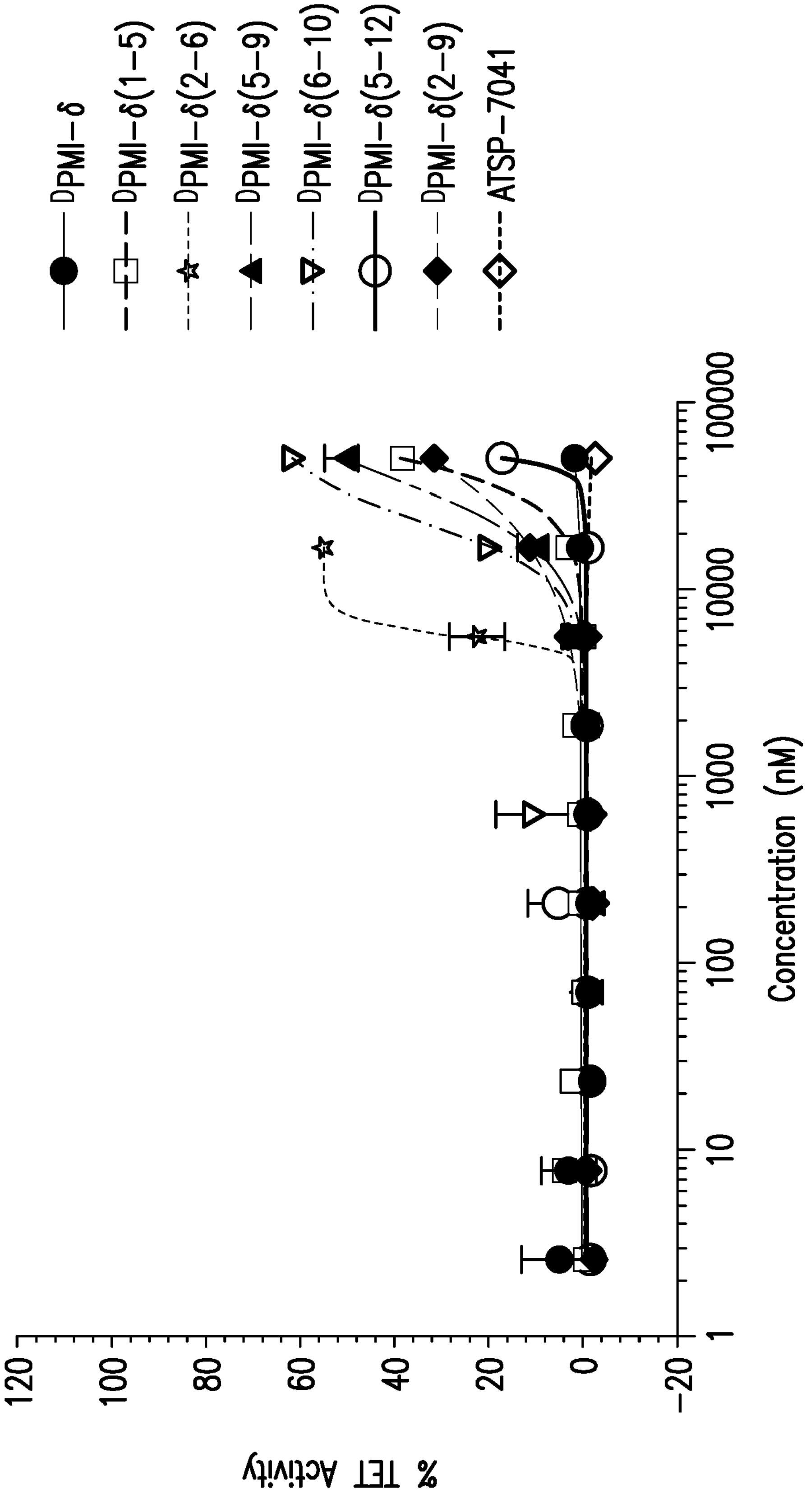
DPMI-δ
DPMI-δ(1-5)
DPMI-δ(2-6)
DPMI-δ(5-9)
DPMI-δ(6-10)
DPMI-δ(5-12)
DPMI-δ(2-9)
ATSP-7041
% TET Activity
120
100
80
60
40
20
0
-20
1
10
100
1000
10000
100000
Concentration (nM)

FIG.7C

| Peptide | Sequence | | | | | | | | | | | | |
|---|---|---|---|---|---|---|---|---|---|---|---|---|---|
| $^{D}$PMI−δ | T | A | 6−F−W | Y | A | N | $p$−$CF_3$−F | E | K | L | L | R |
| $^{D}$PMI−δ(1−5) | $R_5$ | A | 6−F−W | Y | $R_5$ | N | $p$−$CF_3$−F | E | K | L | L | R |
| $^{D}$PMI−δ(5−12) | T | A | 6−F−W | Y | $S_8$ | N | $p$−$CF_3$−F | E | K | L | L | $R_5$ |
| $^{D}$PMI−δ(1,5,12) | $R_5$ | A | 6−F−W | Y | $B_5$ | N | $p$−$CF_3$−F | E | K | L | L | $R_8$ |
| $^{D}$PMI−δ(1−5, 9−12) | $R_5$ | A | 6−F−W | Y | $R_5$ | N | $p$−$CF_3$−F | E | $S_5$ | L | L | $R_8$ |

**$^{D}$PMI−δ (1,5,12)**

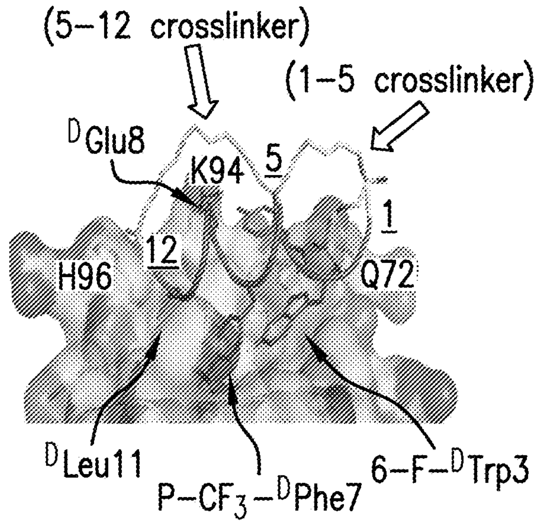


FIG.8A

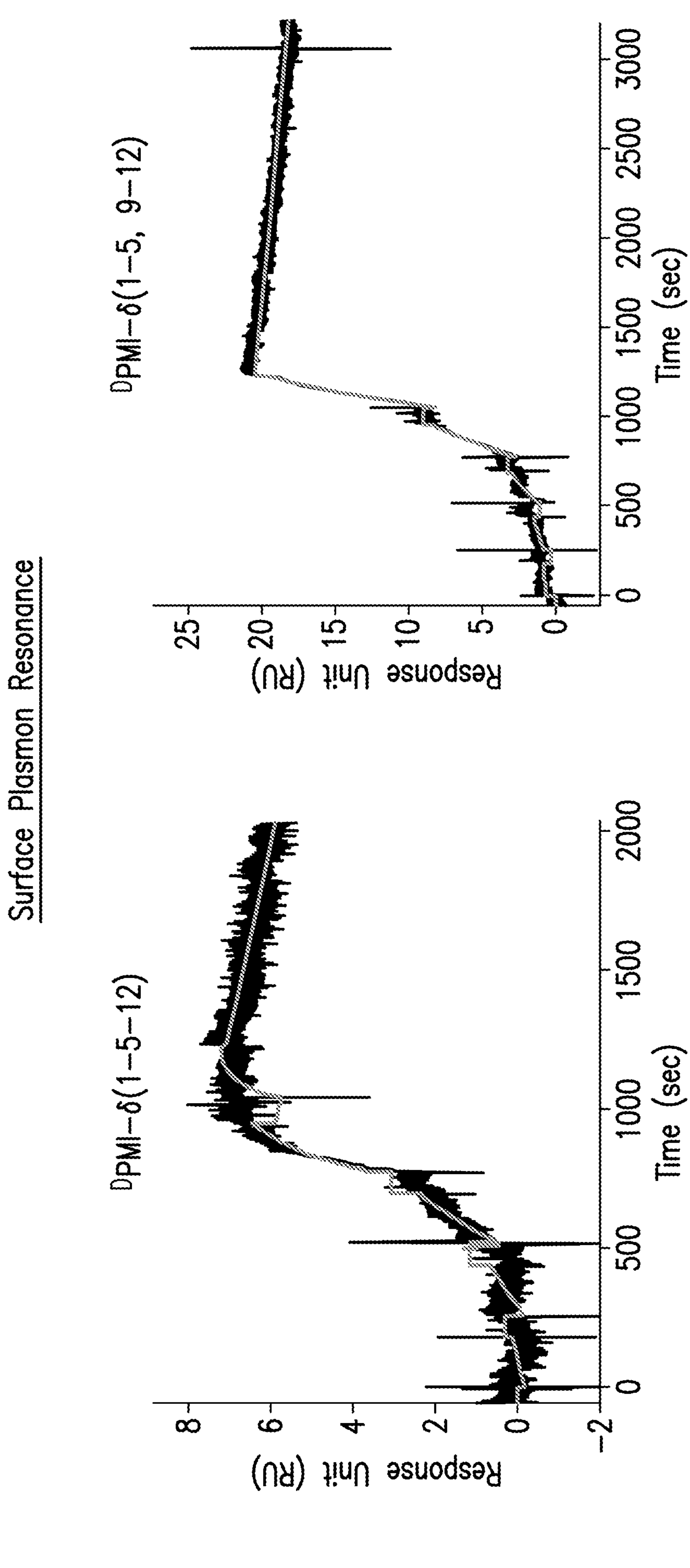
Surface Plasmon Resonance
DPMI-δ(1-5-12)
Response Unit (RU)
Time (sec)
DPMI-δ(1-5, 9-12)
Response Unit (RU)
Time (sec)

FIG.8B

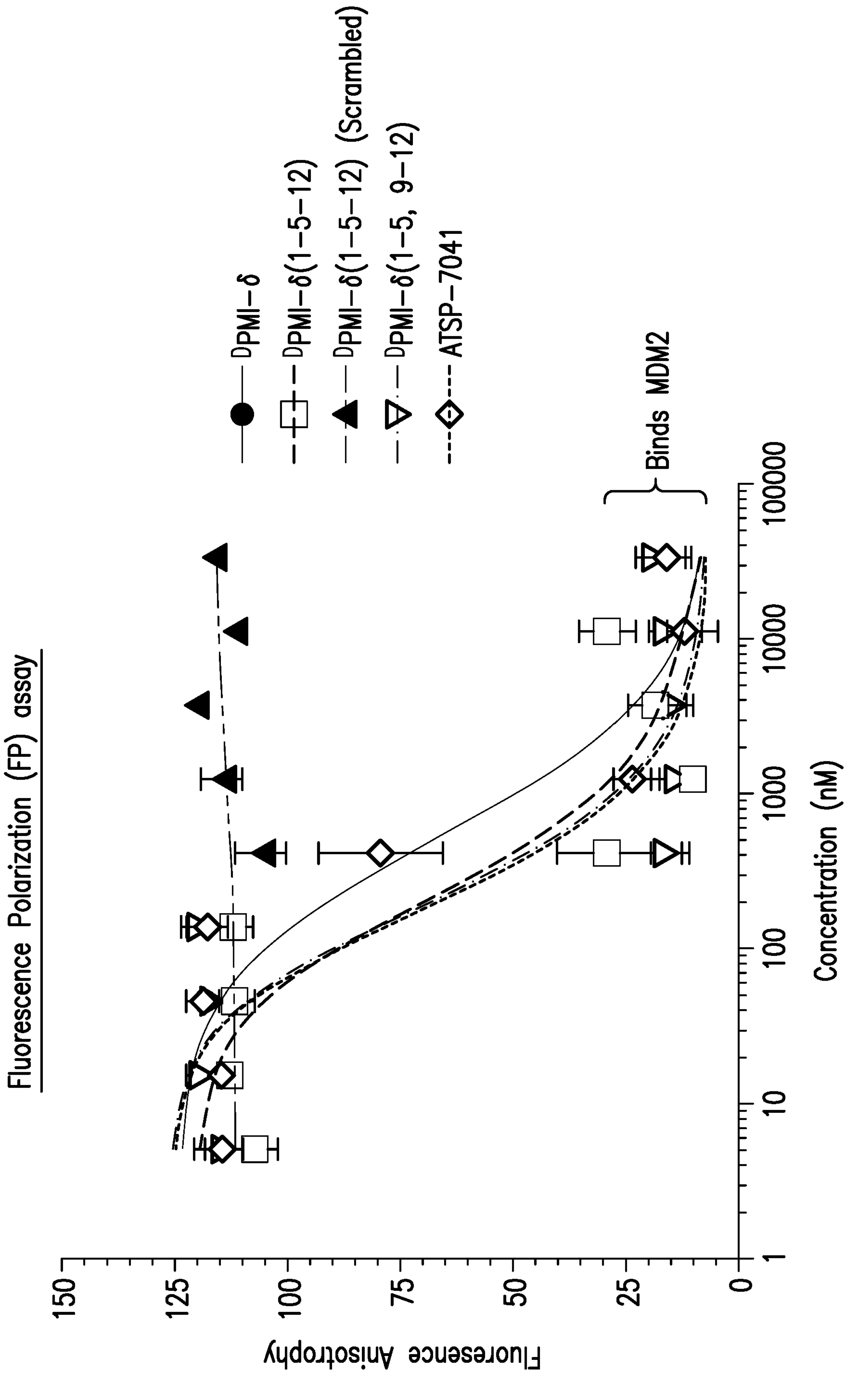

Fluorescence Polarization (FP) assay
DPMI-δ
DPMI-δ(1-5-12)
DPMI-δ(1-5-12) (Scrambled)
DPMI-δ(1-5, 9-12)
ATSP-7041
Binds MDM2
Fluoresence Anisotrophy
150
125
100
75
50
25
0
Concentration (nM)
1
10
100
1000
10000
100000


FIG.8C

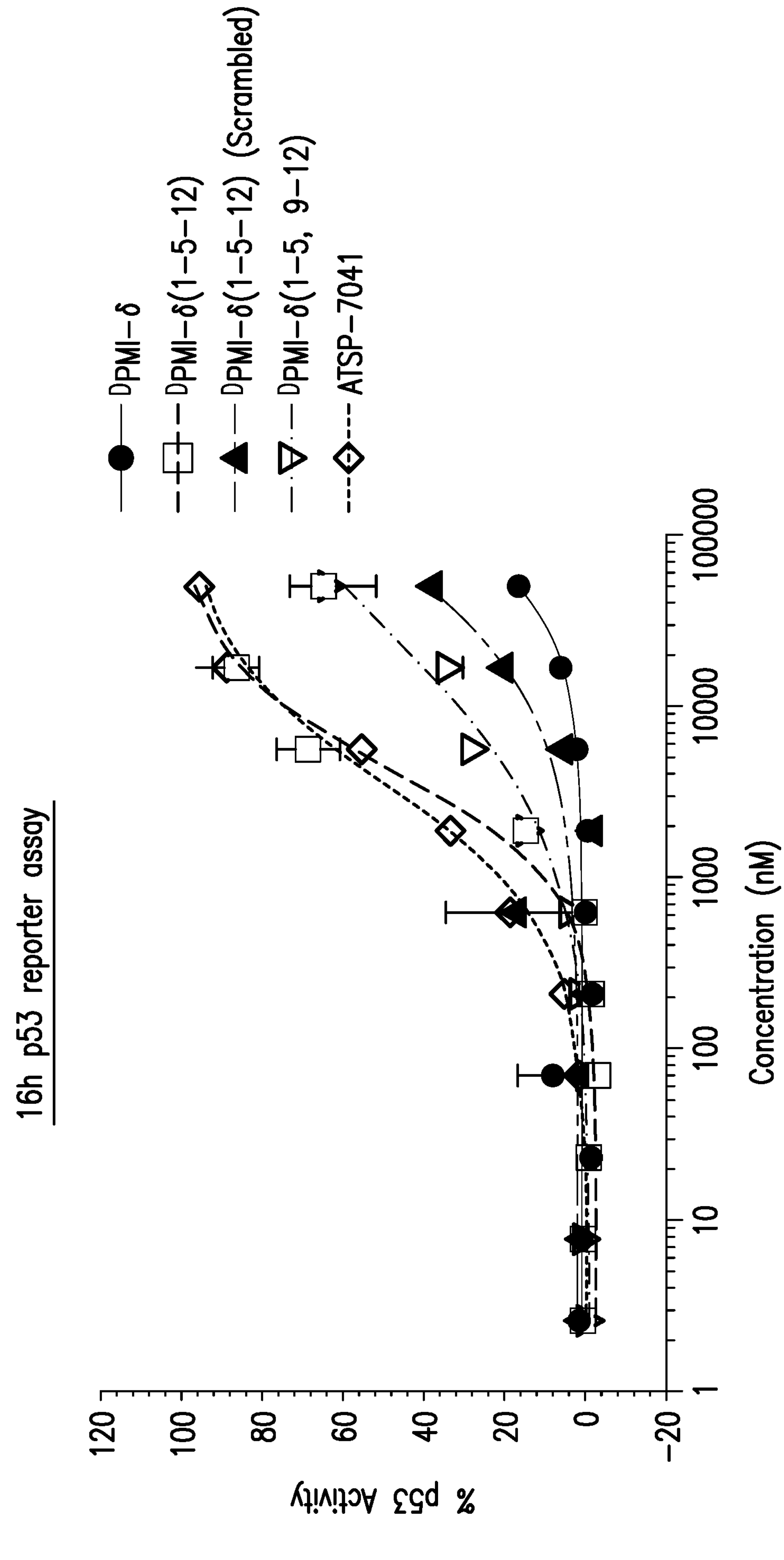
16h p53 reporter assay
% p53 Activity
120
100
80
60
40
20
0
-20
1
10
100
1000
10000
100000
Concentration (nM)
DPMI-δ
DPMI-δ(1-5-12)
DPMI-δ(1-5-12) (Scrambled)
DPMI-δ(1-5, 9-12)
ATSP-7041

FIG.8D

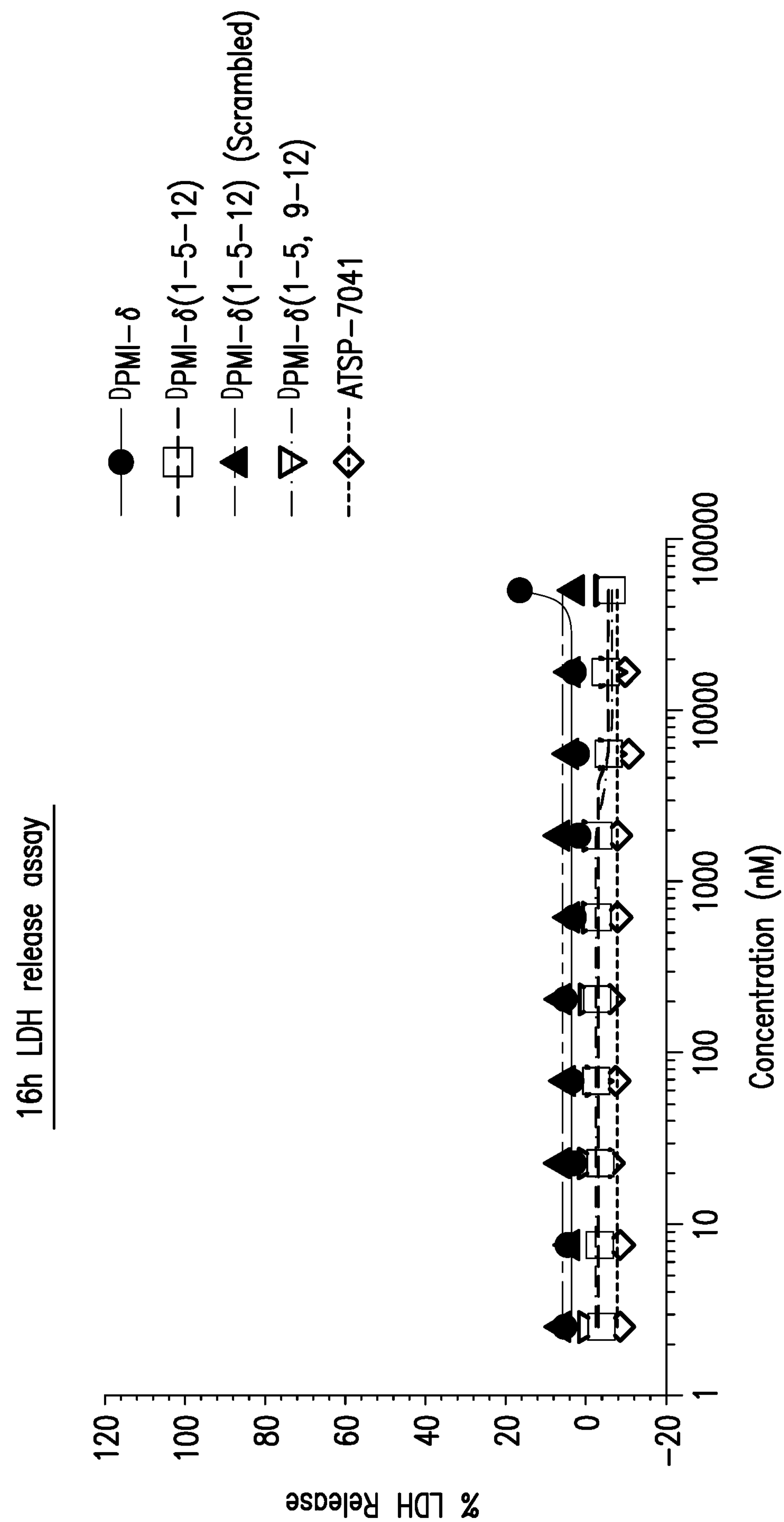
16h LDH release assay
% LDH Release
120
100
80
60
40
20
0
-20
1
10
100
1000
10000
100000
Concentration (nM)
DPMI-δ
DPMI-δ(1-5-12)
DPMI-δ(1-5-12) (Scrambled)
DPMI-δ(1-5, 9-12)
ATSP-7041

FIG.8E

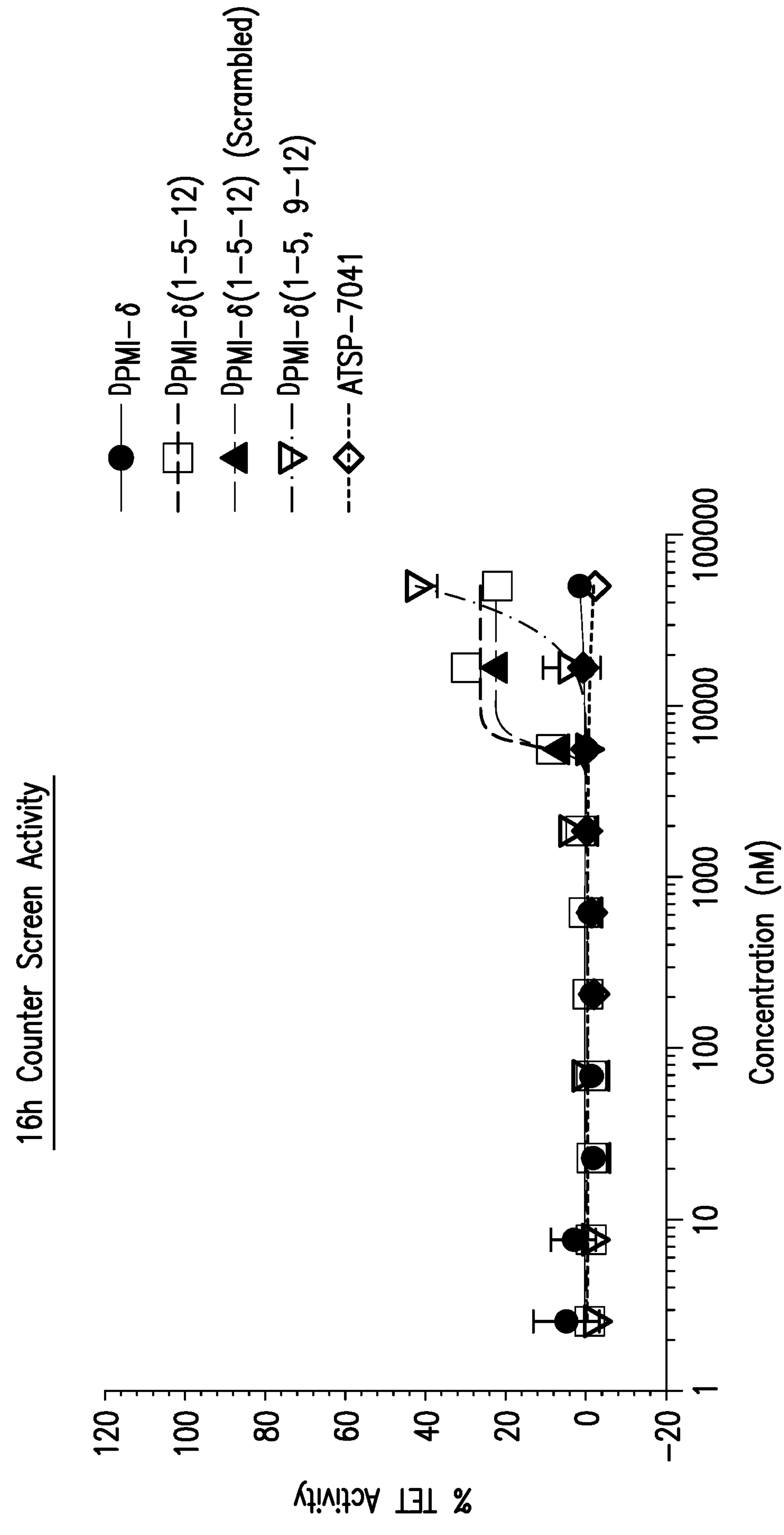
16h Counter Screen Activity
% TET Activity
120
100
80
60
40
20
0
−20
1
10
100
1000
10000
100000
Concentration (nM)
DPMI−δ
DPMI−δ(1−5−12)
DPMI−δ(1−5−12) (Scrambled)
DPMI−δ(1−5, 9−12)
ATSP−7041

FIG.8F

```
               10        20        30        40
               |         |         |         |
MDM2  MCNTNMSVPTDGAVTTSQIPASEQETLVRPKPLLKLLKSVG  42
MDM4  MTSFSTSAQCSTSDSACRISPG-QINQVRPKLPLLKILHAAG  41

             50        60        70        80
             |         |         |         |
MDM2  AQKDTYTMKEVLFYLGQYIMTKRLYDEKQQHIVYCSNDLLGD  84
MDM4  AQGEMFTVKEVMHYLGQYIMVKQLYDQQEQHMVYCGGDLLGE  83
             *************   **********      *

           90        100       110       120
           |         |         |         |
MDM2  LFGVPSFSVKEHRKIYTMIYRNLVVVNQQESSDSGT-SVSEN  125
MDM4  LLGRQSFSVKDPSPLYDMLRKNLVTLATATTDAAQTLALAQD  125
            ******  **
```

FIG.9A

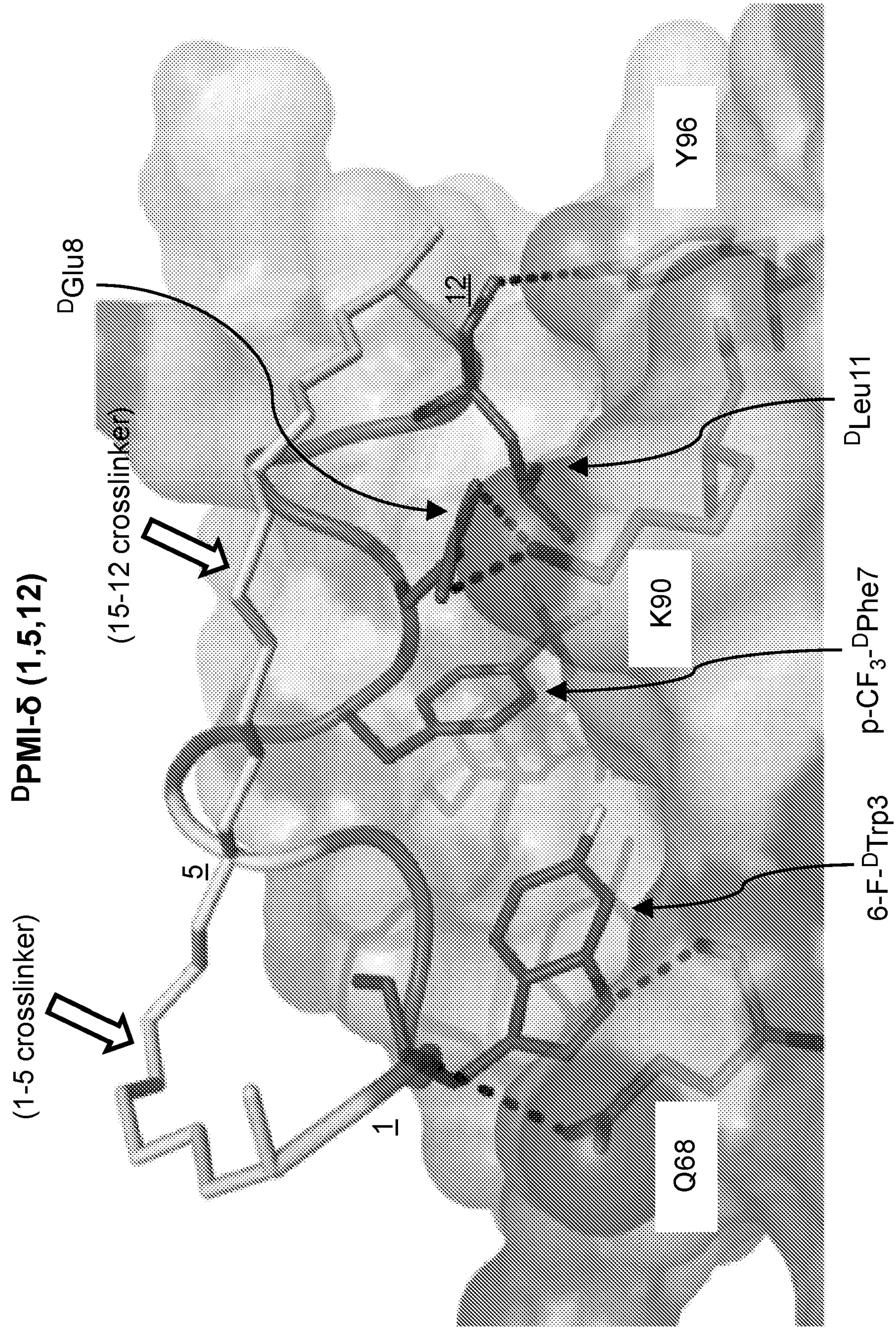
$^{D}$PMI-δ (1,5,12)
(1-5 crosslinker)
(15-12 crosslinker)
$^{D}$Glu8
1
5
12
$^{D}$Leu11
6-F-$^{D}$Trp3
p-CF$_3$-$^{D}$Phe7
Q68
K90
Y96

FIG.9B

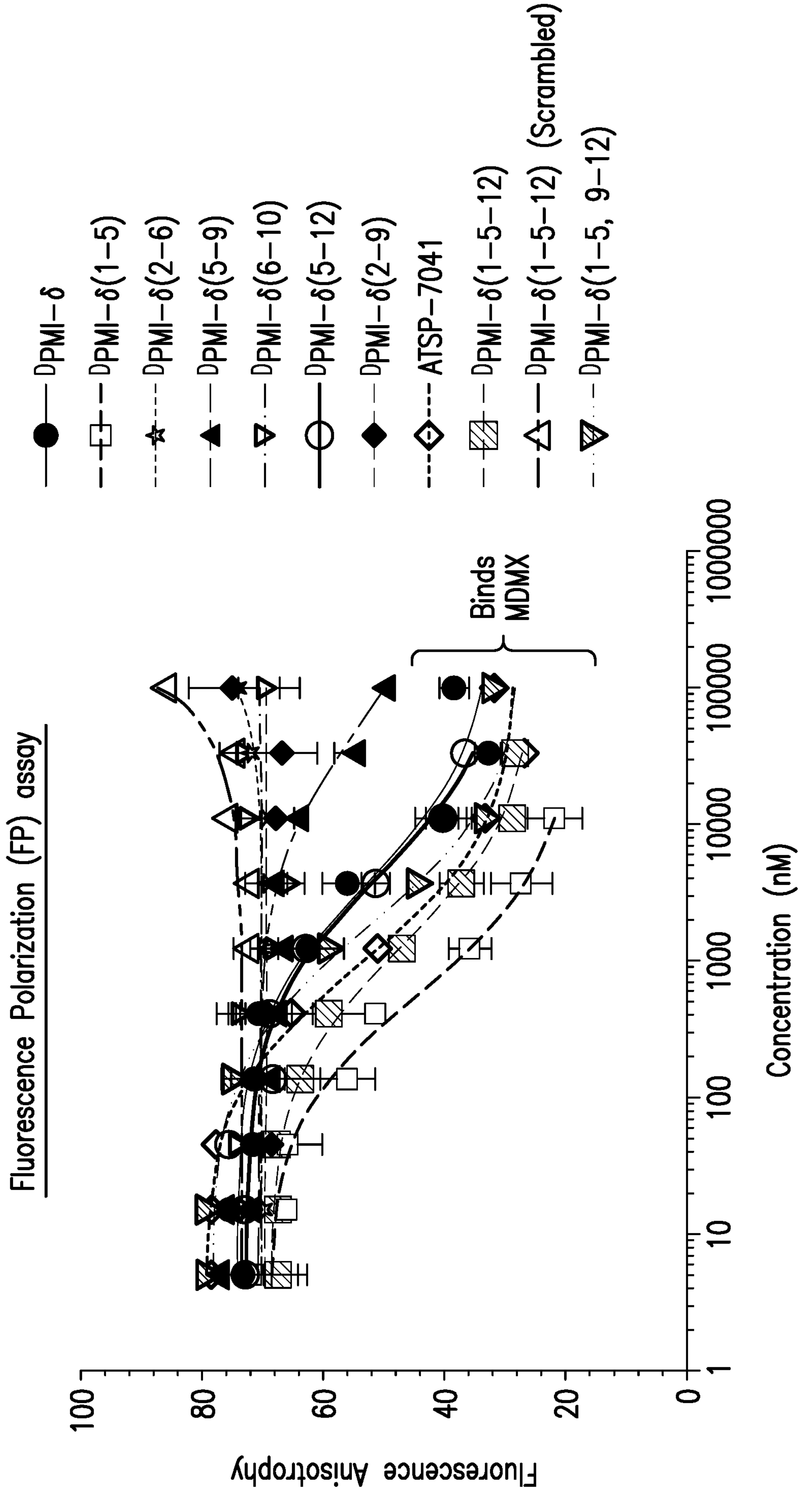
Fluorescence Polarization (FP) assay
Fluorescence Anisotropy
100
80
60
40
20
0
1
10
100
1000
10000
100000
1000000
Concentration (nM)
Binds MDMX
DPMI-δ
DPMI-δ(1-5)
DPMI-δ(2-6)
DPMI-δ(5-9)
DPMI-δ(6-10)
DPMI-δ(5-12)
DPMI-δ(2-9)
ATSP-7041
DPMI-δ(1-5-12)
DPMI-δ(1-5-12) (Scrambled)
DPMI-δ(1-5, 9-12)

FIG.9C

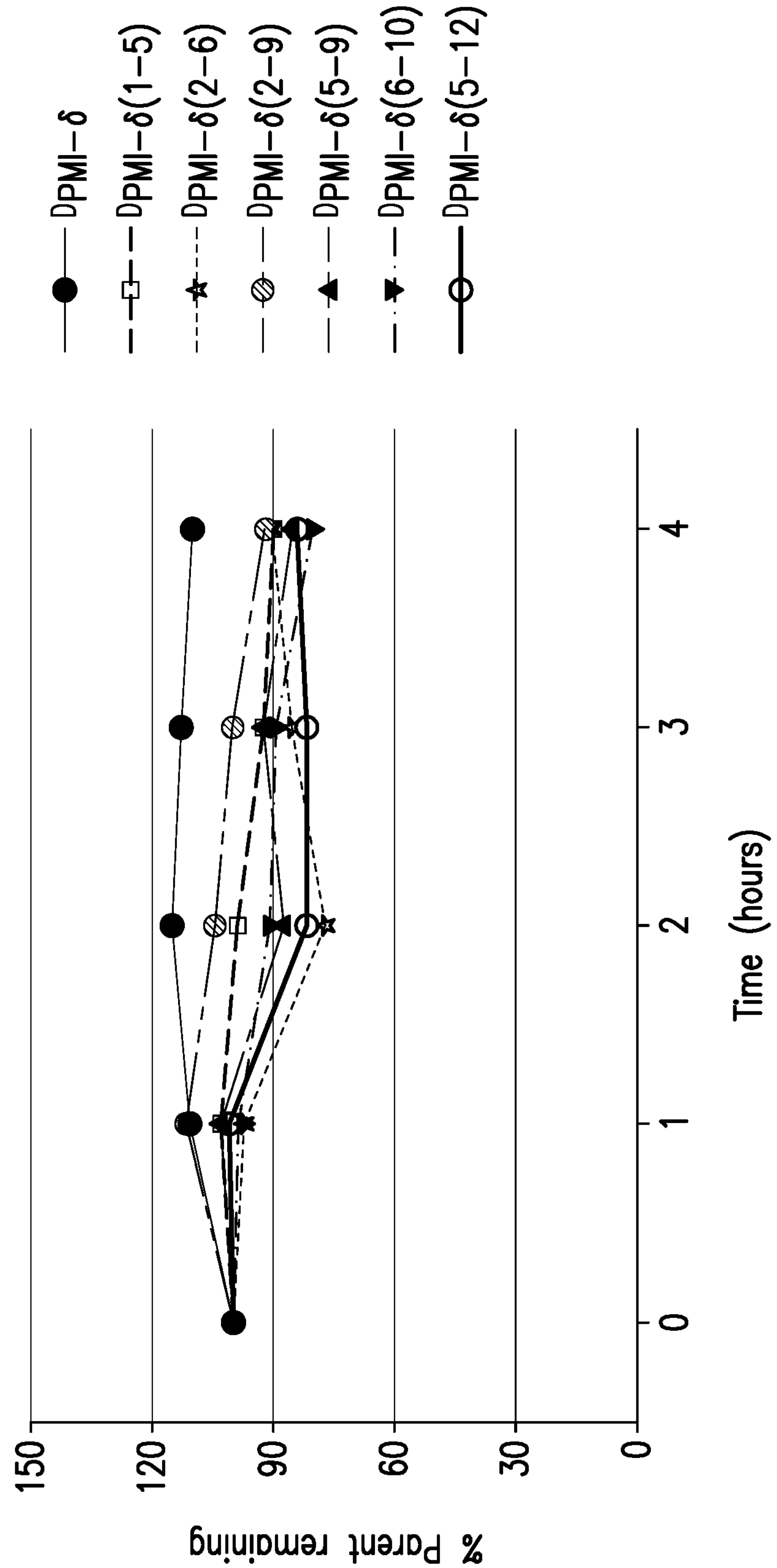
% Parent remaining
150
120
90
60
30
0
0
1
2
3
4
Time (hours)
DPMI-δ
DPMI-δ(1-5)
DPMI-δ(2-6)
DPMI-δ(2-9)
DPMI-δ(5-9)
DPMI-δ(6-10)
DPMI-δ(5-12)

FIG.10

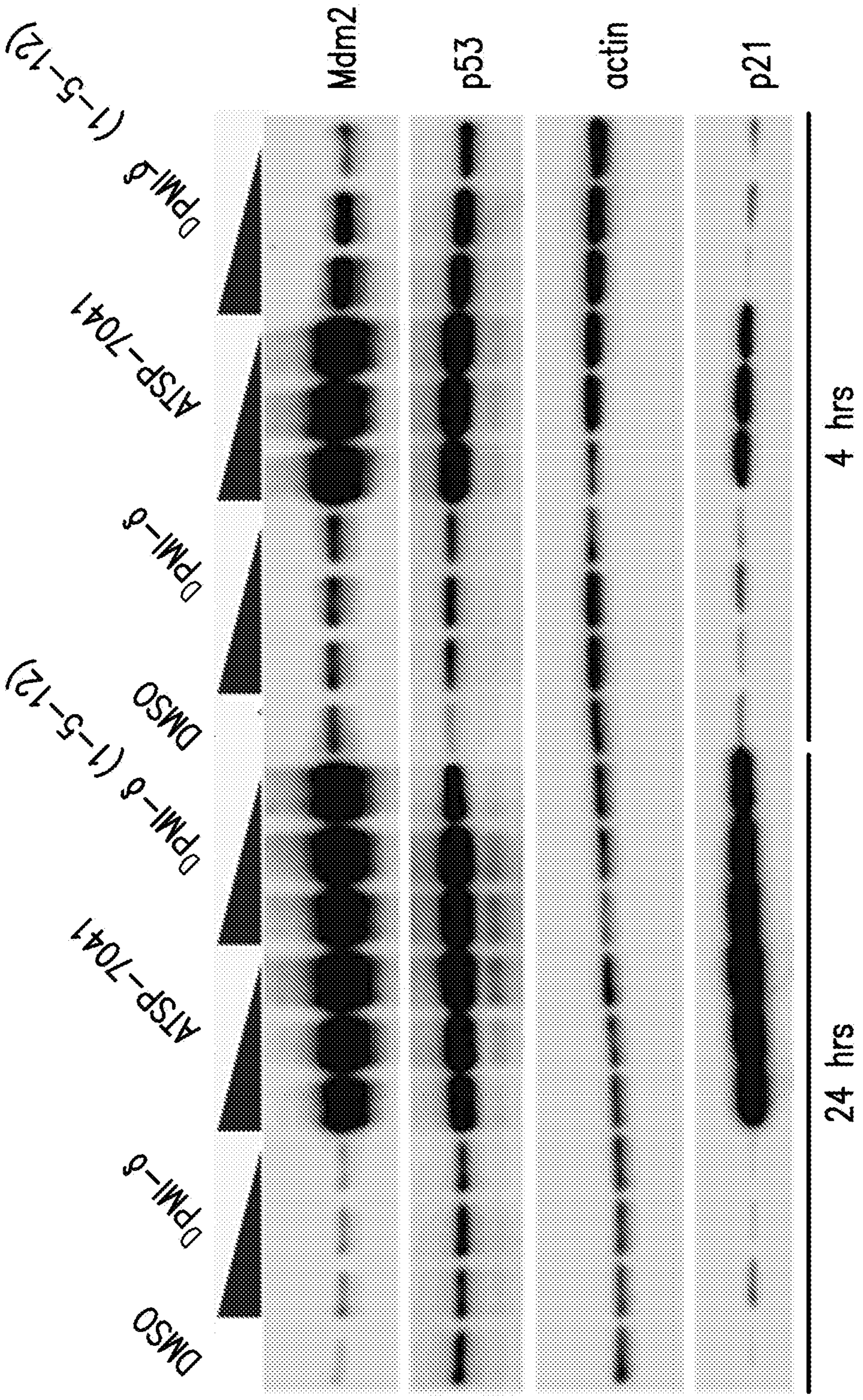

DMSO
DPMI-δ
ATSP-7041
DPMI-δ (1-5-12)
DMSO
DPMI-δ
ATSP-7041
DPMI-δ (1-5-12)
Mdm2
p53
actin
p21
4 hrs
24 hrs


FIG.11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013123266 A
- US 20120328692 A
- US 2012328692 A, Lu
- US 5811515 A

### Non-patent literature cited in the description


- **BERNAL et al.** *Cancer Cell*, 2010, vol. 18, 411-422
- **BAEK et al.** *JACS*, vol. 134, 103-106
- **ZHANG et al.** *J. Med. Chem.*, 2012, vol. 55, 6237-6241
- **WATSON et al.** Molecular Biology of the Gene. The Benjamin/Cummings Pub. Co., 1987, 224
- **GRUBBS et al.** Ring Closing Metathesis and Related Processes in Organic Synthesis. *Acc. Chem. Res.*, 1995, vol. 28, 446-452
- *Pharmaceutical Research*, 1986, vol. 3 (6), 318
- **WAWRZYNCZAK**. Antibody Therapy. Bios Scientific Pub. Ltd, 1996
- Monoclonal Antibodies, Cytokines and Arthritis. Marcel Dekker, 1991
- Monoclonal Antibodies and Peptide Therapy in Autoimmune Diseases. Marcel Dekker, 1993
- **BAERT et al.** *New Engl. J. Med.*, 2003, vol. 348, 601-608
- **MILGROM et al.** *New Engl. J. Med.*, 1999, vol. 341, 1966-1973
- **SLAMON et al.** *New Engl. J. Med.*, 2001, vol. 344, 783-792
- **BENIAMINOVITZ et al.** *New Engl. J. Med.*, 2000, vol. 342, 613-619
- **GHOSH et al.** *New Engl. J. Med.*, 2003, vol. 348, 24-32
- **LIPSKY et al.** *New Engl. J. Med.*, 2000, vol. 343, 1594-1602
- Physicians' Desk Reference 2003 (Physicians' Desk Reference, 57th Ed). Medical Economics Company, November 2002
- **PETTA I.** ; **LIEVENS S.** ; **LIBERT C.** ; **TAVERNIER J.** ; **DE BOSSCHER K.** Modulation of Protein Protein Interactions for the Development of Novel Therapeutics. *Mol. Ther.*, 2015, vol. 24, 707-718
- **MACALINO S.J.Y.** ; **BASITH S.** ; **CLAVIO N.A.B.** ; **CHANG H.** ; **KANG S.** ; **CHOI S.** Evolution of In Silico Strategies for Protein-Protein Interaction Drug Discovery. *Molecules*, 2018, vol. 23, 1963
- **SCOTT, D.E.** ; **BAYLY, A.R.** ; **ABELL, C** ; **SKIDMORE, J.** Small molecules, big targets: drug discovery faces the protein-protein interaction challenge. *Nat. Rev. Drug Discov.*, 2016, vol. 15, 533-550
- **NEVOLA L** ; **GIRALT E.** Modulating protein protein interactions: the potential of peptides. *Chem Commun.*, 2015, vol. 51, 3302-15
- **LAU, J. L.** ; **DUNN, M. K**. Therapeutic peptides: Historical perspectives, current development trends, and future directions Bioorg. *Med. Chem.*, 2017, vol. 26, 2700-2707
- **FOSGERAU, K.** ; **HOFFMANN, T.** Peptide therapeutics: current status and future directions. *Drug Discovery Today*, 2015, vol. 20, 122-128
- **BAKAIL M.** ; **OCHSENBEIN F.** Targeting protein-protein interactions, a wide open field for drug design. *Comptes Rendus Chimie.*, 2016, vol. 19, 19-27
- **A. HENNINOT** ; **J.C. COLLINS** ; **J.M. NUSS**. The current state of peptide drug discovery: back to the future. *J. Med. Chem.*, 2018, vol. 61, 1382-1414
- **MORRISON C.** Constrained peptides' time to shine?.. *Nature Reviews Drug Discovery*, 2018, vol. 17, 531-533
- **VALEUR E. et al.** New Modalities for Challenging Targets in Drug Discovery. *Angew. Chem. Int. Ed.*, 2017, vol. 56, 10294-10323
- **CARY, D. R.** ; **OHUCHI, M.** ; **REID, P. C.** ; **MASUYA, K.** Constrained Peptides in Drug Discovery and Development. *Yuki Gosei Kagaku Kyokaishi*, 2017, vol. 75, 1171-1178
- **VINOGRADOV A.** Macrocyclic Peptides as Drug Candidates: Recent Progress and Remaining Challenges. *J. Am. Chem. Soc.*, 2019, vol. 141 (10), 4167-4181
- **SAWYER, T. K.** Macrocyclic $\alpha$ helical peptide therapeutic modality: A perspective of learnings and challenges. *Bioorg. Med. Chem.*, 2018, vol. 26, 2807-2815

- **L.D. WALENSKY** ; **G.H. BIRD**. Hydrocarbon-stapled peptides: principles, practice, and progress. *J Med Chem*, 2014, vol. 57, 6275-6288
- **Y.S. TAN** ; **D.P. LANE** ; **C.S. VERMA**. Stapled peptide design: principles and roles of computation. *Drug Discov Today*, 2016, vol. 21, 1642-1653
- **ALI AM. et al.** Stapled Peptides Inhibitors: A New Window for Target Drug Discovery. *Computational and Structural Biotechnology Journal*, 2019, vol. 17, 263-281
- **KLEIN M.** Stabilized helical peptides: overview of the technologies and its impact on drug discovery. *Expert Opinion on Drug Discovery.*, 2017, vol. 12, 1117-1125
- **LERGE J et al.** Stapled peptides as a new technology to investigate protein-protein interactions in human platelets. *Chem Sci*, vol. 2108 (9), 4638-4643
- **XU W.** Macrocyclized Extended Peptides: Inhibiting the Substrate-Recognition Domain of Tankyrase. *J. Am. Chem. Soc.*, 2017, vol. 139 (6), 2245-2256
- **WIEDMANN, MM. et al.** Development of Cell-Permeable ,Non-Helical Constrained Peptides to Target a Key Protein-Protein Interaction in Ovarian Cancer. *Angew .Chem. Int.Ed.*, 2017, vol. 56, 524-529
- **L. D. WALENSKY** ; **A. L. KUNG** ; **I. ESCHER** ; **T. J. MALIA** ; **S. BARBUTO** ; **R. D. WRIGHT** ; **G. WAGNER** ; **G. L. VERDINE** ; **S. J. KORSMEYER**. Activation of apoptosis in vivo by a hydrocarbon-stapled BH3 helix. *Science*, 2004, vol. 305, 1466-1470
- **S. A. KAWAMOTO** ; **A. COLESKA** ; **X. RAN** ; **H. YI** ; **C. Y. YANG** ; **S. WANG**. Design of triazole-stapled BCL9 α-helical peptides to target the β-catenin/B-cell CLL/lymphoma 9 (BCL9) protein-protein interaction. *J.Med. Chem.*, 2012, vol. 55, 1137-1146
- **D. HORST** ; **M. FULCINITI** ; **N. C. MUNSHI** ; **W. XU** ; **A. L. KUNG** ; **R. A. SHIVDASANI** ; **L. D. WALENSKY** ; **D. R. CARRASCO**. Targeted disruption of the BCL9/β-catenin complex inhibits oncogenic Wnt signaling. *Sci. Transl. Med.*, 2012, vol. 4, 148-117
- **L DIETRICH** ; **B RATHMER** ; **K EWAN** ; **T BANGE** ; **S HEINRICHS** ; **TC DALE** ; **D SCHADE** ; **TN GROSSMANN**. Cell permeable stapled peptide inhibitor of Wnt signaling that targets β-catenin protein-protein interactions. *Cell Chem. Biol.*, 2017, vol. 24, 958-968
- **SPIEGEL J** ; **CROMM PM** ; **ITZEN A** ; **GOODY RS** ; **GROSSMANN TN** ; **WALDMANN H**. Direct targeting of Rab-GTPase-effector interactions. *Angew Chem Int Ed Engl.*, 24 February 2014, vol. 53 (9), 2498-503
- **XIE M. et al.** Structural Basis of Inhibition of ERα-Coactivator Interaction by High-Affinity N-Terminus Isoaspartic Acid Tethered Helical Peptides. *J. Med. Chem.*, 2017, vol. 60 (21), 8731-8740
- **PAOLA DE. et al.** Cullin3-BTB interface: a novel target for stapled peptides. *PLoS One*, 07 April 2015, vol. 10 (4), e0121149
- **MISAWA T. et al.** Structural development of stapled short helical peptides as vitamin D receptor (VDR)-coactivator interaction inhibitors. *Bioorg Med Chem.*, 01 March 2015, vol. 23 (5), 1055-61
- **LAMA D. et al.** Structural insights reveal a recognition feature for tailoring hydrocarbon stapled-peptides against the eukaryotic translation initiation factor 4E protein. *Chemical Science*, 2019, vol. 10, 2489-2500
- **F. BEMAL** ; **A. F. TYLER** ; **S. J. KORSMEYER** ; **L. D. WALENSKY** ; **G. L. VERDINE**. Reactivation of the p53 tumor suppressor pathway by a stapled p53 peptide. *J. Am. Chem. Soc.*, 2007, vol. 129, 2456-2457
- **L. K. HENCHEY** ; **J. R. PORTER** ; **I. GHOSH** ; **P. S. ARORA**. High specificity in protein recognition by hydrogen-bond-surrogate a-helices: selective inhibition of the p53/MDM2 complex. *Chembiochem*, 2010, vol. 11, 2104-2107
- **C. J. BROWN, S. T.** ; **QUAH, J. JONG** ; **A. M. GOH, P. C.** ; **CHIAM, K. H. KHOO** ; **M. L. CHOONG** ; **M. A. LEE** ; **L. YURLOVA** ; **K. ZOLGHADR** ; **T. L. JOSEPH** ; **C. S. VERMA**. Stapled peptides with improved potency and specificity that activate p53. *ACS Chem. Biol.*, 2013, vol. 8, 506-512
- **Y. S. CHANG** ; **B. GRAVES** ; **V. GUERLAVAIS** ; **C. TOVAR** ; **K. PACKMAN** ; **T. TO, K. OLSON** ; **K. KESAVAN** ; **P. GANGURDE** ; **A. MUKHERJEE** ; **T. BAKER**. Stapled a-helical peptide drug development: a potent dual inhibitor of MDM2 and MDMX for p53-dependent cancer therapy. *Proc. Natl. Acad. Sci. U.S.A*, 2013, vol. 110, E3445-E3455
- **A. BURGESS** ; **K. M. CHIA** ; **S. HAUPT** ; **D. THOMAS** ; **Y.HAUPT** ; **E. LIM**. Clinical overview of MDM2/Xtargeted therapies. *Front. Oncol.*, 2016, vol. 6
- **K. KOJIMA** ; **J. ISHIZAWA** ; **M. ANDREEFF**. Pharmacological activation of wild-type p53 in the therapy of leukemia. *Exp. Hematol.*, 2016, vol. 44, 791-798
- **V. TISATO** ; **R. VOLTAN** ; **A. GONELLI** ; **P. SECCHIERO** ; **G. ZAULI**. MDM2/X inhibitors under clinical evaluation: Perspectives for the management of hematological malignancies and pediatric cancer. *J. Hematol. Oncol.*, 2017, vol. 10, 133
- **CROMM PM. et al.** Protease-Resistant and Cell-Permeable Double-Stapled Peptides Targeting the Rab8a GTPase. *ACS Chem. Biol.*, 2016, vol. 11 (8), 2375-2382
- **BIRD GH. et al.** Hydrocarbon double-stapling remedies the proteolytic instability of a lengthy peptide therapeutic. *PNAS*, 10 August 2010, vol. 107 (32), 14093-14098

- **GAILLARD V. et al.** A Short Double-Stapled Peptide Inhibits Respiratory Syncytial Virus Entry and Spreading. *Antimicrobial Agents and Chemotherapy*, March 2017, vol. 61 (4), e02241-16
- **M. LIU** ; **M. PAZGIER** ; **C. LI** ; **W. YUAN** ; **C. LI** ; **W. LU**. *Angew. Chem., Int. Ed.*, 2010, vol. 49, 3649
- **M. LIU** ; **C. LI** ; **M. PAZGIER** ; **C. LI** ; **Y. MAO** ; **Y. LV** ; **B. GU** ; **G. WEI** ; **W. YUAN** ; **C. ZHAN**. *Proc. Natl. Acad. Sci. USA*, 2010, vol. 107, 14321
- **K. MANDAL** ; **M. UPPALAPATI** ; **D. AULT-RICHÉ** ; **J. KENNEY** ; **J. LOWITZ** ; **S.S. SIDHU** ; **S.B. KENT**. *Proc. Natl. Acad. Sci. USA*, 2012, vol. 109, 14779
- **H.N. CHANG** ; **B.Y. LIU** ; **Y.K. QI** ; **Y. ZHOU** ; **Y.P. CHEN** ; **K.M. PAN** ; **W.W. LI** ; **X.M. ZHOU** ; **W.W. MA** ; **C.Y. FU**. *Angew. Chem., Int. Ed.*, 2015, vol. 54, 11760
- **WELCH BD** ; **FRANCIS JN** ; **REDMAN JS** ; **PAUL S** ; **WEINSTOCK MT** ; **REEVES JD** ; **LIE YS** ; **WHITBY FG** ; **ECKERT DM** ; **HILL CP**. Design of a potent D-peptide HIV-1 entry inhibitor with a strong barrier to resistance. *J Virol.*, 2010, vol. 84 (21), 11235-44
- **LANE DP**. p53, guardian of the genome. *Nature*, 02 July 1992, vol. 358 (6381), 15-6
- **AMI-SCHMIDT, O.** ; **M. LOKSHIN** ; **C. PRIVES**. The roles of MDM2 and MDMX in cancer. *Annu. Rev. Pathol.*, 2016, vol. 11, 617-644
- **DONGSHENG PEI** ; **YANPING ZHANG** ; **JUNNIAN ZHENG**. Regulation of p53: a collaboration between MDM2 and MDMX.. *Oncotarget*, March 2012, vol. 3 (3), 228-235
- **TISATO V** ; **VOLTAN R** ; **GONELLI A** ; **SECCHIERO P** ; **ZAULI G**. MDM2/X inhibitors under clinical evaluation: perspectives for the management of hematological malignancies and pediatric cancer. *J Hematol Oncol.*, 03 July 2017, vol. 10 (1), 133
- **F. FUNDA MERIC-BERNSTAM** ; **M. S. SALEH** ; **J. R. INFANTE** ; **S. GOEL** ; **G. S. FALCHOOK** ; **G. SHAPIRO** ; **K. Y. CHUNG** ; **R. M. CONRY** ; **D. S. HONG** ; **J. S. WANG**. Phase I trial of a novel stapled peptide ALRN-6924 disrupting MDMX- and MDM2-mediated inhibition of WT p53 in patients with solid tumors and lymphomas. *J. Clin. Oncol.*, 2017, vol. 35, 2505-2505
- **ZHAN, C.** ; **ZHAO, L.** ; **WEI, X.** ; **WU, X.** ; **CHEN, X.** ; **YUAN, W.** ; **LU, W. Y.** ; **PAZGIER, M.** ; **LU, W.** An ultrahigh affinity d-peptide antagonist of MDM2. *J. Med. Chem.*, 2012, vol. 55, 6237-6241
- **CJ BROWN** ; **ST QUAH** ; **J JONG** ; **AM GOH** ; **PC CHIAM** ; **KH KHOO** ; **ML CHOONG et al.** Stapled peptides with improved potency and specificity that activate p53. *ACS chemical biology*, vol. 8 (3), 506-512
- **TAN, Y. S. et al.** Benzene Probes in Molecular Dynamics Simulations Reveal Novel Binding Sites for Ligand Design. *J Phys Chem Lett*, vol. 7, 3452-3457
- **D. THEAN** ; **J. S. EBO** ; **T. LUXTON** ; **XUE'ER CHERYL LEE** ; **T. Y. YUEN** ; **F. J. FERRER** ; **C. W. JOHANNES** ; **D. P. LANE** ; **C. J. BROWN**. Enhancing Specific Disruption of Intracellular Protein Complexes by Hydrocarbon Stapled Peptides Using Lipid Based Delivery. *Scientific Reportsvolume*, 2017, vol. 7
- **KANNAN S** ; **ZACHARIAS M**. Enhanced sampling of peptide and protein conformations using replica exchange simulations with a peptide backbone biasing-potential.. *Proteins*, 2007, vol. 66, 697-7006
- **OSTERMEIR K** ; **ZACHARIAS M**. Hamiltonian replica-exchange simulations with adaptive biasing of peptide backbone and side chain dihedral angles. *J. Comput. Chem.*, 2014, vol. 35
- **SCHAFMEISTER, C. E.** ; **PO, J.** ; **VERDINE, G. L.** An all-hydrocarbon cross-linking system for enhancing the helicity and metabolic stability of peptides. *J. Am. Chem. Soc.*, 2000, vol. 122, 5891-5892, 150-158
- **LD WALENSKY** ; **GH BIRD**. ydrocarbon-Stapled Peptides: Principles, Practice, and Progress. *J.Med.Chem.*, 2014, vol. 57, 6275-6288
- **Y.S. CHANG** ; **B. GRAVES** ; **V. GUERLAVAIS** ; **C. TOVAR** ; **K. PACKMAN** ; **T. TO** ; **K. OLSON** ; **K. KESAVAN** ; **P. GANGURDE** ; **A. MUKHERJEE**. Stapled a-helical peptide drug development: a potent dual inhibitor of MDM2 and MDMX for p53-dependent cancer therapy. *Proc. Natl. Acad. Sci. U.S.A*, 2013, vol. 110, E3445-E3455
- **A. FURUKAWA** ; **C.E. TOWNSEND** ; **J. SCHWOCHERT** ; **C.R. PYE** ; **M.A. BEDNAREK** ; **R.S. LOKEY**. Passive membrane permeability in cyclic peptomer scaffolds is robust to extensive variation in side chain functionality and backbone geometry. *J Med Chem*, 2016, vol. 59, 9503-9512
- **LI YC et al.** A versatile platform to analyze low-affinity and transient protein-protein interactions in living cells in real time. *Cell Rep.*, 2014, vol. 9, 1946-58
- **BIRD GH et al.** Mucosal delivery of a double-stapled RSV peptide prevents nasopulmonary infection. *J Clin Invest.*, 2014, vol. 124, 2113-24
- **THOMAS JC** ; **COOPER JM** ; **CLAYTON NS** ; **WANG C** ; **WHITE MA** ; **ABELL C** ; **OWEN D** ; **MOTT HR**. Inhibition of Ral GTPases using a stapled peptide approach.. *J Biol Chem*, 2016, vol. 291, 18310-18325
- **P. M. CROMM** ; **J. SPIEGEL** ; **P. KÜCHLER** ; **L. DIETRICH** ; **J. KRIEGESMANN** ; **M. WENDT** ; **R. S. GOODY** ; **H. WALDMANN** ; **T. N. GROSSMANN**. *ACS Chem. Biol.*, 2016, vol. 11, 2375-2382
- **SPELTZ T.E.** ; **MAYNE C.G.** ; **FANNING S.W.** ; **SIDDIQUI Z.** ; **TAJKHORSHID E.** ; **GREENE G.L.** A "cross-stitched" peptide with improved helicity and proteolytic stability. *Org Biomol Chem.*, 2018, vol. 16, 3702-3706

- **KAWAMOTO S.A.** ; **COLESKA A.** ; **RAN X.** ; **YI H.** ; **YANG C.Y.** ; **WANG S.** Design of triazole-stapled BCL9 a-helical peptides to target the B-catenin/B-cell CLL/lymphoma 9 (BCL9) protein-protein interaction. *J Med Chem.*, 2012, vol. 55, 1137-1146
- **HILINSKI G.J.** ; **KIM Y.-W.** ; **HONG J.** ; **KUTCHUKIAN P.S.** ; **CRENSHAW C.M.** ; **BERKOVITCH S.S. et al.** Stitched α-helical peptides via bis ring-closing metathesis.. *J Am Chem Soc*, 03 September 2014, vol. 136 (35), 12314-22